# EUROPEAN PATENT APPLICATION

(11) **EP 3 072 964 A1**
(43) Date of publication of application: **28.09.2016**
(21) Application number: 14863814.1
(22) Date of filing: 17.11.2014
(51) Int. Cl.: C12N 15/09, A61K 39/395, A61K 45/00, C07K 14/705, C07K 19/00, C12N 9/12, C12Q 1/02, G01N 33/15, G01N 33/50, G01N 33/53, G01N 33/574

(54) **NOVEL CHIMERA GENE ATF7IP-PDGFRB FOR ACUTE LYMPHOBLASTIC LEUKEMIA**

(30) Priority: 22.11.2013 JP 2013242285
(71) Applicant: National Center For Child Health And Development, Tokyo 157-8535 (JP); National Cancer Center, Tokyo 104-0045 (JP); LSIP, LLC, Chiyoda-ku Tokyo 100-0005 (JP)
(72) Inventor: KIYOKAWA, Nobutaka, Tokyo 157-8535 (JP); MATSUMOTO, Kenji, Tokyo 157-8535 (JP); KOBAYASHI, Kenichiro, Tokyo 157-8535 (JP); ICHIKAWA, Hitoshi, Tokyo 104-0045 (JP)
(74) Representative: Cabinet Plasseraud
(86) International application number: PCT/JP2014/080306
(87) International publication number: WO 2015/076213

(57) **Abstract**

To identify a mutation that can serve as an indicator for predicting the effectiveness of drug treatment in cancers such as leukemia; to provide a means for detecting said mutation; and to provide a means for identifying, based on said mutation, patients with cancer or subjects with a risk of cancer, in whom a drug targeting a gene having said mutation or a protein encoded by said gene shows a therapeutic effect.

A method for detecting a gene fusion serving as a responsible mutation (driver mutation) for cancer, the method comprising the step of detecting an ATF7IP-PDGFRB fusion polynucleotide or a polypeptide encoded thereby, in an isolated sample from a subject.

SEQUENCE LISTING

<110> National Center for Child Health and Development
      National Cancer Center
      LSIP, LLC

<120> Novel chimeric gene ATF7IP-PDGFRB in acute lymphoblastic leukemia

<130> FA1575-14162

<150> JP 2013-242285
<151> 2013-11-22

<160> 26

<170> PatentIn version 3.5

<210> 1
<211> 5022
<212> DNA
<213> Homo sapiens


<220>
<221> CDS
<222> (1)..(5022)

<400> 1
atg gac agt tta gaa gaa cct cag aaa aaa gtc ttt aag gct cga aaa      48
Met Asp Ser Leu Glu Glu Pro Gln Lys Lys Val Phe Lys Ala Arg Lys
1               5                   10                  15

acg atg aga gtg agt gat cgt cag caa ctt gaa gca gtg tac aag gtc      96
Thr Met Arg Val Ser Asp Arg Gln Gln Leu Glu Ala Val Tyr Lys Val
            20                  25                  30

aaa gaa gaa ctg ttg aaa act gat gtc aag ctg tta aat ggc aac cat      144
Lys Glu Glu Leu Leu Lys Thr Asp Val Lys Leu Leu Asn Gly Asn His
        35                  40                  45

gaa aat gga gat ttg gac cca acc tca cct ttg gaa aac atg gat tac      192
Glu Asn Gly Asp Leu Asp Pro Thr Ser Pro Leu Glu Asn Met Asp Tyr
    50                  55                  60

att aaa gac aag gaa gag gtg aat ggc att gaa gag att tgt ttt gat      240
Ile Lys Asp Lys Glu Glu Val Asn Gly Ile Glu Glu Ile Cys Phe Asp
65                  70                  75                  80

cct gaa gga agt aaa gca gaa tgg aag gaa aca ccc tgt atc cta agt      288
Pro Glu Gly Ser Lys Ala Glu Trp Lys Glu Thr Pro Cys Ile Leu Ser
                85                  90                  95

gtt aat gta aaa aac aag cag gat gat gat tta aat tgt gaa cct ttg      336
Val Asn Val Lys Asn Lys Gln Asp Asp Asp Leu Asn Cys Glu Pro Leu
            100                 105                 110

tct ccc cat aat ata act cca gaa cca gtc tct aaa ctg cct gct gaa      384
Ser Pro His Asn Ile Thr Pro Glu Pro Val Ser Lys Leu Pro Ala Glu
        115                 120                 125

cca gtt tct ggt gat cca gcc cct ggt gat ctg gat gcc gga gat cca      432
Pro Val Ser Gly Asp Pro Ala Pro Gly Asp Leu Asp Ala Gly Asp Pro
    130                 135                 140

gcc tcc gga gta ctg gcc tct ggt gat tcc acc tct ggt gat ccc acc     480
Ala Ser Gly Val Leu Ala Ser Gly Asp Ser Thr Ser Gly Asp Pro Thr
145                 150                 155                 160

tct agc gag ccc tcc tct agt gat gct gcc tct ggt gat gca acc tct     528
Ser Ser Glu Pro Ser Ser Ser Asp Ala Ala Ser Gly Asp Ala Thr Ser
                165                 170                 175

ggt gat gcc cct tct ggt gat gtg tcc cct ggt gat gcc acc tct ggt     576
Gly Asp Ala Pro Ser Gly Asp Val Ser Pro Gly Asp Ala Thr Ser Gly
            180                 185                 190

gat gcc act gct gat gat ctc tcc tct ggt gat ccc acc tct agt gat     624
Asp Ala Thr Ala Asp Asp Leu Ser Ser Gly Asp Pro Thr Ser Ser Asp
        195                 200                 205

ccc atc cca ggt gaa ccg gtc cct gtt gaa ccc att tct ggt gat tgt     672
Pro Ile Pro Gly Glu Pro Val Pro Val Glu Pro Ile Ser Gly Asp Cys
    210                 215                 220

gcc gct gat gat ata gcc tct agt gaa ata act tct gtt gat ctg gct     720
Ala Ala Asp Asp Ile Ala Ser Ser Glu Ile Thr Ser Val Asp Leu Ala
225                 230                 235                 240

tct gga gca cca gct tcc act gat cca gcc tct gat gat ctg gcc tct     768
Ser Gly Ala Pro Ala Ser Thr Asp Pro Ala Ser Asp Asp Leu Ala Ser
                245                 250                 255

ggt gat cta tcc tct agt gaa ctg gcc tct gat gat ctg gcc act ggt     816
Gly Asp Leu Ser Ser Ser Glu Leu Ala Ser Asp Asp Leu Ala Thr Gly
            260                 265                 270

gaa ctg gcc tct gat gag ctg act tct gaa tca acc ttt gat cgt acc     864
Glu Leu Ala Ser Asp Glu Leu Thr Ser Glu Ser Thr Phe Asp Arg Thr
        275                 280                 285

ttt gaa cca aag tct gta cca gtt tgt gaa cca gtt cct gaa att gac     912
Phe Glu Pro Lys Ser Val Pro Val Cys Glu Pro Val Pro Glu Ile Asp
    290                 295                 300

aat ata gaa cca agt agc aat aaa gat gat gat ttt ctt gaa aaa aat     960
Asn Ile Glu Pro Ser Ser Asn Lys Asp Asp Asp Phe Leu Glu Lys Asn
305                 310                 315                 320

gga gct gat gaa aaa tta gag caa att cag agt aaa gac tca ttg gat     1008
Gly Ala Asp Glu Lys Leu Glu Gln Ile Gln Ser Lys Asp Ser Leu Asp
                325                 330                 335

gag aaa aat aaa gct gat aat aat att gat gct aat gaa gaa act cta     1056
Glu Lys Asn Lys Ala Asp Asn Asn Ile Asp Ala Asn Glu Glu Thr Leu
            340                 345                 350

gaa aca gat gat aca act att tgt tca gat cga cct cct gaa aat gaa     1104
Glu Thr Asp Asp Thr Thr Ile Cys Ser Asp Arg Pro Pro Glu Asn Glu
        355                 360                 365

aag aag gta gag gaa gat att atc aca gag ctt gct ctt gga gaa gat     1152
Lys Lys Val Glu Glu Asp Ile Ile Thr Glu Leu Ala Leu Gly Glu Asp
    370                 375                 380

gct ata tct agc agt atg gaa att gac caa ggt gaa aag aat gaa gat     1200
Ala Ile Ser Ser Ser Met Glu Ile Asp Gln Gly Glu Lys Asn Glu Asp

385                 390                 395                 400

gaa act tct gca gat ctt gta gaa acg att aat gaa aat gtt att gaa      1248
Glu Thr Ser Ala Asp Leu Val Glu Thr Ile Asn Glu Asn Val Ile Glu
                405                 410                 415

gat aac aaa agt gag aat atc tta gaa aat aca gac tct atg gag aca      1296
Asp Asn Lys Ser Glu Asn Ile Leu Glu Asn Thr Asp Ser Met Glu Thr
            420                 425                 430

gat gaa atc att cct att ttg gaa aag ctt gca cct tct gag gat gaa      1344
Asp Glu Ile Ile Pro Ile Leu Glu Lys Leu Ala Pro Ser Glu Asp Glu
        435                 440                 445

ctt act tgc ttt tct aaa aca tct ctc ctt cca atc gat gag aca aat      1392
Leu Thr Cys Phe Ser Lys Thr Ser Leu Leu Pro Ile Asp Glu Thr Asn
    450                 455                 460

cca gat ttg gaa gag aaa atg gaa agt tct ttt ggt tca cca tct aaa      1440
Pro Asp Leu Glu Glu Lys Met Glu Ser Ser Phe Gly Ser Pro Ser Lys
465                 470                 475                 480

caa gaa agt agt gag agt ttg cca aaa gaa gcc ttt ctg gtc ctc tct      1488
Gln Glu Ser Ser Glu Ser Leu Pro Lys Glu Ala Phe Leu Val Leu Ser
                485                 490                 495

gat gaa gag gat att tcg ggt gaa aaa gat gag tct gaa gtt ata tcg      1536
Asp Glu Glu Asp Ile Ser Gly Glu Lys Asp Glu Ser Glu Val Ile Ser
            500                 505                 510

caa aat gaa acg tgc tct cca gca gaa gta gaa agt aat gaa aag gac      1584
Gln Asn Glu Thr Cys Ser Pro Ala Glu Val Glu Ser Asn Glu Lys Asp
        515                 520                 525

aac aaa cct gag gaa gaa gag caa gta ata cat gaa gat gat gaa aga      1632
Asn Lys Pro Glu Glu Glu Glu Gln Val Ile His Glu Asp Asp Glu Arg
    530                 535                 540

cct tct gag aaa aat gaa ttt tct aga cga aaa cgt tct aaa tca gaa      1680
Pro Ser Glu Lys Asn Glu Phe Ser Arg Arg Lys Arg Ser Lys Ser Glu
545                 550                 555                 560

gac atg gac aat gta cag tct aaa cgt cgt cga tat atg gaa gaa gaa      1728
Asp Met Asp Asn Val Gln Ser Lys Arg Arg Arg Tyr Met Glu Glu Glu
                565                 570                 575

tat gag gca gaa ttt caa gta aag att aca gcc aaa gga gac att aac      1776
Tyr Glu Ala Glu Phe Gln Val Lys Ile Thr Ala Lys Gly Asp Ile Asn
            580                 585                 590

cag aaa ctt caa aag gtt ata cag tgg ttg ctg gaa gaa aaa ttg tgt      1824
Gln Lys Leu Gln Lys Val Ile Gln Trp Leu Leu Glu Glu Lys Leu Cys
        595                 600                 605

gcg ctg cag tgt gct gta ttt gat aag act ttg gca gaa ttg aaa aca      1872
Ala Leu Gln Cys Ala Val Phe Asp Lys Thr Leu Ala Glu Leu Lys Thr
    610                 615                 620

cga gtg gaa aag att gaa tgt aac aag agg cat aaa aca gtt ctc act      1920
Arg Val Glu Lys Ile Glu Cys Asn Lys Arg His Lys Thr Val Leu Thr
625                 630                 635                 640

gaa cta cag gcc aag ata gcc agg tta acc aaa cgc ttt gaa gca gcc      1968

Glu Leu Gln Ala Lys Ile Ala Arg Leu Thr Lys Arg Phe Glu Ala Ala
                645                 650                 655

aaa gaa gat ctt aag aaa aga cat gaa cat cca ccc aac cca cca gta      2016
Lys Glu Asp Leu Lys Lys Arg His Glu His Pro Pro Asn Pro Pro Val
            660                 665                 670

tca cca gga aaa act gta aat gat gtc aac agc aat aat aac atg tct      2064
Ser Pro Gly Lys Thr Val Asn Asp Val Asn Ser Asn Asn Asn Met Ser
        675                 680                 685

tac aga aat gca ggc aca gtg aga cag atg ctg gag tcc aaa aga aat      2112
Tyr Arg Asn Ala Gly Thr Val Arg Gln Met Leu Glu Ser Lys Arg Asn
    690                 695                 700

gta agc gag agt gca cca cca tcc ttt caa act cct gtg aat aca gta      2160
Val Ser Glu Ser Ala Pro Pro Ser Phe Gln Thr Pro Val Asn Thr Val
705                 710                 715                 720

tct tca acc aat ctt gtc act cct cca gca gtt gtc agt agt caa cct      2208
Ser Ser Thr Asn Leu Val Thr Pro Pro Ala Val Val Ser Ser Gln Pro
                725                 730                 735

aaa ttg cag act cca gtg act tcg ggt tcc ctc aca gca acg tca gtt      2256
Lys Leu Gln Thr Pro Val Thr Ser Gly Ser Leu Thr Ala Thr Ser Val
            740                 745                 750

ctt cct gca ccc aat aca gct act gta gtt gct act act cag gtg cct      2304
Leu Pro Ala Pro Asn Thr Ala Thr Val Val Ala Thr Thr Gln Val Pro
        755                 760                 765

agt gga aat ccc cag cct aca atc tct tta cag cct ttg cca gtg att      2352
Ser Gly Asn Pro Gln Pro Thr Ile Ser Leu Gln Pro Leu Pro Val Ile
    770                 775                 780

ttg cat gta cct gtt gca gta tcc tcc cag cct cag ctt cta cag agc      2400
Leu His Val Pro Val Ala Val Ser Ser Gln Pro Gln Leu Leu Gln Ser
785                 790                 795                 800

cat cca ggg act ttg gtg act aat caa cca tct ggc aat gtt gaa ttc      2448
His Pro Gly Thr Leu Val Thr Asn Gln Pro Ser Gly Asn Val Glu Phe
                805                 810                 815

att tct gtg caa agc cca cct aca gtg agt ggt ctt acc aaa aat cca      2496
Ile Ser Val Gln Ser Pro Pro Thr Val Ser Gly Leu Thr Lys Asn Pro
            820                 825                 830

gta tcc ttg cca tcc ttg cca aat ccc act aaa cca aac aac gtt cct      2544
Val Ser Leu Pro Ser Leu Pro Asn Pro Thr Lys Pro Asn Asn Val Pro
        835                 840                 845

tct gtg ccc agt cct agt att caa agg aac cct act gcc agt gct gca      2592
Ser Val Pro Ser Pro Ser Ile Gln Arg Asn Pro Thr Ala Ser Ala Ala
    850                 855                 860

cca ttg gga aca aca ctt gct gtg cag gct gtt cca aca gca cac tct      2640
Pro Leu Gly Thr Thr Leu Ala Val Gln Ala Val Pro Thr Ala His Ser
865                 870                 875                 880

att gta caa gcc aca agg act tct tta ccc aca gtg ggc cca tca gga      2688
Ile Val Gln Ala Thr Arg Thr Ser Leu Pro Thr Val Gly Pro Ser Gly
                885                 890                 895

ctc tat agt cca tca act aat cga ggt cct ata cag atg aaa att cca 2736
Leu Tyr Ser Pro Ser Thr Asn Arg Gly Pro Ile Gln Met Lys Ile Pro
            900                 905                 910

att tct gca ttt agt act tcg tct gct gca gaa cag aac agc aat acc 2784
Ile Ser Ala Phe Ser Thr Ser Ser Ala Ala Glu Gln Asn Ser Asn Thr
        915                 920                 925

acc cca aga att gaa aac cag aca aac aaa aca ata gat gct tct gtc 2832
Thr Pro Arg Ile Glu Asn Gln Thr Asn Lys Thr Ile Asp Ala Ser Val
    930                 935                 940

agt aag aaa gca gct gat agc aca tca cag tgt gga aaa gcc act ggc 2880
Ser Lys Lys Ala Ala Asp Ser Thr Ser Gln Cys Gly Lys Ala Thr Gly
945                 950                 955                 960

agt gat tca agt ggt gtc att gat ctc aca atg gat gat gaa gag agt 2928
Ser Asp Ser Ser Gly Val Ile Asp Leu Thr Met Asp Asp Glu Glu Ser
                965                 970                 975

gga gct tca caa gac ccc aaa aaa cta aat cac act cct gta tca acc 2976
Gly Ala Ser Gln Asp Pro Lys Lys Leu Asn His Thr Pro Val Ser Thr
            980                 985                 990

atg agt tct tct cag cct gtg tca cga cca ttg caa ccc ata caa cca 3024
Met Ser Ser Ser Gln Pro Val Ser Arg Pro Leu Gln Pro Ile Gln Pro
        995                 1000                1005

gca ccg cct ctt caa cca tct ggg gtg cca aca agt gga cca tct 3069
Ala Pro Pro Leu Gln Pro Ser Gly Val Pro Thr Ser Gly Pro Ser
    1010                1015                1020

cag acc acc ata cac tta cta cct aca gct cca act acc gtg aat 3114
Gln Thr Thr Ile His Leu Leu Pro Thr Ala Pro Thr Thr Val Asn
    1025                1030                1035

gta aca cat cgt cca gta act cag gtg acc aca aga ctc cct gta 3159
Val Thr His Arg Pro Val Thr Gln Val Thr Thr Arg Leu Pro Val
    1040                1045                1050

cca aga gct cct gca aac cac cag gtg gtt tat aca act ctt cct 3204
Pro Arg Ala Pro Ala Asn His Gln Val Val Tyr Thr Thr Leu Pro
    1055                1060                1065

gca cca cca gct cag gct ccc ttg cga gga act gtt atg cag gct 3249
Ala Pro Pro Ala Gln Ala Pro Leu Arg Gly Thr Val Met Gln Ala
    1070                1075                1080

cct gct gtt cgg cag gtc aat ccc caa aat acc ttg ccc ttt aag 3294
Pro Ala Val Arg Gln Val Asn Pro Gln Asn Thr Leu Pro Phe Lys
    1085                1090                1095

gtg gtg gtg atc tca gcc atc ctg gcc ctg gtg gtg ctc acc atc 3339
Val Val Val Ile Ser Ala Ile Leu Ala Leu Val Val Leu Thr Ile
    1100                1105                1110

atc tcc ctt atc atc ctc atc atg ctt tgg cag aag aag cca cgt 3384
Ile Ser Leu Ile Ile Leu Ile Met Leu Trp Gln Lys Lys Pro Arg
    1115                1120                1125

tac gag atc cga tgg aag gtg att gag tct gtg agc tct gac ggc 3429
Tyr Glu Ile Arg Trp Lys Val Ile Glu Ser Val Ser Ser Asp Gly
    1130                1135                1140

cat gag tac atc tac gtg gac ccc atg cag ctg ccc tat gac tcc 3474
His Glu Tyr Ile Tyr Val Asp Pro Met Gln Leu Pro Tyr Asp Ser
    1145                1150                1155

acg tgg gag ctg ccg cgg gac cag ctt gtg ctg gga cgc acc ctc 3519
Thr Trp Glu Leu Pro Arg Asp Gln Leu Val Leu Gly Arg Thr Leu
    1160                1165                1170

ggc tct ggg gcc ttt ggg cag gtg gtg gag gcc acg gct cat ggc 3564
Gly Ser Gly Ala Phe Gly Gln Val Val Glu Ala Thr Ala His Gly
    1175                1180                1185

ctg agc cat tct cag gcc acg atg aaa gtg gcc gtc aag atg ctt 3609
Leu Ser His Ser Gln Ala Thr Met Lys Val Ala Val Lys Met Leu
    1190                1195                1200

aaa tcc aca gcc cgc agc agt gag aag caa gcc ctt atg tcg gag 3654
Lys Ser Thr Ala Arg Ser Ser Glu Lys Gln Ala Leu Met Ser Glu
    1205                1210                1215

ctg aag atc atg agt cac ctt ggg ccc cac ctg aac gtg gtc aac 3699
Leu Lys Ile Met Ser His Leu Gly Pro His Leu Asn Val Val Asn
    1220                1225                1230

ctg ttg ggg gcc tgc acc aaa gga gga ccc atc tat atc atc act 3744
Leu Leu Gly Ala Cys Thr Lys Gly Gly Pro Ile Tyr Ile Ile Thr
    1235                1240                1245

gag tac tgc cgc tac gga gac ctg gtg gac tac ctg cac cgc aac 3789
Glu Tyr Cys Arg Tyr Gly Asp Leu Val Asp Tyr Leu His Arg Asn
    1250                1255                1260

aaa cac acc ttc ctg cag cac cac tcc gac aag cgc cgc ccg ccc 3834
Lys His Thr Phe Leu Gln His His Ser Asp Lys Arg Arg Pro Pro
    1265                1270                1275

agc gcg gag ctc tac agc aat gct ctg ccc gtt ggg ctc ccc ctg 3879
Ser Ala Glu Leu Tyr Ser Asn Ala Leu Pro Val Gly Leu Pro Leu
    1280                1285                1290

ccc agc cat gtg tcc ttg acc ggg gag agc gac ggt ggc tac atg 3924
Pro Ser His Val Ser Leu Thr Gly Glu Ser Asp Gly Gly Tyr Met
    1295                1300                1305

gac atg agc aag gac gag tcg gtg gac tat gtg ccc atg ctg gac 3969
Asp Met Ser Lys Asp Glu Ser Val Asp Tyr Val Pro Met Leu Asp
    1310                1315                1320

atg aaa gga gac gtc aaa tat gca gac atc gag tcc tcc aac tac 4014
Met Lys Gly Asp Val Lys Tyr Ala Asp Ile Glu Ser Ser Asn Tyr
    1325                1330                1335

atg gcc cct tac gat aac tac gtt ccc tct gcc cct gag agg acc 4059
Met Ala Pro Tyr Asp Asn Tyr Val Pro Ser Ala Pro Glu Arg Thr
    1340                1345                1350

tgc cga gca act ttg atc aac gag tct cca gtg cta agc tac atg 4104
Cys Arg Ala Thr Leu Ile Asn Glu Ser Pro Val Leu Ser Tyr Met
    1355                1360                1365

gac ctc gtg ggc ttc agc tac cag gtg gcc aat ggc atg gag ttt 4149
Asp Leu Val Gly Phe Ser Tyr Gln Val Ala Asn Gly Met Glu Phe

1370                    1375                    1380

ctg gcc  tcc aag aac tgc gtc  cac aga gac ctg gcg  gct agg aac        4194
Leu Ala  Ser Lys Asn Cys Val  His Arg Asp Leu Ala  Ala Arg Asn
    1385                    1390                    1395

gtg ctc  atc tgt gaa ggc aag  ctg gtc aag atc tgt  gac ttt ggc        4239
Val Leu  Ile Cys Glu Gly Lys  Leu Val Lys Ile Cys  Asp Phe Gly
    1400                    1405                    1410

ctg gct  cga gac atc atg cgg  gac tcg aat tac atc  tcc aaa ggc        4284
Leu Ala  Arg Asp Ile Met Arg  Asp Ser Asn Tyr Ile  Ser Lys Gly
    1415                    1420                    1425

agc acc  ttt ttg cct tta aag  tgg atg gct ccg gag  agc atc ttc        4329
Ser Thr  Phe Leu Pro Leu Lys  Trp Met Ala Pro Glu  Ser Ile Phe
    1430                    1435                    1440

aac agc  ctc tac acc acc ctg  agc gac gtg tgg tcc  ttc ggg atc        4374
Asn Ser  Leu Tyr Thr Thr Leu  Ser Asp Val Trp Ser  Phe Gly Ile
    1445                    1450                    1455

ctg ctc  tgg gag atc ttc acc  ttg ggt ggc acc cct  tac cca gag        4419
Leu Leu  Trp Glu Ile Phe Thr  Leu Gly Gly Thr Pro  Tyr Pro Glu
    1460                    1465                    1470

ctg ccc  atg aac gag cag ttc  tac aat gcc atc aaa  cgg ggt tac        4464
Leu Pro  Met Asn Glu Gln Phe  Tyr Asn Ala Ile Lys  Arg Gly Tyr
    1475                    1480                    1485

cgc atg  gcc cag cct gcc cat  gcc tcc gac gag atc  tat gag atc        4509
Arg Met  Ala Gln Pro Ala His  Ala Ser Asp Glu Ile  Tyr Glu Ile
    1490                    1495                    1500

atg cag  aag tgc tgg gaa gag  aag ttt gag att cgg  ccc ccc ttc        4554
Met Gln  Lys Cys Trp Glu Glu  Lys Phe Glu Ile Arg  Pro Pro Phe
    1505                    1510                    1515

tcc cag  ctg gtg ctg ctt ctc  gag aga ctg ttg ggc  gaa ggt tac        4599
Ser Gln  Leu Val Leu Leu Leu  Glu Arg Leu Leu Gly  Glu Gly Tyr
    1520                    1525                    1530

aaa aag  aag tac cag cag gtg  gat gag gag ttt ctg  agg agt gac        4644
Lys Lys  Lys Tyr Gln Gln Val  Asp Glu Glu Phe Leu  Arg Ser Asp
    1535                    1540                    1545

cac cca  gcc atc ctt cgg tcc  cag gcc cgc ttg cct  ggg ttc cat        4689
His Pro  Ala Ile Leu Arg Ser  Gln Ala Arg Leu Pro  Gly Phe His
    1550                    1555                    1560

ggc ctc  cga tct ccc ctg gac  acc agc tcc gtc ctc  tat act gcc        4734
Gly Leu  Arg Ser Pro Leu Asp  Thr Ser Ser Val Leu  Tyr Thr Ala
    1565                    1570                    1575

gtg cag  ccc aat gag ggt gac  aac gac tat atc atc  ccc ctg cct        4779
Val Gln  Pro Asn Glu Gly Asp  Asn Asp Tyr Ile Ile  Pro Leu Pro
    1580                    1585                    1590

gac ccc  aaa ccc gag gtt gct  gac gag ggc cca ctg  gag ggt tcc        4824
Asp Pro  Lys Pro Glu Val Ala  Asp Glu Gly Pro Leu  Glu Gly Ser
    1595                    1600                    1605

ccc agc  cta gcc agc tcc acc  ctg aat gaa gtc aac  acc tcc tca        4869

Pro Ser Leu Ala Ser Ser Thr Leu Asn Glu Val Asn Thr Ser Ser
    1610                1615                1620

acc atc tcc tgt gac agc ccc ctg gag ccc cag gac gaa cca gag 4914
Thr Ile Ser Cys Asp Ser Pro Leu Glu Pro Gln Asp Glu Pro Glu
    1625                1630                1635

cca gag ccc cag ctt gag ctc cag gtg gag ccg gag cca gag ctg 4959
Pro Glu Pro Gln Leu Glu Leu Gln Val Glu Pro Glu Pro Glu Leu
    1640                1645                1650

gaa cag ttg ccg gat tcg ggg tgc cct gcg cct cgg gcg gaa gca 5004
Glu Gln Leu Pro Asp Ser Gly Cys Pro Ala Pro Arg Ala Glu Ala
    1655                1660                1665

gag gat agc ttc ctg tag 5022
Glu Asp Ser Phe Leu
    1670

<210> 2
<211> 1673
<212> PRT
<213> Homo sapiens

<400> 2

Met Asp Ser Leu Glu Glu Pro Gln Lys Lys Val Phe Lys Ala Arg Lys
1               5                   10                  15

Thr Met Arg Val Ser Asp Arg Gln Gln Leu Glu Ala Val Tyr Lys Val
            20                  25                  30

Lys Glu Glu Leu Leu Lys Thr Asp Val Lys Leu Leu Asn Gly Asn His
        35                  40                  45

Glu Asn Gly Asp Leu Asp Pro Thr Ser Pro Leu Glu Asn Met Asp Tyr
    50                  55                  60

Ile Lys Asp Lys Glu Glu Val Asn Gly Ile Glu Glu Ile Cys Phe Asp
65                  70                  75                  80

Pro Glu Gly Ser Lys Ala Glu Trp Lys Glu Thr Pro Cys Ile Leu Ser
                85                  90                  95

Val Asn Val Lys Asn Lys Gln Asp Asp Asp Leu Asn Cys Glu Pro Leu
            100                 105                 110

Ser Pro His Asn Ile Thr Pro Glu Pro Val Ser Lys Leu Pro Ala Glu
        115                 120                 125

Pro Val Ser Gly Asp Pro Ala Pro Gly Asp Leu Asp Ala Gly Asp Pro
    130                 135                 140

Ala Ser Gly Val Leu Ala Ser Gly Asp Ser Thr Ser Gly Asp Pro Thr
145                 150                 155                 160

Ser Ser Glu Pro Ser Ser Ser Asp Ala Ala Ser Gly Asp Ala Thr Ser
                165                 170                 175

Gly Asp Ala Pro Ser Gly Asp Val Ser Pro Gly Asp Ala Thr Ser Gly
            180                 185                 190

Asp Ala Thr Ala Asp Asp Leu Ser Ser Gly Asp Pro Thr Ser Ser Asp
        195                 200                 205

Pro Ile Pro Gly Glu Pro Val Pro Val Glu Pro Ile Ser Gly Asp Cys
    210                 215                 220

Ala Ala Asp Asp Ile Ala Ser Ser Glu Ile Thr Ser Val Asp Leu Ala
225                 230                 235                 240

Ser Gly Ala Pro Ala Ser Thr Asp Pro Ala Ser Asp Asp Leu Ala Ser
                245                 250                 255

Gly Asp Leu Ser Ser Ser Glu Leu Ala Ser Asp Asp Leu Ala Thr Gly
            260                 265                 270

Glu Leu Ala Ser Asp Glu Leu Thr Ser Glu Ser Thr Phe Asp Arg Thr
        275                 280                 285

Phe Glu Pro Lys Ser Val Pro Val Cys Glu Pro Val Pro Glu Ile Asp
    290                 295                 300

Asn Ile Glu Pro Ser Ser Asn Lys Asp Asp Asp Phe Leu Glu Lys Asn
305                 310                 315                 320

Gly Ala Asp Glu Lys Leu Glu Gln Ile Gln Ser Lys Asp Ser Leu Asp
                325                 330                 335

Glu Lys Asn Lys Ala Asp Asn Asn Ile Asp Ala Asn Glu Glu Thr Leu
            340                 345                 350

Glu Thr Asp Asp Thr Thr Ile Cys Ser Asp Arg Pro Pro Glu Asn Glu
        355                 360                 365

Lys Lys Val Glu Glu Asp Ile Ile Thr Glu Leu Ala Leu Gly Glu Asp
    370                 375                 380

Ala Ile Ser Ser Ser Met Glu Ile Asp Gln Gly Glu Lys Asn Glu Asp
385                 390                 395                 400

Glu Thr Ser Ala Asp Leu Val Glu Thr Ile Asn Glu Asn Val Ile Glu
                405                 410                 415

Asp Asn Lys Ser Glu Asn Ile Leu Glu Asn Thr Asp Ser Met Glu Thr
            420                 425                 430

Asp Glu Ile Ile Pro Ile Leu Glu Lys Leu Ala Pro Ser Glu Asp Glu
        435                 440                 445

Leu Thr Cys Phe Ser Lys Thr Ser Leu Leu Pro Ile Asp Glu Thr Asn
    450                 455                 460

Pro Asp Leu Glu Glu Lys Met Glu Ser Ser Phe Gly Ser Pro Ser Lys
465                 470                 475                 480

Gln Glu Ser Ser Glu Ser Leu Pro Lys Glu Ala Phe Leu Val Leu Ser
                485                 490                 495

Asp Glu Glu Asp Ile Ser Gly Glu Lys Asp Glu Ser Glu Val Ile Ser
            500                 505                 510

Gln Asn Glu Thr Cys Ser Pro Ala Glu Val Glu Ser Asn Glu Lys Asp
        515                 520                 525

Asn Lys Pro Glu Glu Glu Glu Gln Val Ile His Glu Asp Asp Glu Arg
    530                 535                 540

Pro Ser Glu Lys Asn Glu Phe Ser Arg Arg Lys Arg Ser Lys Ser Glu
545                 550                 555                 560

Asp Met Asp Asn Val Gln Ser Lys Arg Arg Arg Tyr Met Glu Glu Glu
                565                 570                 575

Tyr Glu Ala Glu Phe Gln Val Lys Ile Thr Ala Lys Gly Asp Ile Asn
            580                 585                 590

Gln Lys Leu Gln Lys Val Ile Gln Trp Leu Leu Glu Glu Lys Leu Cys
        595                 600                 605

Ala Leu Gln Cys Ala Val Phe Asp Lys Thr Leu Ala Glu Leu Lys Thr
    610                 615                 620

Arg Val Glu Lys Ile Glu Cys Asn Lys Arg His Lys Thr Val Leu Thr
625                 630                 635                 640

Glu Leu Gln Ala Lys Ile Ala Arg Leu Thr Lys Arg Phe Glu Ala Ala
                645                 650                 655

Lys Glu Asp Leu Lys Lys Arg His Glu His Pro Pro Asn Pro Pro Val
            660                 665                 670

Ser Pro Gly Lys Thr Val Asn Asp Val Asn Ser Asn Asn Asn Met Ser
        675                 680                 685

Tyr Arg Asn Ala Gly Thr Val Arg Gln Met Leu Glu Ser Lys Arg Asn
    690                 695                 700

Val Ser Glu Ser Ala Pro Pro Ser Phe Gln Thr Pro Val Asn Thr Val
705                 710                 715                 720

Ser Ser Thr Asn Leu Val Thr Pro Pro Ala Val Val Ser Ser Gln Pro
                725                 730                 735

Lys Leu Gln Thr Pro Val Thr Ser Gly Ser Leu Thr Ala Thr Ser Val
            740                 745                 750

Leu Pro Ala Pro Asn Thr Ala Thr Val Val Ala Thr Thr Gln Val Pro
        755                 760                 765

Ser Gly Asn Pro Gln Pro Thr Ile Ser Leu Gln Pro Leu Pro Val Ile
    770                 775                 780

Leu His Val Pro Val Ala Val Ser Ser Gln Pro Gln Leu Leu Gln Ser
785                 790                 795                 800

His Pro Gly Thr Leu Val Thr Asn Gln Pro Ser Gly Asn Val Glu Phe
                805                 810                 815

Ile Ser Val Gln Ser Pro Pro Thr Val Ser Gly Leu Thr Lys Asn Pro
            820                 825                 830

Val Ser Leu Pro Ser Leu Pro Asn Pro Thr Lys Pro Asn Asn Val Pro
        835                 840                 845

Ser Val Pro Ser Pro Ser Ile Gln Arg Asn Pro Thr Ala Ser Ala Ala
    850                 855                 860

Pro Leu Gly Thr Thr Leu Ala Val Gln Ala Val Pro Thr Ala His Ser
865                 870                 875                 880

Ile Val Gln Ala Thr Arg Thr Ser Leu Pro Thr Val Gly Pro Ser Gly
                885                 890                 895

Leu Tyr Ser Pro Ser Thr Asn Arg Gly Pro Ile Gln Met Lys Ile Pro

900                 905                 910

Ile Ser Ala Phe Ser Thr Ser Ser Ala Ala Glu Gln Asn Ser Asn Thr
        915                 920                 925

Thr Pro Arg Ile Glu Asn Gln Thr Asn Lys Thr Ile Asp Ala Ser Val
    930                 935                 940

Ser Lys Lys Ala Ala Asp Ser Thr Ser Gln Cys Gly Lys Ala Thr Gly
945                 950                 955                 960

Ser Asp Ser Ser Gly Val Ile Asp Leu Thr Met Asp Asp Glu Glu Ser
                965                 970                 975

Gly Ala Ser Gln Asp Pro Lys Lys Leu Asn His Thr Pro Val Ser Thr
            980                 985                 990

Met Ser Ser Ser Gln Pro Val Ser  Arg Pro Leu Gln Pro  Ile Gln Pro
        995                 1000                1005

Ala Pro  Pro Leu Gln Pro Ser  Gly Val Pro Thr Ser  Gly Pro Ser
    1010                1015                1020

Gln Thr  Thr Ile His Leu Leu  Pro Thr Ala Pro Thr  Thr Val Asn
    1025                1030                1035

Val Thr  His Arg Pro Val Thr  Gln Val Thr Thr Arg  Leu Pro Val
    1040                1045                1050

Pro Arg  Ala Pro Ala Asn His  Gln Val Val Tyr Thr  Thr Leu Pro
    1055                1060                1065

Ala Pro  Pro Ala Gln Ala Pro  Leu Arg Gly Thr Val  Met Gln Ala
    1070                1075                1080

Pro Ala  Val Arg Gln Val Asn  Pro Gln Asn Thr Leu  Pro Phe Lys
    1085                1090                1095

Val Val  Val Ile Ser Ala Ile  Leu Ala Leu Val Val  Leu Thr Ile
    1100                1105                1110

Ile Ser  Leu Ile Ile Leu Ile  Met Leu Trp Gln Lys  Lys Pro Arg
    1115                1120                1125

Tyr Glu  Ile Arg Trp Lys Val  Ile Glu Ser Val Ser  Ser Asp Gly
    1130                1135                1140

His Glu Tyr Ile Tyr Val Asp Pro Met Gln Leu Pro Tyr Asp Ser
    1145                1150                1155

Thr Trp Glu Leu Pro Arg Asp Gln Leu Val Leu Gly Arg Thr Leu
    1160                1165                1170

Gly Ser Gly Ala Phe Gly Gln Val Val Glu Ala Thr Ala His Gly
    1175                1180                1185

Leu Ser His Ser Gln Ala Thr Met Lys Val Ala Val Lys Met Leu
    1190                1195                1200

Lys Ser Thr Ala Arg Ser Ser Glu Lys Gln Ala Leu Met Ser Glu
    1205                1210                1215

Leu Lys Ile Met Ser His Leu Gly Pro His Leu Asn Val Val Asn
    1220                1225                1230

Leu Leu Gly Ala Cys Thr Lys Gly Gly Pro Ile Tyr Ile Ile Thr
    1235                1240                1245

Glu Tyr Cys Arg Tyr Gly Asp Leu Val Asp Tyr Leu His Arg Asn
    1250                1255                1260

Lys His Thr Phe Leu Gln His His Ser Asp Lys Arg Arg Pro Pro
    1265                1270                1275

Ser Ala Glu Leu Tyr Ser Asn Ala Leu Pro Val Gly Leu Pro Leu
    1280                1285                1290

Pro Ser His Val Ser Leu Thr Gly Glu Ser Asp Gly Gly Tyr Met
    1295                1300                1305

Asp Met Ser Lys Asp Glu Ser Val Asp Tyr Val Pro Met Leu Asp
    1310                1315                1320

Met Lys Gly Asp Val Lys Tyr Ala Asp Ile Glu Ser Ser Asn Tyr
    1325                1330                1335

Met Ala Pro Tyr Asp Asn Tyr Val Pro Ser Ala Pro Glu Arg Thr
    1340                1345                1350

Cys Arg Ala Thr Leu Ile Asn Glu Ser Pro Val Leu Ser Tyr Met
    1355                1360                1365

Asp Leu Val Gly Phe Ser Tyr Gln Val Ala Asn Gly Met Glu Phe
    1370                1375                1380

Leu Ala Ser Lys Asn Cys Val His Arg Asp Leu Ala Ala Arg Asn
    1385                1390                1395

Val Leu Ile Cys Glu Gly Lys Leu Val Lys Ile Cys Asp Phe Gly
    1400                1405                1410

Leu Ala Arg Asp Ile Met Arg Asp Ser Asn Tyr Ile Ser Lys Gly
    1415                1420                1425

Ser Thr Phe Leu Pro Leu Lys Trp Met Ala Pro Glu Ser Ile Phe
    1430                1435                1440

Asn Ser Leu Tyr Thr Thr Leu Ser Asp Val Trp Ser Phe Gly Ile
    1445                1450                1455

Leu Leu Trp Glu Ile Phe Thr Leu Gly Gly Thr Pro Tyr Pro Glu
    1460                1465                1470

Leu Pro Met Asn Glu Gln Phe Tyr Asn Ala Ile Lys Arg Gly Tyr
    1475                1480                1485

Arg Met Ala Gln Pro Ala His Ala Ser Asp Glu Ile Tyr Glu Ile
    1490                1495                1500

Met Gln Lys Cys Trp Glu Glu Lys Phe Glu Ile Arg Pro Pro Phe
    1505                1510                1515

Ser Gln Leu Val Leu Leu Leu Glu Arg Leu Leu Gly Glu Gly Tyr
    1520                1525                1530

Lys Lys Lys Tyr Gln Gln Val Asp Glu Glu Phe Leu Arg Ser Asp
    1535                1540                1545

His Pro Ala Ile Leu Arg Ser Gln Ala Arg Leu Pro Gly Phe His
    1550                1555                1560

Gly Leu Arg Ser Pro Leu Asp Thr Ser Ser Val Leu Tyr Thr Ala
    1565                1570                1575

Val Gln Pro Asn Glu Gly Asp Asn Asp Tyr Ile Ile Pro Leu Pro
    1580                1585                1590

Asp Pro Lys Pro Glu Val Ala Asp Glu Gly Pro Leu Glu Gly Ser
    1595                1600                1605

Pro Ser Leu Ala Ser Ser Thr Leu Asn Glu Val Asn Thr Ser Ser
    1610                1615                1620

Thr Ile Ser Cys Asp Ser Pro Leu Glu Pro Gln Asp Glu Pro Glu
    1625                1630                1635

Pro Glu Pro Gln Leu Glu Leu Gln Val Glu Pro Glu Pro Glu Leu
    1640                1645                1650

Glu Gln Leu Pro Asp Ser Gly Cys Pro Ala Pro Arg Ala Glu Ala
    1655                1660                1665

Glu Asp Ser Phe Leu
    1670

<210> 3
<211> 5046
<212> DNA
<213> Homo sapiens

<220>
<221> CDS
<222> (1)..(5046)

<400> 3
atg cat cag gac cag aga ttc aga atg gac agt tta gaa gaa cct cag 48
aaa aaa gtc ttt aag gct cga aaa acg atg aga gtg agt gat cgt cag 96
caa ctt gaa gca gtg tac aag gtc aaa gaa gaa ctg ttg aaa act gat 144
gtc aag ctg tta aat ggc aac cat gaa aat gga gat ttg gac cca acc 192
tca cct ttg gaa aac atg gat tac att aaa gac aag gaa gag gtg aat 240
ggc att gaa gag att tgt ttt gat cct gaa gga agt aaa gca gaa tgg 288
aag gaa aca ccc tgt atc cta agt gtt aat gta aaa aac aag cag gat 336
gat gat tta aat tgt gaa cct ttg tct ccc cat aat ata act cca gaa 384
cca gtc tct aaa ctg cct gct gaa cca gtt tct ggt gat cca gcc cct 432
ggt gat ctg gat gcc gga gat cca gcc tcc gga gta ctg gcc tct ggt      480
gat tcc acc tct ggt gat ccc acc tct agc gag ccc tcc tct agt gat      528
gct gcc tct ggt gat gca acc tct ggt gat gcc cct tct ggt gat gtg      576
tcc cct ggt gat gcc acc tct ggt gat gcc act gct gat gat ctc tcc      624
tct ggt gat ccc acc tct agt gat ccc atc cca ggt gaa ccg gtc cct      672
gtt gaa ccc att tct ggt gat tgt gcc gct gat gat ata gcc tct agt      720
gaa ata act tct gtt gat ctg gct tct gga gca cca gct tcc act gat      768
cca gcc tct gat gat ctg gcc tct ggt gat cta tcc tct agt gaa ctg      816
gcc tct gat gat ctg gcc act ggt gaa ctg gcc tct gat gag ctg act      864
tct gaa tca acc ttt gat cgt acc ttt gaa cca aag tct gta cca gtt      912
tgt gaa cca gtt cct gaa att gac aat ata gaa cca agt agc aat aaa      960
gat gat gat ttt ctt gaa aaa aat gga gct gat gaa aaa tta gag caa     1008
att cag agt aaa gac tca ttg gat gag aaa aat aaa gct gat aat aat     1056
att gat gct aat gaa gaa act cta gaa aca gat gat aca act att tgt     1104
tca gat cga cct cct gaa aat gaa aag aag gta gag gaa gat att atc     1152
aca gag ctt gct ctt gga gaa gat gct ata tct agc agt atg gaa att     1200
gac caa ggt gaa aag aat gaa gat gaa act tct gca gat ctt gta gaa      1248
acg att aat gaa aat gtt att gaa gat aac aaa agt gag aat atc tta      1296
gaa aat aca gac tct atg gag aca gat gaa atc att cct att ttg gaa      1344
aag ctt gca cct tct gag gat gaa ctt act tgc ttt tct aaa aca tct      1392
ctc ctt cca atc gat gag aca aat cca gat ttg gaa gag aaa atg gaa      1440
agt tct ttt ggt tca cca tct aaa caa gaa agt agt gag agt ttg cca      1488
aaa gaa gcc ttt ctg gtc ctc tct gat gaa gag gat att tcg ggt gaa      1536
aaa gat gag tct gaa gtt ata tcg caa aat gaa acg tgc tct cca gca      1584
gaa gta gaa agt aat gaa aag gac aac aaa cct gag gaa gaa gag caa      1632
gta ata cat gaa gat gat gaa aga cct tct gag aaa aat gaa ttt tct      1680
aga cga aaa cgt tct aaa tca gaa gac atg gac aat gta cag tct aaa      1728
cgt cgt cga tat atg gaa gaa gaa tat gag gca gaa ttt caa gta aag      1776
att aca gcc aaa gga gac att aac cag aaa ctt caa aag gtt ata cag      1824
tgg ttg ctg gaa gaa aaa ttg tgt gcg ctg cag tgt gct gta ttt gat      1872
aag act ttg gca gaa ttg aaa aca cga gtg gaa aag att gaa tgt aac      1920
aag agg cat aaa aca gtt ctc act gaa cta cag gcc aag ata gcc agg 1968
tta acc aaa cgc ttt gaa gca gcc aaa gaa gat ctt aag aaa aga cat 2016
gaa cat cca ccc aac cca cca gta tca cca gga aaa act gta aat gat 2064
gtc aac agc aat aat aac atg tct tac aga aat gca ggc aca gtg aga 2112
cag atg ctg gag tcc aaa aga aat gta agc gag agt gca cca cca tcc 2160
ttt caa act cct gtg aat aca gta tct tca acc aat ctt gtc act cct 2208
cca gca gtt gtc agt agt caa cct aaa ttg cag act cca gtg act tcg 2256
ggt tcc ctc aca gca acg tca gtt ctt cct gca ccc aat aca gct act 2304
gta gtt gct act act cag gtg cct agt gga aat ccc cag cct aca atc 2352
tct tta cag cct ttg cca gtg att ttg cat gta cct gtt gca gta tcc 2400
tcc cag cct cag ctt cta cag agc cat cca ggg act ttg gtg act aat 2448
caa cca tct ggc aat gtt gaa ttc att tct gtg caa agc cca cct aca 2496
gtg agt ggt ctt acc aaa aat cca gta tcc ttg cca tcc ttg cca aat 2544
ccc act aaa cca aac aac gtt cct tct gtg ccc agt cct agt att caa 2592
agg aac cct act gcc agt gct gca cca ttg gga aca aca ctt gct gtg 2640
cag gct gtt cca aca gca cac tct att gta caa gcc aca agg act tct 2688
tta ccc aca gtg ggc cca tca gga ctc tat agt cca tca act aat cga 2736
ggt cct ata cag atg aaa att cca att tct gca ttt agt act tcg tct 2784
gct gca gaa cag aac agc aat acc acc cca aga att gaa aac cag aca 2832
aac aaa aca ata gat gct tct gtc agt aag aaa gca gct gat agc aca 2880
tca cag tgt gga aaa gcc act ggc agt gat tca agt ggt gtc att gat 2928
ctc aca atg gat gat gaa gag agt gga gct tca caa gac ccc aaa aaa 2976
cta aat cac act cct gta tca acc atg agt tct tct cag cct gtg tca 3024
cga cca ttg caa ccc ata caa cca gca ccg cct ctt caa cca tct 3069
ggg gtg cca aca agt gga cca tct cag acc acc ata cac tta cta 3114
cct aca gct cca act acc gtg aat gta aca cat cgt cca gta act 3159
cag gtg acc aca aga ctc cct gta cca aga gct cct gca aac cac 3204
cag gtg gtt tat aca act ctt cct gca cca cca gct cag gct ccc 3249
ttg cga gga act gtt atg cag gct cct gct gtt cgg cag gtc aat 3294
ccc caa aat acc ttg ccc ttt aag gtg gtg gtg atc tca gcc atc 3339
ctg gcc ctg gtg gtg ctc acc atc atc tcc ctt atc atc ctc atc 3384
atg ctt tgg cag aag aag cca cgt tac gag atc cga tgg aag gtg 3429
att gag tct gtg agc tct gac ggc cat gag tac atc tac gtg gac        3474
ccc atg cag ctg ccc tat gac tcc acg tgg gag ctg ccg cgg gac        3519
cag ctt gtg ctg gga cgc acc ctc ggc tct ggg gcc ttt ggg cag        3564
gtg gtg gag gcc acg gct cat ggc ctg agc cat tct cag gcc acg        3609
atg aaa gtg gcc gtc aag atg ctt aaa tcc aca gcc cgc agc agt        3654
gag aag caa gcc ctt atg tcg gag ctg aag atc atg agt cac ctt        3699
ggg ccc cac ctg aac gtg gtc aac ctg ttg ggg gcc tgc acc aaa        3744
gga gga ccc atc tat atc atc act gag tac tgc cgc tac gga gac        3789
ctg gtg gac tac ctg cac cgc aac aaa cac acc ttc ctg cag cac        3834
cac tcc gac aag cgc cgc ccg ccc agc gcg gag ctc tac agc aat        3879
gct ctg ccc gtt ggg ctc ccc ctg ccc agc cat gtg tcc ttg acc        3924
ggg gag agc gac ggt ggc tac atg gac atg agc aag gac gag tcg        3969
gtg gac tat gtg ccc atg ctg gac atg aaa gga gac gtc aaa tat        4014
gca gac atc gag tcc tcc aac tac atg gcc cct tac gat aac tac        4059
gtt ccc tct gcc cct gag agg acc tgc cga gca act ttg atc aac        4104
gag tct cca gtg cta agc tac atg gac ctc gtg ggc ttc agc tac        4149
cag gtg gcc aat ggc atg gag ttt ctg gcc tcc aag aac tgc gtc      4194
cac aga gac ctg gcg gct agg aac gtg ctc atc tgt gaa ggc aag      4239
ctg gtc aag atc tgt gac ttt ggc ctg gct cga gac atc atg cgg      4284
gac tcg aat tac atc tcc aaa ggc agc acc ttt ttg cct tta aag      4329
tgg atg gct ccg gag agc atc ttc aac agc ctc tac acc acc ctg      4374
agc gac gtg tgg tcc ttc ggg atc ctg ctc tgg gag atc ttc acc      4419
ttg ggt ggc acc cct tac cca gag ctg ccc atg aac gag cag ttc      4464
tac aat gcc atc aaa cgg ggt tac cgc atg gcc cag cct gcc cat      4509
gcc tcc gac gag atc tat gag atc atg cag aag tgc tgg gaa gag      4554
aag ttt gag att cgg ccc ccc ttc tcc cag ctg gtg ctg ctt ctc      4599
gag aga ctg ttg ggc gaa ggt tac aaa aag aag tac cag cag gtg      4644
gat gag gag ttt ctg agg agt gac cac cca gcc atc ctt cgg tcc      4689
cag gcc cgc ttg cct ggg ttc cat ggc ctc cga tct ccc ctg gac      4734
acc agc tcc gtc ctc tat act gcc gtg cag ccc aat gag ggt gac      4779
aac gac tat atc atc ccc ctg cct gac ccc aaa ccc gag gtt gct      4824
gac gag ggc cca ctg gag ggt tcc ccc agc cta gcc agc tcc acc 4869
ctg aat gaa gtc aac acc tcc tca acc atc tcc tgt gac agc ccc 4914
ctg gag ccc cag gac gaa cca gag cca gag ccc cag ctt gag ctc 4959
cag gtg gag ccg gag cca gag ctg gaa cag ttg ccg gat tcg ggg 5004
tgc cct gcg cct cgg gcg gaa gca gag gat agc ttc ctg tag 5046

<210> 4
<211> 1681
<212> PRT
<213> Homo sapiens

<400> 4

<210>  5
<211>  4661
<212>  DNA
<213>  Homo sapiens

<220>
<221>  CDS
<222>  (159)..(3971)

<400>  5
   tttttgaatc tgcggaggcg gcggcggtgg cagcggcggc gcggcgactg aagcgcgcga      60
   aaagctgagg cggcaacgtc ggggacggct gcgcgggacg gctctgtagg aaggaacttg     120
   gttccccctc cctcagcttc cgccccaaaa gattcaga atg gac agt tta gaa gaa     176
                                             Met Asp Ser Leu Glu Glu
                                             1               5
   cct cag aaa aaa gtc ttt aag gct cga aaa acg atg aga gtg agt gat      224
   Pro Gln Lys Lys Val Phe Lys Ala Arg Lys Thr Met Arg Val Ser Asp
               10                  15                  20
   cgt cag caa ctt gaa gca gtg tac aag gtc aaa gaa gaa ctg ttg aaa      272
   Arg Gln Gln Leu Glu Ala Val Tyr Lys Val Lys Glu Glu Leu Leu Lys
           25                  30                  35
   act gat gtc aag ctg tta aat ggc aac cat gaa aat gga gat ttg gac      320
   Thr Asp Val Lys Leu Leu Asn Gly Asn His Glu Asn Gly Asp Leu Asp
       40                  45                  50
   cca acc tca cct ttg gaa aac atg gat tac att aaa gac aag gaa gag      368
   Pro Thr Ser Pro Leu Glu Asn Met Asp Tyr Ile Lys Asp Lys Glu Glu
   55                  60                  65                  70
   gtg aat ggc att gaa gag att tgt ttt gat cct gaa gga agt aaa gca      416
   Val Asn Gly Ile Glu Glu Ile Cys Phe Asp Pro Glu Gly Ser Lys Ala
                   75                  80                  85
   gaa tgg aag gaa aca ccc tgt atc cta agt gtt aat gta aaa aac aag      464
   Glu Trp Lys Glu Thr Pro Cys Ile Leu Ser Val Asn Val Lys Asn Lys
               90                  95                  100
   cag gat gat gat tta aat tgt gaa cct ttg tct ccc cat aat ata act      512
   Gln Asp Asp Asp Leu Asn Cys Glu Pro Leu Ser Pro His Asn Ile Thr
           105                 110                 115
   cca gaa cca gtc tct aaa ctg cct gct gaa cca gtt tct ggt gat cca      560
   Pro Glu Pro Val Ser Lys Leu Pro Ala Glu Pro Val Ser Gly Asp Pro
       120                 125                 130
   gcc cct ggt gat ctg gat gcc gga gat cca gcc tcc gga gta ctg gcc      608
   Ala Pro Gly Asp Leu Asp Ala Gly Asp Pro Ala Ser Gly Val Leu Ala
   135                 140                 145                 150
   tct ggt gat tcc acc tct ggt gat ccc acc tct agc gag ccc tcc tct      656
   Ser Gly Asp Ser Thr Ser Gly Asp Pro Thr Ser Ser Glu Pro Ser Ser
                   155                 160                 165
   agt gat gct gcc tct ggt gat gca acc tct ggt gat gcc cct tct ggt      704
   Ser Asp Ala Ala Ser Gly Asp Ala Thr Ser Gly Asp Ala Pro Ser Gly
               170                 175                 180
   gat gtg tcc cct ggt gat gcc acc tct ggt gat gcc act gct gat gat      752
   Asp Val Ser Pro Gly Asp Ala Thr Ser Gly Asp Ala Thr Ala Asp Asp
           185                 190                 195
   ctc tcc tct ggt gat ccc acc tct agt gat ccc atc cca ggt gaa ccg      800
   Leu Ser Ser Gly Asp Pro Thr Ser Ser Asp Pro Ile Pro Gly Glu Pro
       200                 205                 210
   gtc cct gtt gaa ccc att tct ggt gat tgt gcc gct gat gat ata gcc      848
   Val Pro Val Glu Pro Ile Ser Gly Asp Cys Ala Ala Asp Asp Ile Ala
   215                 220                 225                 230
   tct agt gaa ata act tct gtt gat ctg gct tct gga gca cca gct tcc      896
   Ser Ser Glu Ile Thr Ser Val Asp Leu Ala Ser Gly Ala Pro Ala Ser
                   235                 240                 245
   act gat cca gcc tct gat gat ctg gcc tct ggt gat cta tcc tct agt      944
   Thr Asp Pro Ala Ser Asp Asp Leu Ala Ser Gly Asp Leu Ser Ser Ser
               250                 255                 260
   gaa ctg gcc tct gat gat ctg gcc act ggt gaa ctg gcc tct gat gag      992
   Glu Leu Ala Ser Asp Asp Leu Ala Thr Gly Glu Leu Ala Ser Asp Glu
           265                 270                 275
   ctg act tct gaa tca acc ttt gat cgt acc ttt gaa cca aag tct gta     1040
   Leu Thr Ser Glu Ser Thr Phe Asp Arg Thr Phe Glu Pro Lys Ser Val
       280                 285                 290
   cca gtt tgt gaa cca gtt cct gaa att gac aat ata gaa cca agt agc     1088
   Pro Val Cys Glu Pro Val Pro Glu Ile Asp Asn Ile Glu Pro Ser Ser
   295                 300                 305                 310
   aat aaa gat gat gat ttt ctt gaa aaa aat gga gct gat gaa aaa tta     1136
   Asn Lys Asp Asp Asp Phe Leu Glu Lys Asn Gly Ala Asp Glu Lys Leu
                   315                 320                 325
   gag caa att cag agt aaa gac tca ttg gat gag aaa aat aaa gct gat     1184
   Glu Gln Ile Gln Ser Lys Asp Ser Leu Asp Glu Lys Asn Lys Ala Asp
               330                 335                 340
   aat aat att gat gct aat gaa gaa act cta gaa aca gat gat aca act     1232
   Asn Asn Ile Asp Ala Asn Glu Glu Thr Leu Glu Thr Asp Asp Thr Thr
           345                 350                 355
   att tgt tca gat cga cct cct gaa aat gaa aag aag gta gag gaa gat 1280
   Ile Cys Ser Asp Arg Pro Pro Glu Asn Glu Lys Lys Val Glu Glu Asp
   360 365 370
   att atc aca gag ctt gct ctt gga gaa gat gct ata tct agc agt atg 1328
   Ile Ile Thr Glu Leu Ala Leu Gly Glu Asp Ala Ile Ser Ser Ser Met
   375 380 385 390
   gaa att gac caa ggt gaa aag aat gaa gat gaa act tct gca gat ctt 1376
   Glu Ile Asp Gln Gly Glu Lys Asn Glu Asp Glu Thr Ser Ala Asp Leu
   395 400 405
   gta gaa acg att aat gaa aat gtt att gaa gat aac aaa agt gag aat 1424
   Val Glu Thr Ile Asn Glu Asn Val Ile Glu Asp Asn Lys Ser Glu Asn
   410 415 420
   atc tta gaa aat aca gac tct atg gag aca gat gaa atc att cct att 1472
   Ile Leu Glu Asn Thr Asp Ser Met Glu Thr Asp Glu Ile Ile Pro Ile
   425 430 435
   ttg gaa aag ctt gca cct tct gag gat gaa ctt act tgc ttt tct aaa 1520
   Leu Glu Lys Leu Ala Pro Ser Glu Asp Glu Leu Thr Cys Phe Ser Lys
   440 445 450
   aca tct ctc ctt cca atc gat gag aca aat cca gat ttg gaa gag aaa 1568
   Thr Ser Leu Leu Pro Ile Asp Glu Thr Asn Pro Asp Leu Glu Glu Lys
   455 460 465 470
   atg gaa agt tct ttt ggt tca cca tct aaa caa gaa agt agt gag agt 1616
   Met Glu Ser Ser Phe Gly Ser Pro Ser Lys Gln Glu Ser Ser Glu Ser
   475 480 485
   ttg cca aaa gaa gcc ttt ctg gtc ctc tct gat gaa gag gat att tcg 1664
   Leu Pro Lys Glu Ala Phe Leu Val Leu Ser Asp Glu Glu Asp Ile Ser
   490 495 500
   ggt gaa aaa gat gag tct gaa gtt ata tcg caa aat gaa acg tgc tct 1712
   Gly Glu Lys Asp Glu Ser Glu Val Ile Ser Gln Asn Glu Thr Cys Ser
   505 510 515
   cca gca gaa gta gaa agt aat gaa aag gac aac aaa cct gag gaa gaa 1760
   Pro Ala Glu Val Glu Ser Asn Glu Lys Asp Asn Lys Pro Glu Glu Glu
   520 525 530
   gag caa gta ata cat gaa gat gat gaa aga cct tct gag aaa aat gaa 1808
   Glu Gln Val Ile His Glu Asp Asp Glu Arg Pro Ser Glu Lys Asn Glu
   535 540 545 550
   ttt tct aga cga aaa cgt tct aaa tca gaa gac atg gac aat gta cag 1856
   Phe Ser Arg Arg Lys Arg Ser Lys Ser Glu Asp Met Asp Asn Val Gln
   555 560 565
   tct aaa cgt cgt cga tat atg gaa gaa gaa tat gag gca gaa ttt caa 1904
   Ser Lys Arg Arg Arg Tyr Met Glu Glu Glu Tyr Glu Ala Glu Phe Gln
   570 575 580
   gta aag att aca gcc aaa gga gac att aac cag aaa ctt caa aag gtt 1952
   Val Lys Ile Thr Ala Lys Gly Asp Ile Asn Gln Lys Leu Gln Lys Val
   585 590 595
   ata cag tgg ttg ctg gaa gaa aaa ttg tgt gcg ctg cag tgt gct gta 2000
   Ile Gln Trp Leu Leu Glu Glu Lys Leu Cys Ala Leu Gln Cys Ala Val
   600 605 610
   ttt gat aag act ttg gca gaa ttg aaa aca cga gtg gaa aag att gaa 2048
   Phe Asp Lys Thr Leu Ala Glu Leu Lys Thr Arg Val Glu Lys Ile Glu
   615 620 625 630
   tgt aac aag agg cat aaa aca gtt ctc act gaa cta cag gcc aag ata 2096
   Cys Asn Lys Arg His Lys Thr Val Leu Thr Glu Leu Gln Ala Lys Ile
   635 640 645
   gcc agg tta acc aaa cgc ttt gaa gca gcc aaa gaa gat ctt aag aaa 2144
   Ala Arg Leu Thr Lys Arg Phe Glu Ala Ala Lys Glu Asp Leu Lys Lys
   650 655 660
   aga cat gaa cat cca ccc aac cca cca gta tca cca gga aaa act gta 2192
   Arg His Glu His Pro Pro Asn Pro Pro Val Ser Pro Gly Lys Thr Val
   665 670 675
   aat gat gtc aac agc aat aat aac atg tct tac aga aat gca ggc aca 2240
   Asn Asp Val Asn Ser Asn Asn Asn Met Ser Tyr Arg Asn Ala Gly Thr
   680 685 690
   gtg aga cag atg ctg gag tcc aaa aga aat gta agc gag agt gca cca 2288
   Val Arg Gln Met Leu Glu Ser Lys Arg Asn Val Ser Glu Ser Ala Pro
   695 700 705 710
   cca tcc ttt caa act cct gtg aat aca gta tct tca acc aat ctt gtc 2336
   Pro Ser Phe Gln Thr Pro Val Asn Thr Val Ser Ser Thr Asn Leu Val
   715 720 725
   act cct cca gca gtt gtc agt agt caa cct aaa ttg cag act cca gtg 2384
   Thr Pro Pro Ala Val Val Ser Ser Gln Pro Lys Leu Gln Thr Pro Val
   730 735 740
   act tcg ggt tcc ctc aca gca acg tca gtt ctt cct gca ccc aat aca 2432
   Thr Ser Gly Ser Leu Thr Ala Thr Ser Val Leu Pro Ala Pro Asn Thr
   745 750 755
   gct act gta gtt gct act act cag gtg cct agt gga aat ccc cag cct 2480
   Ala Thr Val Val Ala Thr Thr Gln Val Pro Ser Gly Asn Pro Gln Pro
   760 765 770
   aca atc tct tta cag cct ttg cca gtg att ttg cat gta cct gtt gca 2528
   Thr Ile Ser Leu Gln Pro Leu Pro Val Ile Leu His Val Pro Val Ala
   775 780 785 790
   gta tcc tcc cag cct cag ctt cta cag agc cat cca ggg act ttg gtg 2576
   Val Ser Ser Gln Pro Gln Leu Leu Gln Ser His Pro Gly Thr Leu Val
   795 800 805
   act aat caa cca tct ggc aat gtt gaa ttc att tct gtg caa agc cca 2624
   Thr Asn Gln Pro Ser Gly Asn Val Glu Phe Ile Ser Val Gln Ser Pro
   810 815 820
   cct aca gtg agt ggt ctt acc aaa aat cca gta tcc ttg cca tcc ttg 2672
   Pro Thr Val Ser Gly Leu Thr Lys Asn Pro Val Ser Leu Pro Ser Leu
   825 830 835
   cca aat ccc act aaa cca aac aac gtt cct tct gtg ccc agt cct agt 2720
   Pro Asn Pro Thr Lys Pro Asn Asn Val Pro Ser Val Pro Ser Pro Ser
   840 845 850
   att caa agg aac cct act gcc agt gct gca cca ttg gga aca aca ctt 2768
   Ile Gln Arg Asn Pro Thr Ala Ser Ala Ala Pro Leu Gly Thr Thr Leu
   855                 860                 865                 870
   gct gtg cag gct gtt cca aca gca cac tct att gta caa gcc aca agg       2816
   Ala Val Gln Ala Val Pro Thr Ala His Ser Ile Val Gln Ala Thr Arg
                   875                 880                 885
   act tct tta ccc aca gtg ggc cca tca gga ctc tat agt cca tca act       2864
   Thr Ser Leu Pro Thr Val Gly Pro Ser Gly Leu Tyr Ser Pro Ser Thr
               890                 895                 900
   aat cga ggt cct ata cag atg aaa att cca att tct gca ttt agt act       2912
   Asn Arg Gly Pro Ile Gln Met Lys Ile Pro Ile Ser Ala Phe Ser Thr
           905                 910                 915
   tcg tct gct gca gaa cag aac agc aat acc acc cca aga att gaa aac       2960
   Ser Ser Ala Ala Glu Gln Asn Ser Asn Thr Thr Pro Arg Ile Glu Asn
       920                 925                 930
   cag aca aac aaa aca ata gat gct tct gtc agt aag aaa gca gct gat       3008
   Gln Thr Asn Lys Thr Ile Asp Ala Ser Val Ser Lys Lys Ala Ala Asp
   935                 940                 945                 950
   agc aca tca cag tgt gga aaa gcc act ggc agt gat tca agt ggt gtc       3056
   Ser Thr Ser Gln Cys Gly Lys Ala Thr Gly Ser Asp Ser Ser Gly Val
                   955                 960                 965
   att gat ctc aca atg gat gat gaa gag agt gga gct tca caa gac ccc       3104
   Ile Asp Leu Thr Met Asp Asp Glu Glu Ser Gly Ala Ser Gln Asp Pro
               970                 975                 980
   aaa aaa cta aat cac act cct gta tca acc atg agt tct tct cag cct       3152
   Lys Lys Leu Asn His Thr Pro Val Ser Thr Met Ser Ser Ser Gln Pro
           985                 990                 995
   gtg tca  cga cca ttg caa ccc  ata caa cca gca ccg  cct ctt caa       3197
   Val Ser  Arg Pro Leu Gln Pro  Ile Gln Pro Ala Pro  Pro Leu Gln
       1000                1005                1010
   cca tct  ggg gtg cca aca agt  gga cca tct cag acc  acc ata cac       3242
   Pro Ser  Gly Val Pro Thr Ser  Gly Pro Ser Gln Thr  Thr Ile His
       1015                1020                1025
   tta cta  cct aca gct cca act  acc gtg aat gta aca  cat cgt cca       3287
   Leu Leu  Pro Thr Ala Pro Thr  Thr Val Asn Val Thr  His Arg Pro
       1030                1035                1040
   gta act  cag gtg acc aca aga  ctc cct gta cca aga  gct cct gca       3332
   Val Thr  Gln Val Thr Thr Arg  Leu Pro Val Pro Arg  Ala Pro Ala
       1045                1050                1055
   aac cac  cag gtg gtt tat aca  act ctt cct gca cca  cca gct cag       3377
   Asn His  Gln Val Val Tyr Thr  Thr Leu Pro Ala Pro  Pro Ala Gln
       1060                1065                1070
   gct ccc  ttg cga gga act gtt  atg cag gct cct gct  gtt cgg cag       3422
   Ala Pro  Leu Arg Gly Thr Val  Met Gln Ala Pro Ala  Val Arg Gln
       1075                1080                1085
   gtc aat  ccc caa aat agt gtt  aca gtt cga gtg cct  caa aca acc       3467
   Val Asn  Pro Gln Asn Ser Val  Thr Val Arg Val Pro  Gln Thr Thr
       1090                1095                1100
   aca tat  gtt gta aac aat gga  cta acc ctg gga tca  aca gga cct       3512
   Thr Tyr Val Val Asn Asn Gly Leu Thr Leu Gly Ser Thr Gly Pro
   1105 1110 1115
   cag ctc aca gtg cat cac cga cca cca caa gtg cat act gag ccc 3557
   Gln Leu Thr Val His His Arg Pro Pro Gln Val His Thr Glu Pro
   1120 1125 1130
   cca cgc ccc gtg cac cca gca ccc tta cca gaa gct cca caa cca 3602
   Pro Arg Pro Val His Pro Ala Pro Leu Pro Glu Ala Pro Gln Pro
   1135 1140 1145
   cag cgt ctg ccc cca gaa gct gcc agc aca tct ctg cct cag aag 3647
   Gln Arg Leu Pro Pro Glu Ala Ala Ser Thr Ser Leu Pro Gln Lys
   1150 1155 1160
   cca cac ttg aag tta gca cgc gtt cag agt caa aat ggc ata gta 3692
   Pro His Leu Lys Leu Ala Arg Val Gln Ser Gln Asn Gly Ile Val
   1165 1170 1175
   ctg tca tgg agt gtc ctg gag gtg gat cga agc tgt gcc act gtt 3737
   Leu Ser Trp Ser Val Leu Glu Val Asp Arg Ser Cys Ala Thr Val
   1180 1185 1190
   gat agc tac cat ctc tat gct tac cat gag gaa ccc agt gcc act 3782
   Asp Ser Tyr His Leu Tyr Ala Tyr His Glu Glu Pro Ser Ala Thr
   1195 1200 1205
   gtg ccc tca caa tgg aaa aag att ggg gaa gtc aag gca ctt ccc 3827
   Val Pro Ser Gln Trp Lys Lys Ile Gly Glu Val Lys Ala Leu Pro
   1210 1215 1220
   ttg ccc atg gca tgt act ctc acc cag ttt gta tct ggt agc aaa 3872
   Leu Pro Met Ala Cys Thr Leu Thr Gln Phe Val Ser Gly Ser Lys
   1225 1230 1235
   tac tac ttt gca gta cga gcc aag gat att tat gga cgt ttt ggg 3917
   Tyr Tyr Phe Ala Val Arg Ala Lys Asp Ile Tyr Gly Arg Phe Gly
   1240 1245 1250
   cct ttc tgt gat cct cag tca aca gat gtg atc tct tct acc cag 3962
   Pro Phe Cys Asp Pro Gln Ser Thr Asp Val Ile Ser Ser Thr Gln
   1255 1260 1265
   agc agt taa accttggagc ctttatattt tcctctttta aaatttccac 4011
   Ser Ser
   1270
   cttttggtct tgtttttaat cttgtgcatg ataccccatg taaaatccac cttgtgcaag 4071
   atttcttgga cagatgtgtg tatacactac atttgtttat aaccagaagc aaaataaact 4131
   cagcccacaa agctagaatc ttttcctgga cagtttaggc tttggggttt ggaaatgtaa 4191
   atgtgtacct tgctttagtt ttgaggctgg ggaatatgtg tgggtgttta tgtgtgtttt 4251
   tccttatgta ggtgttattg cattggagtc tcccattttc attctcaaat ttacctctta 4311
   aagtacgaag taagtagatc aaaggatttg agatgtgtaa ctggcatgat tctgcttttg 4371
   aaggatctat agtatcattt tagttaagtg ggtcaaacag aatcaaaaca aaacccaaag 4431
   aaataaataa aaaacaaaat ggctaaatag tttaaaatag gttaattcga acacaggaaa 4491
   ggatctattt gttgtttctt ttgtctggtc tcctgagttg ttaattaggt gaaaaaagat 4551
   ctgcaatggc cccctccctt tcctaatctg gcttttacat ttattttgtg ccttaaagat 4611
   taactacaaa gataaacatg gccaaaaata aataaataaa tatggccata 4661

<210> 6
<211> 1270
<212> PRT
<213> Homo sapiens

<400> 6

Met Asp Ser Leu Glu Glu Pro Gln Lys Lys Val Phe Lys Ala Arg Lys
1               5                   10                  15

Thr Met Arg Val Ser Asp Arg Gln Gln Leu Glu Ala Val Tyr Lys Val
            20                  25                  30

Lys Glu Glu Leu Leu Lys Thr Asp Val Lys Leu Leu Asn Gly Asn His
        35                  40                  45

Glu Asn Gly Asp Leu Asp Pro Thr Ser Pro Leu Glu Asn Met Asp Tyr
    50                  55                  60

Ile Lys Asp Lys Glu Glu Val Asn Gly Ile Glu Glu Ile Cys Phe Asp
65                  70                  75                  80

Pro Glu Gly Ser Lys Ala Glu Trp Lys Glu Thr Pro Cys Ile Leu Ser
                85                  90                  95

Val Asn Val Lys Asn Lys Gln Asp Asp Asp Leu Asn Cys Glu Pro Leu
            100                 105                 110

Ser Pro His Asn Ile Thr Pro Glu Pro Val Ser Lys Leu Pro Ala Glu
        115                 120                 125

Pro Val Ser Gly Asp Pro Ala Pro Gly Asp Leu Asp Ala Gly Asp Pro
    130                 135                 140

Ala Ser Gly Val Leu Ala Ser Gly Asp Ser Thr Ser Gly Asp Pro Thr
145                 150                 155                 160

Ser Ser Glu Pro Ser Ser Ser Asp Ala Ala Ser Gly Asp Ala Thr Ser
                165                 170                 175

Gly Asp Ala Pro Ser Gly Asp Val Ser Pro Gly Asp Ala Thr Ser Gly
            180                 185                 190

Asp Ala Thr Ala Asp Asp Leu Ser Ser Gly Asp Pro Thr Ser Ser Asp

195                 200                 205

Pro Ile Pro Gly Glu Pro Val Pro Val Glu Pro Ile Ser Gly Asp Cys
    210                 215                 220

Ala Ala Asp Asp Ile Ala Ser Ser Glu Ile Thr Ser Val Asp Leu Ala
225                 230                 235                 240

Ser Gly Ala Pro Ala Ser Thr Asp Pro Ala Ser Asp Asp Leu Ala Ser
                245                 250                 255

Gly Asp Leu Ser Ser Ser Glu Leu Ala Ser Asp Asp Leu Ala Thr Gly
            260                 265                 270

Glu Leu Ala Ser Asp Glu Leu Thr Ser Glu Ser Thr Phe Asp Arg Thr
        275                 280                 285

Phe Glu Pro Lys Ser Val Pro Val Cys Glu Pro Val Pro Glu Ile Asp
    290                 295                 300

Asn Ile Glu Pro Ser Ser Asn Lys Asp Asp Asp Phe Leu Glu Lys Asn
305                 310                 315                 320

Gly Ala Asp Glu Lys Leu Glu Gln Ile Gln Ser Lys Asp Ser Leu Asp
                325                 330                 335

Glu Lys Asn Lys Ala Asp Asn Asn Ile Asp Ala Asn Glu Glu Thr Leu
            340                 345                 350

Glu Thr Asp Asp Thr Thr Ile Cys Ser Asp Arg Pro Pro Glu Asn Glu
        355                 360                 365

Lys Lys Val Glu Glu Asp Ile Ile Thr Glu Leu Ala Leu Gly Glu Asp
    370                 375                 380

Ala Ile Ser Ser Ser Met Glu Ile Asp Gln Gly Glu Lys Asn Glu Asp
385                 390                 395                 400

Glu Thr Ser Ala Asp Leu Val Glu Thr Ile Asn Glu Asn Val Ile Glu
                405                 410                 415

Asp Asn Lys Ser Glu Asn Ile Leu Glu Asn Thr Asp Ser Met Glu Thr
            420                 425                 430

Asp Glu Ile Ile Pro Ile Leu Glu Lys Leu Ala Pro Ser Glu Asp Glu
        435                 440                 445

Leu Thr Cys Phe Ser Lys Thr Ser Leu Leu Pro Ile Asp Glu Thr Asn
    450                 455                 460

Pro Asp Leu Glu Glu Lys Met Glu Ser Ser Phe Gly Ser Pro Ser Lys
465                 470                 475                 480

Gln Glu Ser Ser Glu Ser Leu Pro Lys Glu Ala Phe Leu Val Leu Ser
                485                 490                 495

Asp Glu Glu Asp Ile Ser Gly Glu Lys Asp Glu Ser Glu Val Ile Ser
            500                 505                 510

Gln Asn Glu Thr Cys Ser Pro Ala Glu Val Glu Ser Asn Glu Lys Asp
        515                 520                 525

Asn Lys Pro Glu Glu Glu Glu Gln Val Ile His Glu Asp Asp Glu Arg
    530                 535                 540

Pro Ser Glu Lys Asn Glu Phe Ser Arg Arg Lys Arg Ser Lys Ser Glu
545                 550                 555                 560

Asp Met Asp Asn Val Gln Ser Lys Arg Arg Arg Tyr Met Glu Glu Glu
                565                 570                 575

Tyr Glu Ala Glu Phe Gln Val Lys Ile Thr Ala Lys Gly Asp Ile Asn
            580                 585                 590

Gln Lys Leu Gln Lys Val Ile Gln Trp Leu Leu Glu Glu Lys Leu Cys
        595                 600                 605

Ala Leu Gln Cys Ala Val Phe Asp Lys Thr Leu Ala Glu Leu Lys Thr
    610                 615                 620

Arg Val Glu Lys Ile Glu Cys Asn Lys Arg His Lys Thr Val Leu Thr
625                 630                 635                 640

Glu Leu Gln Ala Lys Ile Ala Arg Leu Thr Lys Arg Phe Glu Ala Ala
                645                 650                 655

Lys Glu Asp Leu Lys Lys Arg His Glu His Pro Pro Asn Pro Pro Val
            660                 665                 670

Ser Pro Gly Lys Thr Val Asn Asp Val Asn Ser Asn Asn Asn Met Ser
        675                 680                 685

Tyr Arg Asn Ala Gly Thr Val Arg Gln Met Leu Glu Ser Lys Arg Asn
    690                 695                 700

Val Ser Glu Ser Ala Pro Pro Ser Phe Gln Thr Pro Val Asn Thr Val
705                 710                 715                 720

Ser Ser Thr Asn Leu Val Thr Pro Pro Ala Val Val Ser Ser Gln Pro
                725                 730                 735

Lys Leu Gln Thr Pro Val Thr Ser Gly Ser Leu Thr Ala Thr Ser Val
            740                 745                 750

Leu Pro Ala Pro Asn Thr Ala Thr Val Val Ala Thr Thr Gln Val Pro
        755                 760                 765

Ser Gly Asn Pro Gln Pro Thr Ile Ser Leu Gln Pro Leu Pro Val Ile
    770                 775                 780

Leu His Val Pro Val Ala Val Ser Ser Gln Pro Gln Leu Leu Gln Ser
785                 790                 795                 800

His Pro Gly Thr Leu Val Thr Asn Gln Pro Ser Gly Asn Val Glu Phe
                805                 810                 815

Ile Ser Val Gln Ser Pro Pro Thr Val Ser Gly Leu Thr Lys Asn Pro
            820                 825                 830

Val Ser Leu Pro Ser Leu Pro Asn Pro Thr Lys Pro Asn Asn Val Pro
        835                 840                 845

Ser Val Pro Ser Pro Ser Ile Gln Arg Asn Pro Thr Ala Ser Ala Ala
    850                 855                 860

Pro Leu Gly Thr Thr Leu Ala Val Gln Ala Val Pro Thr Ala His Ser
865                 870                 875                 880

Ile Val Gln Ala Thr Arg Thr Ser Leu Pro Thr Val Gly Pro Ser Gly
                885                 890                 895

Leu Tyr Ser Pro Ser Thr Asn Arg Gly Pro Ile Gln Met Lys Ile Pro
            900                 905                 910

Ile Ser Ala Phe Ser Thr Ser Ser Ala Ala Glu Gln Asn Ser Asn Thr
        915                 920                 925

Thr Pro Arg Ile Glu Asn Gln Thr Asn Lys Thr Ile Asp Ala Ser Val
    930                 935                 940

Ser Lys Lys Ala Ala Asp Ser Thr Ser Gln Cys Gly Lys Ala Thr Gly
945                 950                 955                 960

Ser Asp Ser Ser Gly Val Ile Asp Leu Thr Met Asp Asp Glu Glu Ser
                965                 970                 975

Gly Ala Ser Gln Asp Pro Lys Lys Leu Asn His Thr Pro Val Ser Thr
            980                 985                 990

Met Ser Ser Ser Gln Pro Val Ser  Arg Pro Leu Gln Pro  Ile Gln Pro
        995                 1000                1005

Ala Pro  Pro Leu Gln Pro Ser  Gly Val Pro Thr Ser  Gly Pro Ser
    1010                 1015                 1020

Gln Thr  Thr Ile His Leu Leu  Pro Thr Ala Pro Thr  Thr Val Asn
    1025                 1030                 1035

Val Thr  His Arg Pro Val Thr  Gln Val Thr Thr Arg  Leu Pro Val
    1040                 1045                 1050

Pro Arg  Ala Pro Ala Asn His  Gln Val Val Tyr Thr  Thr Leu Pro
    1055                 1060                 1065

Ala Pro  Pro Ala Gln Ala Pro  Leu Arg Gly Thr Val  Met Gln Ala
    1070                 1075                 1080

Pro Ala  Val Arg Gln Val Asn  Pro Gln Asn Ser Val  Thr Val Arg
    1085                 1090                 1095

Val Pro  Gln Thr Thr Thr Tyr  Val Val Asn Asn Gly  Leu Thr Leu
    1100                 1105                 1110

Gly Ser  Thr Gly Pro Gln Leu  Thr Val His His Arg  Pro Pro Gln
    1115                 1120                 1125

Val His  Thr Glu Pro Pro Arg  Pro Val His Pro Ala  Pro Leu Pro
    1130                 1135                 1140

Glu Ala  Pro Gln Pro Gln Arg  Leu Pro Pro Glu Ala  Ala Ser Thr
    1145                 1150                 1155

Ser Leu  Pro Gln Lys Pro His  Leu Lys Leu Ala Arg  Val Gln Ser
    1160                 1165                 1170

Gln Asn  Gly Ile Val Leu Ser  Trp Ser Val Leu Glu  Val Asp Arg
    1175                 1180                 1185

Ser Cys  Ala Thr Val Asp Ser  Tyr His Leu Tyr Ala  Tyr His Glu

1190                1195                1200

Glu Pro Ser Ala Thr Val Pro Ser Gln Trp Lys Lys Ile Gly Glu
    1205                1210                1215

Val Lys Ala Leu Pro Leu Pro Met Ala Cys Thr Leu Thr Gln Phe
    1220                1225                1230

Val Ser Gly Ser Lys Tyr Tyr Phe Ala Val Arg Ala Lys Asp Ile
    1235                1240                1245

Tyr Gly Arg Phe Gly Pro Phe Cys Asp Pro Gln Ser Thr Asp Val
    1250                1255                1260

Ile Ser Ser Thr Gln Ser Ser
    1265                1270

<210>  7
<211>  9019
<212>  DNA
<213>  Homo sapiens

<220>
<221>  CDS
<222>  (321)..(4157)

<400>  7
gacgggattc acaggcatta tgttacaaaa ctggcttgct gccttctgtt ttctttttct      60

gtgtcctgtg tagctgcatt gtttgttgca gtatatgcat tagctgttgt ctatgttcag     120

tttcctgcag gctgacaaag tgggaggggc tttgtttgtt acatagctga gttttcactg     180

tgtgagttct tgagtgcaca ttgcagccat cttaattaca cttttgtgca actgagactg     240

tgcagctgtc tcaaaatggc tgctaaccta gtctttgtag ctgcttaggc agcaagtatg     300

tcagtgagga gacttgtcac atg cat cag gac cag aga ttc aga atg gac agt     353
                      Met His Gln Asp Gln Arg Phe Arg Met Asp Ser
                      1               5                   10

tta gaa gaa cct cag aaa aaa gtc ttt aag gct cga aaa acg atg aga     401
Leu Glu Glu Pro Gln Lys Lys Val Phe Lys Ala Arg Lys Thr Met Arg
            15                  20                  25

gtg agt gat cgt cag caa ctt gaa gca gtg tac aag gtc aaa gaa gaa     449
Val Ser Asp Arg Gln Gln Leu Glu Ala Val Tyr Lys Val Lys Glu Glu
        30                  35                  40

ctg ttg aaa act gat gtc aag ctg tta aat ggc aac cat gaa aat gga     497
Leu Leu Lys Thr Asp Val Lys Leu Leu Asn Gly Asn His Glu Asn Gly
    45                  50                  55

gat ttg gac cca acc tca cct ttg gaa aac atg gat tac att aaa gac     545
Asp Leu Asp Pro Thr Ser Pro Leu Glu Asn Met Asp Tyr Ile Lys Asp
60                  65                  70                  75

aag gaa gag gtg aat ggc att gaa gag att tgt ttt gat cct gaa gga 593
Lys Glu Glu Val Asn Gly Ile Glu Glu Ile Cys Phe Asp Pro Glu Gly
                80                  85                  90

agt aaa gca gaa tgg aag gaa aca ccc tgt atc cta agt gtt aat gta 641
Ser Lys Ala Glu Trp Lys Glu Thr Pro Cys Ile Leu Ser Val Asn Val
            95                  100                 105

aaa aac aag cag gat gat gat tta aat tgt gaa cct ttg tct ccc cat 689
Lys Asn Lys Gln Asp Asp Asp Leu Asn Cys Glu Pro Leu Ser Pro His
        110                 115                 120

aat ata act cca gaa cca gtc tct aaa ctg cct gct gaa cca gtt tct 737
Asn Ile Thr Pro Glu Pro Val Ser Lys Leu Pro Ala Glu Pro Val Ser
    125                 130                 135

ggt gat cca gcc cct ggt gat ctg gat gcc gga gat cca gcc tcc gga 785
Gly Asp Pro Ala Pro Gly Asp Leu Asp Ala Gly Asp Pro Ala Ser Gly
140                 145                 150                 155

gta ctg gcc tct ggt gat tcc acc tct ggt gat ccc acc tct agc gag 833
Val Leu Ala Ser Gly Asp Ser Thr Ser Gly Asp Pro Thr Ser Ser Glu
                160                 165                 170

ccc tcc tct agt gat gct gcc tct ggt gat gca acc tct ggt gat gcc 881
Pro Ser Ser Ser Asp Ala Ala Ser Gly Asp Ala Thr Ser Gly Asp Ala
            175                 180                 185

cct tct ggt gat gtg tcc cct ggt gat gcc acc tct ggt gat gcc act 929
Pro Ser Gly Asp Val Ser Pro Gly Asp Ala Thr Ser Gly Asp Ala Thr
        190                 195                 200

gct gat gat ctc tcc tct ggt gat ccc acc tct agt gat ccc atc cca 977
Ala Asp Asp Leu Ser Ser Gly Asp Pro Thr Ser Ser Asp Pro Ile Pro
    205                 210                 215

ggt gaa ccg gtc cct gtt gaa ccc att tct ggt gat tgt gcc gct gat 1025
Gly Glu Pro Val Pro Val Glu Pro Ile Ser Gly Asp Cys Ala Ala Asp
220                 225                 230                 235

gat ata gcc tct agt gaa ata act tct gtt gat ctg gct tct gga gca 1073
Asp Ile Ala Ser Ser Glu Ile Thr Ser Val Asp Leu Ala Ser Gly Ala
                240                 245                 250

cca gct tcc act gat cca gcc tct gat gat ctg gcc tct ggt gat cta 1121
Pro Ala Ser Thr Asp Pro Ala Ser Asp Asp Leu Ala Ser Gly Asp Leu
            255                 260                 265

tcc tct agt gaa ctg gcc tct gat gat ctg gcc act ggt gaa ctg gcc 1169
Ser Ser Ser Glu Leu Ala Ser Asp Asp Leu Ala Thr Gly Glu Leu Ala
        270                 275                 280

tct gat gag ctg act tct gaa tca acc ttt gat cgt acc ttt gaa cca 1217
Ser Asp Glu Leu Thr Ser Glu Ser Thr Phe Asp Arg Thr Phe Glu Pro
    285                 290                 295

aag tct gta cca gtt tgt gaa cca gtt cct gaa att gac aat ata gaa 1265
Lys Ser Val Pro Val Cys Glu Pro Val Pro Glu Ile Asp Asn Ile Glu
300                 305                 310                 315

cca agt agc aat aaa gat gat gat ttt ctt gaa aaa aat gga gct gat 1313
Pro Ser Ser Asn Lys Asp Asp Asp Phe Leu Glu Lys Asn Gly Ala Asp

320                 325                 330

gaa aaa tta gag caa att cag agt aaa gac tca ttg gat gag aaa aat      1361
Glu Lys Leu Glu Gln Ile Gln Ser Lys Asp Ser Leu Asp Glu Lys Asn
            335                 340                 345

aaa gct gat aat aat att gat gct aat gaa gaa act cta gaa aca gat      1409
Lys Ala Asp Asn Asn Ile Asp Ala Asn Glu Glu Thr Leu Glu Thr Asp
        350                 355                 360

gat aca act att tgt tca gat cga cct cct gaa aat gaa aag aag gta      1457
Asp Thr Thr Ile Cys Ser Asp Arg Pro Pro Glu Asn Glu Lys Lys Val
    365                 370                 375

gag gaa gat att atc aca gag ctt gct ctt gga gaa gat gct ata tct      1505
Glu Glu Asp Ile Ile Thr Glu Leu Ala Leu Gly Glu Asp Ala Ile Ser
380                 385                 390                 395

agc agt atg gaa att gac caa ggt gaa aag aat gaa gat gaa act tct      1553
Ser Ser Met Glu Ile Asp Gln Gly Glu Lys Asn Glu Asp Glu Thr Ser
                400                 405                 410

gca gat ctt gta gaa acg att aat gaa aat gtt att gaa gat aac aaa      1601
Ala Asp Leu Val Glu Thr Ile Asn Glu Asn Val Ile Glu Asp Asn Lys
            415                 420                 425

agt gag aat atc tta gaa aat aca gac tct atg gag aca gat gaa atc      1649
Ser Glu Asn Ile Leu Glu Asn Thr Asp Ser Met Glu Thr Asp Glu Ile
        430                 435                 440

att cct att ttg gaa aag ctt gca cct tct gag gat gaa ctt act tgc      1697
Ile Pro Ile Leu Glu Lys Leu Ala Pro Ser Glu Asp Glu Leu Thr Cys
    445                 450                 455

ttt tct aaa aca tct ctc ctt cca atc gat gag aca aat cca gat ttg      1745
Phe Ser Lys Thr Ser Leu Leu Pro Ile Asp Glu Thr Asn Pro Asp Leu
460                 465                 470                 475

gaa gag aaa atg gaa agt tct ttt ggt tca cca tct aaa caa gaa agt      1793
Glu Glu Lys Met Glu Ser Ser Phe Gly Ser Pro Ser Lys Gln Glu Ser
                480                 485                 490

agt gag agt ttg cca aaa gaa gcc ttt ctg gtc ctc tct gat gaa gag      1841
Ser Glu Ser Leu Pro Lys Glu Ala Phe Leu Val Leu Ser Asp Glu Glu
            495                 500                 505

gat att tcg ggt gaa aaa gat gag tct gaa gtt ata tcg caa aat gaa      1889
Asp Ile Ser Gly Glu Lys Asp Glu Ser Glu Val Ile Ser Gln Asn Glu
        510                 515                 520

acg tgc tct cca gca gaa gta gaa agt aat gaa aag gac aac aaa cct      1937
Thr Cys Ser Pro Ala Glu Val Glu Ser Asn Glu Lys Asp Asn Lys Pro
    525                 530                 535

gag gaa gaa gag caa gta ata cat gaa gat gat gaa aga cct tct gag      1985
Glu Glu Glu Glu Gln Val Ile His Glu Asp Asp Glu Arg Pro Ser Glu
540                 545                 550                 555

aaa aat gaa ttt tct aga cga aaa cgt tct aaa tca gaa gac atg gac      2033
Lys Asn Glu Phe Ser Arg Arg Lys Arg Ser Lys Ser Glu Asp Met Asp
                560                 565                 570

aat gta cag tct aaa cgt cgt cga tat atg gaa gaa gaa tat gag gca      2081

Asn Val Gln Ser Lys Arg Arg Arg Tyr Met Glu Glu Glu Tyr Glu Ala
            575                 580                 585

gaa ttt caa gta aag att aca gcc aaa gga gac att aac cag aaa ctt      2129
Glu Phe Gln Val Lys Ile Thr Ala Lys Gly Asp Ile Asn Gln Lys Leu
        590                 595                 600

caa aag gtt ata cag tgg ttg ctg gaa gaa aaa ttg tgt gcg ctg cag      2177
Gln Lys Val Ile Gln Trp Leu Leu Glu Glu Lys Leu Cys Ala Leu Gln
    605                 610                 615

tgt gct gta ttt gat aag act ttg gca gaa ttg aaa aca cga gtg gaa      2225
Cys Ala Val Phe Asp Lys Thr Leu Ala Glu Leu Lys Thr Arg Val Glu
620                 625                 630                 635

aag att gaa tgt aac aag agg cat aaa aca gtt ctc act gaa cta cag      2273
Lys Ile Glu Cys Asn Lys Arg His Lys Thr Val Leu Thr Glu Leu Gln
                640                 645                 650

gcc aag ata gcc agg tta acc aaa cgc ttt gaa gca gcc aaa gaa gat      2321
Ala Lys Ile Ala Arg Leu Thr Lys Arg Phe Glu Ala Ala Lys Glu Asp
            655                 660                 665

ctt aag aaa aga cat gaa cat cca ccc aac cca cca gta tca cca gga      2369
Leu Lys Lys Arg His Glu His Pro Pro Asn Pro Pro Val Ser Pro Gly
        670                 675                 680

aaa act gta aat gat gtc aac agc aat aat aac atg tct tac aga aat      2417
Lys Thr Val Asn Asp Val Asn Ser Asn Asn Asn Met Ser Tyr Arg Asn
    685                 690                 695

gca ggc aca gtg aga cag atg ctg gag tcc aaa aga aat gta agc gag      2465
Ala Gly Thr Val Arg Gln Met Leu Glu Ser Lys Arg Asn Val Ser Glu
700                 705                 710                 715

agt gca cca cca tcc ttt caa act cct gtg aat aca gta tct tca acc      2513
Ser Ala Pro Pro Ser Phe Gln Thr Pro Val Asn Thr Val Ser Ser Thr
                720                 725                 730

aat ctt gtc act cct cca gca gtt gtc agt agt caa cct aaa ttg cag      2561
Asn Leu Val Thr Pro Pro Ala Val Val Ser Ser Gln Pro Lys Leu Gln
            735                 740                 745

act cca gtg act tcg ggt tcc ctc aca gca acg tca gtt ctt cct gca      2609
Thr Pro Val Thr Ser Gly Ser Leu Thr Ala Thr Ser Val Leu Pro Ala
        750                 755                 760

ccc aat aca gct act gta gtt gct act act cag gtg cct agt gga aat      2657
Pro Asn Thr Ala Thr Val Val Ala Thr Thr Gln Val Pro Ser Gly Asn
    765                 770                 775

ccc cag cct aca atc tct tta cag cct ttg cca gtg att ttg cat gta      2705
Pro Gln Pro Thr Ile Ser Leu Gln Pro Leu Pro Val Ile Leu His Val
780                 785                 790                 795

cct gtt gca gta tcc tcc cag cct cag ctt cta cag agc cat cca ggg      2753
Pro Val Ala Val Ser Ser Gln Pro Gln Leu Leu Gln Ser His Pro Gly
                800                 805                 810

act ttg gtg act aat caa cca tct ggc aat gtt gaa ttc att tct gtg      2801
Thr Leu Val Thr Asn Gln Pro Ser Gly Asn Val Glu Phe Ile Ser Val
            815                 820                 825

caa agc cca cct aca gtg agt ggt ctt acc aaa aat cca gta tcc ttg 2849
Gln Ser Pro Pro Thr Val Ser Gly Leu Thr Lys Asn Pro Val Ser Leu
        830                 835                 840

cca tcc ttg cca aat ccc act aaa cca aac aac gtt cct tct gtg ccc 2897
Pro Ser Leu Pro Asn Pro Thr Lys Pro Asn Asn Val Pro Ser Val Pro
    845                 850                 855

agt cct agt att caa agg aac cct act gcc agt gct gca cca ttg gga 2945
Ser Pro Ser Ile Gln Arg Asn Pro Thr Ala Ser Ala Ala Pro Leu Gly
860                 865                 870                 875

aca aca ctt gct gtg cag gct gtt cca aca gca cac tct att gta caa 2993
Thr Thr Leu Ala Val Gln Ala Val Pro Thr Ala His Ser Ile Val Gln
                880                 885                 890

gcc aca agg act tct tta ccc aca gtg ggc cca tca gga ctc tat agt 3041
Ala Thr Arg Thr Ser Leu Pro Thr Val Gly Pro Ser Gly Leu Tyr Ser
            895                 900                 905

cca tca act aat cga ggt cct ata cag atg aaa att cca att tct gca 3089
Pro Ser Thr Asn Arg Gly Pro Ile Gln Met Lys Ile Pro Ile Ser Ala
        910                 915                 920

ttt agt act tcg tct gct gca gaa cag aac agc aat acc acc cca aga 3137
Phe Ser Thr Ser Ser Ala Ala Glu Gln Asn Ser Asn Thr Thr Pro Arg
    925                 930                 935

att gaa aac cag aca aac aaa aca ata gat gct tct gtc agt aag aaa 3185
Ile Glu Asn Gln Thr Asn Lys Thr Ile Asp Ala Ser Val Ser Lys Lys
940                 945                 950                 955

gca gct gat agc aca tca cag tgt gga aaa gcc act ggc agt gat tca 3233
Ala Ala Asp Ser Thr Ser Gln Cys Gly Lys Ala Thr Gly Ser Asp Ser
                960                 965                 970

agt ggt gtc att gat ctc aca atg gat gat gaa gag agt gga gct tca 3281
Ser Gly Val Ile Asp Leu Thr Met Asp Asp Glu Glu Ser Gly Ala Ser
            975                 980                 985

caa gac ccc aaa aaa cta aat cac act cct gta tca acc atg agt tct 3329
Gln Asp Pro Lys Lys Leu Asn His Thr Pro Val Ser Thr Met Ser Ser
        990                 995                 1000

tct cag cct gtg tca cga cca ttg caa ccc ata caa cca gca ccg 3374
Ser Gln Pro Val Ser Arg Pro Leu Gln Pro Ile Gln Pro Ala Pro
    1005                1010                1015

cct ctt caa cca tct ggg gtg cca aca agt gga cca tct cag acc 3419
Pro Leu Gln Pro Ser Gly Val Pro Thr Ser Gly Pro Ser Gln Thr
    1020                1025                1030

acc ata cac tta cta cct aca gct cca act acc gtg aat gta aca 3464
Thr Ile His Leu Leu Pro Thr Ala Pro Thr Thr Val Asn Val Thr
    1035                1040                1045

cat cgt cca gta act cag gtg acc aca aga ctc cct gta cca aga 3509
His Arg Pro Val Thr Gln Val Thr Thr Arg Leu Pro Val Pro Arg
    1050                1055                1060

gct cct gca aac cac cag gtg gtt tat aca act ctt cct gca cca 3554
Ala Pro Ala Asn His Gln Val Val Tyr Thr Thr Leu Pro Ala Pro
    1065                1070                1075

cca gct cag gct ccc ttg cga gga act gtt atg cag gct cct gct 3599
Pro Ala Gln Ala Pro Leu Arg Gly Thr Val Met Gln Ala Pro Ala
    1080                1085                1090

gtt cgg cag gtc aat ccc caa aat agt gtt aca gtt cga gtg cct 3644
Val Arg Gln Val Asn Pro Gln Asn Ser Val Thr Val Arg Val Pro
    1095                1100                1105

caa aca acc aca tat gtt gta aac aat gga cta acc ctg gga tca 3689
Gln Thr Thr Thr Tyr Val Val Asn Asn Gly Leu Thr Leu Gly Ser
    1110                1115                1120

aca gga cct cag ctc aca gtg cat cac cga cca cca caa gtg cat 3734
Thr Gly Pro Gln Leu Thr Val His His Arg Pro Pro Gln Val His
    1125                1130                1135

act gag ccc cca cgc ccc gtg cac cca gca ccc tta cca gaa gct 3779
Thr Glu Pro Pro Arg Pro Val His Pro Ala Pro Leu Pro Glu Ala
    1140                1145                1150

cca caa cca cag cgt ctg ccc cca gaa gct gcc agc aca tct ctg 3824
Pro Gln Pro Gln Arg Leu Pro Pro Glu Ala Ala Ser Thr Ser Leu
    1155                1160                1165

cct cag aag cca cac ttg aag tta gca cgc gtt cag agt caa aat 3869
Pro Gln Lys Pro His Leu Lys Leu Ala Arg Val Gln Ser Gln Asn
    1170                1175                1180

ggc ata gta ctg tca tgg agt gtc ctg gag gtg gat cga agc tgt 3914
Gly Ile Val Leu Ser Trp Ser Val Leu Glu Val Asp Arg Ser Cys
    1185                1190                1195

gcc act gtt gat agc tac cat ctc tat gct tac cat gag gaa ccc 3959
Ala Thr Val Asp Ser Tyr His Leu Tyr Ala Tyr His Glu Glu Pro
    1200                1205                1210

agt gcc act gtg ccc tca caa tgg aaa aag att ggg gaa gtc aag 4004
Ser Ala Thr Val Pro Ser Gln Trp Lys Lys Ile Gly Glu Val Lys
    1215                1220                1225

gca ctt ccc ttg ccc atg gca tgt act ctc acc cag ttt gta tct 4049
Ala Leu Pro Leu Pro Met Ala Cys Thr Leu Thr Gln Phe Val Ser
    1230                1235                1240

ggt agc aaa tac tac ttt gca gta cga gcc aag gat att tat gga 4094
Gly Ser Lys Tyr Tyr Phe Ala Val Arg Ala Lys Asp Ile Tyr Gly
    1245                1250                1255

cgt ttt ggg cct ttc tgt gat cct cag tca aca gat gtg atc tct 4139
Arg Phe Gly Pro Phe Cys Asp Pro Gln Ser Thr Asp Val Ile Ser
    1260                1265                1270

tct acc cag agc agt taa accttggagc ctttatattt tcctctttta 4187
Ser Thr Gln Ser Ser
    1275

aaatttccac cttttggtct tgtttttaat cttgtgcatg ataccccatg taaaatccac 4247

cttgtgcaag atttcttgga cagatgtgtg tatacactac atttgtttat aaccagaagc 4307

aaaataaact cagcccacaa agctagaatc ttttcctgga cagtttaggc tttggggttt 4367

ggaaatgtaa atgtgtacct tgctttagtt ttgaggctgg ggaatatgtg tgggtgttta 4427

tgtgtgtttt tccttatgta ggtgttattg cattggagtc tcccattttc attctcaaat 4487

ttacctctta aagtacgaag taagtagatc aaaggatttg agatgtgtaa ctggcatgat 4547

tctgcttttg aaggatctat agtatcattt tagttaagtg ggtcaaacag aatcaaaaca 4607

aaacccaaag aaataaataa aaaacaaaat ggctaaatag tttaaaatag gttaattcga 4667

acacaggaaa ggatctattt gttgtttctt ttgtctggtc tcctgagttg ttaattaggt 4727

gaaaaaagat ctgcaatggc cccctccctt tcctaatctg gcttttacat ttattttgtg 4787

ccttaaagat taactacaaa gataaacatg gccaaaaata aataaataaa tatggccata 4847

tgtccgttgt tgcttagtct tcccttgcag ccttttaccc ttgatttctc cttcatctct 4907

accaaatata gcacaactcc tcaagtaatt tttttttttt aagatggagt tttgctcttg 4967

ttgcccaggc tagagtgcgg tggcacaatc ttggctcact gcaacctctg cctcctgggt 5027

tcaagcaatt cttctgtctc agccccagag tagctgggat tacaggcacc tgtcaccatg 5087

cccggctaat ttttgtattt ttagtagaga tggggtttca ccatgttggc caggctggtc 5147

ttgaactcct gacctcaggt gatccacccg cctcggcctc ccagagtgct gcgattacag 5207

ttgtgagcca ctgtatccag cctactcaag tgatttttaa accaaggtgt gtgtatgtac 5267

atggatgtgt atgtacacac acacacaggt gcatgcatag tctcatctta gccgggcatg 5327

gtggcgacaa tcacatttaa ctccaaaaat ttgacgtgct attttcttct aaaactatag 5387

atgcttttat ttttggtcac tatttaactt tgtgtaaagt ggaccaagag aaaaccatat 5447

tgtggaagac agtttttcac acattttagt atccgtgttg cacactgtgt tagagattat 5507

gaaaaccatt ctagttcagt ttatcaccca aagctatcac ccaacactaa ctccttagta 5567

ttctttcaaa ggaagcaaat agtaagatta caatatagaa tgagctaaaa tatcaagggg 5627

aaagcttcat ttcctcttta ttctctttat cgtccttccc attttaacct tttaactttg 5687

ttttgatggt gccaagttga gtctgatgta cccattgtta gctttggata gaatttaaag 5747

tatatcccag aattagctgg gcatgttggt gcatgcctgt aatcccagct actcaggagg 5807

caaggctgga gaatcgcttg aacccgggag gcggaggttg cagtgagctg agattgcgcc 5867

attgcactcc atcctgggag acaagagcga aactctgcct caaaaaaaaa agaatttaaa 5927

gtatatcctt tgaggtcatt ttatgtaaca attagatata acctcctgtg tatctctctg 5987

ttcctcttca aagttattaa aattcagctt aggtcatgga tttttaatat gagggccagc 6047

tgtactggtg tcaggtaggt gttttgggtg tagataattt aggactggag ggctgagtta 6107

agttttagga ggagaatgtg gttgagaata ttttgaaagg aatttatgga gtttgtgaaa 6167

taacttcgaa gtcctcttcc tttacaatat ttgaattcat atttgtacct tctcaaaata 6227

gtgattcatt tttcctagaa ttacaggagg gagctctttt actaatgttg ttttgtttgc 6287

aactttgatg gcttataata ggaagtattc tagttgtaaa gaaaactctt tagagacttt 6347

tgactggtca gtatactgag gtgtgagatt tgattcatga tgaagaaagc ctatagattg 6407

ccaaaaaatt aattctccaa accacctttc actctcagaa aatgagacca caaaggagta 6467

tgctataaat caaatttgcc aaccaattat gtagatatta ctcattctag gactaatgat 6527

gatggtaaag aagttgccag tgttatggca atgaaaattt cagaaaggag gagttgatga 6587

tcttctagat gtatatgaac acctgtctat atctgcatgt atatgttttg acctgcagtg 6647

gttgcaatgt tgatatgtgt tcaagattat tcctgtctac aaaactgaag gcccatgttc 6707

aaattgttct ttattgggtg tttttatggt cacgtggtaa caattttctt acctaaccta 6767

caaaaggttc tcttgatgaa cattttattt tatatttact aatctttttt aaaaaaagct 6827

ttcatagcat tatataatca gatgaagaaa gcccagtaga ataaaaaaaa aattcattag 6887

cctagcctat attatgtttt ctgtcaaagg aaaacaaatt ctcaaatagg aattctaaaa 6947

atatttacta aagtaaaata actacttaaa atgttttatt ccagttggaa ggaaggtaca 7007

gggagaaatc gcaattattt aggggagaag tatatttatt ataagatggt gtcctcaaat 7067

tagcctacca tggcacgtag gggcagcagc tatattagat ttactagagg tgctaagtta 7127

gaacactagg cttttattga ggcaggtttt aatattgata gatgcttttt gtttggtttg 7187

tttcttctgg gagagaatgg aggacttaag tagaagtagc tactgataac agactttcta 7247

gtagcagttt ccactccacg gttacctttt tagtttcata gtatcttttc acaaagtatt 7307

acaaataagc tagattctcc cagtttggga atgcaagttt gctacatttt tagcctggca 7367

atatttgtgt aggtattgcc ttattggaaa ttctggaaac ctgatactgc aacctgcaat 7427

gtaggatgtt tgtatggcat ttaaaggtaa tggtgatgtt tattattcta tactttgcat 7487

tctgtgagag taattttcac tctgtcttaa gtgtgagtaa gcctcttcta aaaatcttgt 7547

tcttgccaag aaatttataa atcacatacg aagacgtctg ttgctaacag ttaactttat 7607

gaggtaacta tatccttcta tttctctgga ctcattttta aaaaatatgc cgaatactgc 7667

atactgttta aggtagtata taagtttatg agagaagtgg agagctttct tccttgaaaa 7727

gtcggtattt gttgagatac catttgcctc acagagaggt gttccccact cccatcccca 7787

ttgccagata ataaatattt tgagaaaagt gacctaaaac agctgaaatc ttaggtgcat 7847

ctgtctgcag acctccttaa gcaggctgta tcttacaatt cccttactgc actgggtaag 7907

tgttaactta gtttttgttg tttgctcttt tgctttaaat attctccaaa ttaccattta 7967

tgcaacatgg ttagggttaa tactgcatgg tattcattta tcttgtttca tgaactttcc 8027

agtactgtac aaggtcaaca aagtaatgcc tgtggtatcc tcatctctca cttttttact 8087

ctgtgatttt agcacagtaa ggtactgcaa agaccttcct tccaaatgtt ctccttgact 8147

ttatttcttg ggccaattca gtatcctcaa catcctaaga ttttgttgtt ttatcactga 8207

cctgtggttg gcctgtttta ttctaatttc cagaaaagtc aagtcccagt atttgcaata 8267

tcaaataact ctaaaaccga tgtgtgattc taccttcctt actattttta ctgggcaaat 8327

gccctatttt tttaattatt attattttta acttttggga cacacaaaaa tcagcaattc 8387

tcatgaagcg tttgttagtg tggcagactt gtctaattcc tgaaactcat tcatcccctt 8447

gagccagcca atggggagga ataggataat gcaaacacat gttttgtttt ctcattttca 8507

aataatttac catgttaaaa taaacttttc tttgtttttt atttgtagag tcagctaagt 8567

acccatattt aaatgccgtc tttattattt ttttgaggtc tttgtttttg tctgtttttg 8627

ttttgttttg ttttgtaaat aaggtaactg ggcaatcaaa caccttttgg ggattctggc 8687

tttagtattt tatcagccat ttttaaatta aatataaaaa tcctttgtaa gaaacttgca 8747

tcctaatttt tctttattgc aattgaaagt gtaaataata agacaatgta agtaagacct 8807

tcctaatgtc taatacaaac tgggctccag caagtggccc tatttttatt agggttttga 8867

aggtttgtgt gtgtgtgtgc gtgcgtgtgt gtgtttttct tttttaaatg tatagtagag 8927

tggtgtctgt ataagtgtta cctgtagtgg ggttttgtcc agcaagcctg aaatttatac 8987

tttgaaataa aactactggg tttttaacat tc 9019

<210> 8
<211> 1278
<212> PRT
<213> Homo sapiens

<400> 8

Met His Gln Asp Gln Arg Phe Arg Met Asp Ser Leu Glu Glu Pro Gln
1               5                   10                  15

Lys Lys Val Phe Lys Ala Arg Lys Thr Met Arg Val Ser Asp Arg Gln
            20                  25                  30

Gln Leu Glu Ala Val Tyr Lys Val Lys Glu Glu Leu Leu Lys Thr Asp
        35                  40                  45

Val Lys Leu Leu Asn Gly Asn His Glu Asn Gly Asp Leu Asp Pro Thr
    50                  55                  60

Ser Pro Leu Glu Asn Met Asp Tyr Ile Lys Asp Lys Glu Glu Val Asn
65                  70                  75                  80

Gly Ile Glu Glu Ile Cys Phe Asp Pro Glu Gly Ser Lys Ala Glu Trp
                85                  90                  95

Lys Glu Thr Pro Cys Ile Leu Ser Val Asn Val Lys Asn Lys Gln Asp

100                 105                 110

Asp Asp Leu Asn Cys Glu Pro Leu Ser Pro His Asn Ile Thr Pro Glu
        115                 120                 125

Pro Val Ser Lys Leu Pro Ala Glu Pro Val Ser Gly Asp Pro Ala Pro
    130                 135                 140

Gly Asp Leu Asp Ala Gly Asp Pro Ala Ser Gly Val Leu Ala Ser Gly
145                 150                 155                 160

Asp Ser Thr Ser Gly Asp Pro Thr Ser Ser Glu Pro Ser Ser Ser Asp
                165                 170                 175

Ala Ala Ser Gly Asp Ala Thr Ser Gly Asp Ala Pro Ser Gly Asp Val
            180                 185                 190

Ser Pro Gly Asp Ala Thr Ser Gly Asp Ala Thr Ala Asp Asp Leu Ser
        195                 200                 205

Ser Gly Asp Pro Thr Ser Ser Asp Pro Ile Pro Gly Glu Pro Val Pro
    210                 215                 220

Val Glu Pro Ile Ser Gly Asp Cys Ala Ala Asp Asp Ile Ala Ser Ser
225                 230                 235                 240

Glu Ile Thr Ser Val Asp Leu Ala Ser Gly Ala Pro Ala Ser Thr Asp
                245                 250                 255

Pro Ala Ser Asp Asp Leu Ala Ser Gly Asp Leu Ser Ser Ser Glu Leu
            260                 265                 270

Ala Ser Asp Asp Leu Ala Thr Gly Glu Leu Ala Ser Asp Glu Leu Thr
        275                 280                 285

Ser Glu Ser Thr Phe Asp Arg Thr Phe Glu Pro Lys Ser Val Pro Val
    290                 295                 300

Cys Glu Pro Val Pro Glu Ile Asp Asn Ile Glu Pro Ser Ser Asn Lys
305                 310                 315                 320

Asp Asp Asp Phe Leu Glu Lys Asn Gly Ala Asp Glu Lys Leu Glu Gln
                325                 330                 335

Ile Gln Ser Lys Asp Ser Leu Asp Glu Lys Asn Lys Ala Asp Asn Asn
            340                 345                 350

Ile Asp Ala Asn Glu Glu Thr Leu Glu Thr Asp Asp Thr Thr Ile Cys
        355                 360                 365

Ser Asp Arg Pro Pro Glu Asn Glu Lys Lys Val Glu Glu Asp Ile Ile
    370                 375                 380

Thr Glu Leu Ala Leu Gly Glu Asp Ala Ile Ser Ser Ser Met Glu Ile
385                 390                 395                 400

Asp Gln Gly Glu Lys Asn Glu Asp Glu Thr Ser Ala Asp Leu Val Glu
                405                 410                 415

Thr Ile Asn Glu Asn Val Ile Glu Asp Asn Lys Ser Glu Asn Ile Leu
            420                 425                 430

Glu Asn Thr Asp Ser Met Glu Thr Asp Glu Ile Ile Pro Ile Leu Glu
        435                 440                 445

Lys Leu Ala Pro Ser Glu Asp Glu Leu Thr Cys Phe Ser Lys Thr Ser
    450                 455                 460

Leu Leu Pro Ile Asp Glu Thr Asn Pro Asp Leu Glu Glu Lys Met Glu
465                 470                 475                 480

Ser Ser Phe Gly Ser Pro Ser Lys Gln Glu Ser Ser Glu Ser Leu Pro
                485                 490                 495

Lys Glu Ala Phe Leu Val Leu Ser Asp Glu Glu Asp Ile Ser Gly Glu
            500                 505                 510

Lys Asp Glu Ser Glu Val Ile Ser Gln Asn Glu Thr Cys Ser Pro Ala
        515                 520                 525

Glu Val Glu Ser Asn Glu Lys Asp Asn Lys Pro Glu Glu Glu Glu Gln
    530                 535                 540

Val Ile His Glu Asp Asp Glu Arg Pro Ser Glu Lys Asn Glu Phe Ser
545                 550                 555                 560

Arg Arg Lys Arg Ser Lys Ser Glu Asp Met Asp Asn Val Gln Ser Lys
                565                 570                 575

Arg Arg Arg Tyr Met Glu Glu Glu Tyr Glu Ala Glu Phe Gln Val Lys
            580                 585                 590

Ile Thr Ala Lys Gly Asp Ile Asn Gln Lys Leu Gln Lys Val Ile Gln
        595                 600                 605

Trp Leu Leu Glu Glu Lys Leu Cys Ala Leu Gln Cys Ala Val Phe Asp
    610                 615                 620

Lys Thr Leu Ala Glu Leu Lys Thr Arg Val Glu Lys Ile Glu Cys Asn
625                 630                 635                 640

Lys Arg His Lys Thr Val Leu Thr Glu Leu Gln Ala Lys Ile Ala Arg
                645                 650                 655

Leu Thr Lys Arg Phe Glu Ala Ala Lys Glu Asp Leu Lys Lys Arg His
            660                 665                 670

Glu His Pro Pro Asn Pro Pro Val Ser Pro Gly Lys Thr Val Asn Asp
        675                 680                 685

Val Asn Ser Asn Asn Asn Met Ser Tyr Arg Asn Ala Gly Thr Val Arg
    690                 695                 700

Gln Met Leu Glu Ser Lys Arg Asn Val Ser Glu Ser Ala Pro Pro Ser
705                 710                 715                 720

Phe Gln Thr Pro Val Asn Thr Val Ser Ser Thr Asn Leu Val Thr Pro
                725                 730                 735

Pro Ala Val Val Ser Ser Gln Pro Lys Leu Gln Thr Pro Val Thr Ser
            740                 745                 750

Gly Ser Leu Thr Ala Thr Ser Val Leu Pro Ala Pro Asn Thr Ala Thr
        755                 760                 765

Val Val Ala Thr Thr Gln Val Pro Ser Gly Asn Pro Gln Pro Thr Ile
    770                 775                 780

Ser Leu Gln Pro Leu Pro Val Ile Leu His Val Pro Val Ala Val Ser
785                 790                 795                 800

Ser Gln Pro Gln Leu Leu Gln Ser His Pro Gly Thr Leu Val Thr Asn
                805                 810                 815

Gln Pro Ser Gly Asn Val Glu Phe Ile Ser Val Gln Ser Pro Pro Thr
            820                 825                 830

Val Ser Gly Leu Thr Lys Asn Pro Val Ser Leu Pro Ser Leu Pro Asn
        835                 840                 845

Pro Thr Lys Pro Asn Asn Val Pro Ser Val Pro Ser Pro Ser Ile Gln
    850                 855                 860

Arg Asn Pro Thr Ala Ser Ala Ala Pro Leu Gly Thr Thr Leu Ala Val
865                 870                 875                 880

Gln Ala Val Pro Thr Ala His Ser Ile Val Gln Ala Thr Arg Thr Ser
                885                 890                 895

Leu Pro Thr Val Gly Pro Ser Gly Leu Tyr Ser Pro Ser Thr Asn Arg
            900                 905                 910

Gly Pro Ile Gln Met Lys Ile Pro Ile Ser Ala Phe Ser Thr Ser Ser
        915                 920                 925

Ala Ala Glu Gln Asn Ser Asn Thr Thr Pro Arg Ile Glu Asn Gln Thr
    930                 935                 940

Asn Lys Thr Ile Asp Ala Ser Val Ser Lys Lys Ala Ala Asp Ser Thr
945                 950                 955                 960

Ser Gln Cys Gly Lys Ala Thr Gly Ser Asp Ser Ser Gly Val Ile Asp
                965                 970                 975

Leu Thr Met Asp Asp Glu Glu Ser Gly Ala Ser Gln Asp Pro Lys Lys
            980                 985                 990

Leu Asn His Thr Pro Val Ser Thr  Met Ser Ser Ser Gln  Pro Val Ser
        995                 1000                1005

Arg Pro  Leu Gln Pro Ile Gln  Pro Ala Pro Pro Leu  Gln Pro Ser
    1010                1015                1020

Gly Val  Pro Thr Ser Gly Pro  Ser Gln Thr Thr Ile  His Leu Leu
    1025                1030                1035

Pro Thr  Ala Pro Thr Thr Val  Asn Val Thr His Arg  Pro Val Thr
    1040                1045                1050

Gln Val  Thr Thr Arg Leu Pro  Val Pro Arg Ala Pro  Ala Asn His
    1055                1060                1065

Gln Val  Val Tyr Thr Thr Leu  Pro Ala Pro Pro Ala  Gln Ala Pro
    1070                1075                1080

Leu Arg  Gly Thr Val Met Gln  Ala Pro Ala Val Arg  Gln Val Asn
    1085                1090                1095

Pro Gln  Asn Ser Val Thr Val  Arg Val Pro Gln Thr  Thr Thr Tyr

1100                1105                1110

Val Val Asn Asn Gly Leu Thr Leu Gly Ser Thr Gly Pro Gln Leu
    1115                1120                1125

Thr Val His His Arg Pro Pro Gln Val His Thr Glu Pro Pro Arg
    1130                1135                1140

Pro Val His Pro Ala Pro Leu Pro Glu Ala Pro Gln Pro Gln Arg
    1145                1150                1155

Leu Pro Pro Glu Ala Ala Ser Thr Ser Leu Pro Gln Lys Pro His
    1160                1165                1170

Leu Lys Leu Ala Arg Val Gln Ser Gln Asn Gly Ile Val Leu Ser
    1175                1180                1185

Trp Ser Val Leu Glu Val Asp Arg Ser Cys Ala Thr Val Asp Ser
    1190                1195                1200

Tyr His Leu Tyr Ala Tyr His Glu Glu Pro Ser Ala Thr Val Pro
    1205                1210                1215

Ser Gln Trp Lys Lys Ile Gly Glu Val Lys Ala Leu Pro Leu Pro
    1220                1225                1230

Met Ala Cys Thr Leu Thr Gln Phe Val Ser Gly Ser Lys Tyr Tyr
    1235                1240                1245

Phe Ala Val Arg Ala Lys Asp Ile Tyr Gly Arg Phe Gly Pro Phe
    1250                1255                1260

Cys Asp Pro Gln Ser Thr Asp Val Ile Ser Ser Thr Gln Ser Ser
    1265                1270                1275

<210>  9
<211>  5718
<212>  DNA
<213>  Homo sapiens

<220>
<221>  CDS
<222>  (470)..(3790)

<400>  9
ctcctgaggc tgccagcagc cagcagtgac tgcccgccct atctgggacc caggatcgct      60
ctgtgagcaa cttggagcca gagaggagat caacaaggag gaggagagag ccggcccctc     120
agccctgctg cccagcagca gcctgtgctc gccctgccca acgcagacag ccagacccag     180
ggcggcccct ctggcggctc tgctcctccc gaaggatgct tggggagtga ggcgaagctg 240
ggccgctcct ctcccctaca gcagccccct tcctccatcc ctctgttctc ctgagccttc 300
aggagcctgc accagtcctg cctgtccttc tactcagctg ttacccactc tgggaccagc 360
agtctttctg ataactggga gagggcagta aggaggactt cctggagggg gtgactgtcc 420
agagcctgga actgtgccca caccagaagc catcagcagc aaggacacc atg cgg ctt 478
ccg ggt gcg atg cca gct ctg gcc ctc aaa ggc gag ctg ctg ttg ctg 526
tct ctc ctg tta ctt ctg gaa cca cag atc tct cag ggc ctg gtc gtc 574
aca ccc ccg ggg cca gag ctt gtc ctc aat gtc tcc agc acc ttc gtt 622
ctg acc tgc tcg ggt tca gct ccg gtg gtg tgg gaa cgg atg tcc cag 670
gag ccc cca cag gaa atg gcc aag gcc cag gat ggc acc ttc tcc agc 718
gtg ctc aca ctg acc aac ctc act ggg cta gac acg gga gaa tac ttt 766
tgc acc cac aat gac tcc cgt gga ctg gag acc gat gag cgg aaa cgg 814
ctc tac atc ttt gtg cca gat ccc acc gtg ggc ttc ctc cct aat gat 862
gcc gag gaa cta ttc atc ttt ctc acg gaa ata act gag atc acc att 910
cca tgc cga gta aca gac cca cag ctg gtg gtg aca ctg cac gag aag 958
aaa ggg gac gtt gca ctg cct gtc ccc tat gat cac caa cgt ggc ttt 1006
tct ggt atc ttt gag gac aga agc tac atc tgc aaa acc acc att ggg 1054
gac agg gag gtg gat tct gat gcc tac tat gtc tac aga ctc cag gtg 1102
tca tcc atc aac gtc tct gtg aac gca gtg cag act gtg gtc cgc cag 1150
ggt gag aac atc acc ctc atg tgc att gtg atc ggg aat gag gtg gtc 1198
aac ttc gag tgg aca tac ccc cgc aaa gaa agt ggg cgg ctg gtg gag 1246
ccg gtg act gac ttc ctc ttg gat atg cct tac cac atc cgc tcc atc 1294
ctg cac atc ccc agt gcc gag tta gaa gac tcg ggg acc tac acc tgc 1342
aat gtg acg gag agt gtg aat gac cat cag gat gaa aag gcc atc aac 1390
atc acc gtg gtt gag agc ggc tac gtg cgg ctc ctg gga gag gtg ggc 1438
aca cta caa ttt gct gag ctg cat cgg agc cgg aca ctg cag gta gtg 1486
ttc gag gcc tac cca ccg ccc act gtc ctg tgg ttc aaa gac aac cgc 1534
acc ctg ggc gac tcc agc gct ggc gaa atc gcc ctg tcc acg cgc aac 1582
gtg tcg gag acc cgg tat gtg tca gag ctg aca ctg gtt cgc gtg aag 1630
gtg gca gag gct ggc cac tac acc atg cgg gcc ttc cat gag gat gct 1678
gag gtc cag ctc tcc ttc cag cta cag atc aat gtc cct gtc cga gtg 1726
ctg gag cta agt gag agc cac cct gac agt ggg gaa cag aca gtc cgc 1774
tgt cgt ggc cgg ggc atg ccc cag ccg aac atc atc tgg tct gcc tgc 1822
aga gac ctc aaa agg tgt cca cgt gag ctg ccg ccc acg ctg ctg ggg 1870
aac agt tcc gaa gag gag agc cag ctg gag act aac gtg acg tac tgg      1918
gag gag gag cag gag ttt gag gtg gtg agc aca ctg cgt ctg cag cac      1966
gtg gat cgg cca ctg tcg gtg cgc tgc acg ctg cgc aac gct gtg ggc      2014
cag gac acg cag gag gtc atc gtg gtg cca cac tcc ttg ccc ttt aag      2062
gtg gtg gtg atc tca gcc atc ctg gcc ctg gtg gtg ctc acc atc atc      2110
tcc ctt atc atc ctc atc atg ctt tgg cag aag aag cca cgt tac gag      2158
atc cga tgg aag gtg att gag tct gtg agc tct gac ggc cat gag tac      2206
atc tac gtg gac ccc atg cag ctg ccc tat gac tcc acg tgg gag ctg      2254
ccg cgg gac cag ctt gtg ctg gga cgc acc ctc ggc tct ggg gcc ttt      2302
ggg cag gtg gtg gag gcc acg gct cat ggc ctg agc cat tct cag gcc      2350
acg atg aaa gtg gcc gtc aag atg ctt aaa tcc aca gcc cgc agc agt      2398
gag aag caa gcc ctt atg tcg gag ctg aag atc atg agt cac ctt ggg      2446
ccc cac ctg aac gtg gtc aac ctg ttg ggg gcc tgc acc aaa gga gga      2494
ccc atc tat atc atc act gag tac tgc cgc tac gga gac ctg gtg gac      2542
tac ctg cac cgc aac aaa cac acc ttc ctg cag cac cac tcc gac aag      2590
cgc cgc ccg ccc agc gcg gag ctc tac agc aat gct ctg ccc gtt ggg 2638
ctc ccc ctg ccc agc cat gtg tcc ttg acc ggg gag agc gac ggt ggc 2686
tac atg gac atg agc aag gac gag tcg gtg gac tat gtg ccc atg ctg 2734
gac atg aaa gga gac gtc aaa tat gca gac atc gag tcc tcc aac tac 2782
atg gcc cct tac gat aac tac gtt ccc tct gcc cct gag agg acc tgc 2830
cga gca act ttg atc aac gag tct cca gtg cta agc tac atg gac ctc 2878
gtg ggc ttc agc tac cag gtg gcc aat ggc atg gag ttt ctg gcc tcc 2926
aag aac tgc gtc cac aga gac ctg gcg gct agg aac gtg ctc atc tgt 2974
gaa ggc aag ctg gtc aag atc tgt gac ttt ggc ctg gct cga gac atc 3022
atg cgg gac tcg aat tac atc tcc aaa ggc agc acc ttt ttg cct tta 3070
aag tgg atg gct ccg gag agc atc ttc aac agc ctc tac acc acc ctg 3118
agc gac gtg tgg tcc ttc ggg atc ctg ctc tgg gag atc ttc acc ttg 3166
ggt ggc acc cct tac cca gag ctg ccc atg aac gag cag ttc tac aat 3214
gcc atc aaa cgg ggt tac cgc atg gcc cag cct gcc cat gcc tcc gac 3262
gag atc tat gag atc atg cag aag tgc tgg gaa gag aag ttt gag att 3310
cgg ccc ccc ttc tcc cag ctg gtg ctg ctt ctc gag aga ctg ttg ggc 3358
gaa ggt tac aaa aag aag tac cag cag gtg gat gag gag ttt ctg agg     3406
agt gac cac cca gcc atc ctt cgg tcc cag gcc cgc ttg cct ggg ttc     3454
cat ggc ctc cga tct  ccc ctg gac acc agc  tcc gtc ctc tat act     3499
gcc gtg cag ccc aat  gag ggt gac aac gac  tat atc atc ccc ctg     3544
cct gac ccc aaa ccc  gag gtt gct gac gag  ggc cca ctg gag ggt     3589
tcc ccc agc cta gcc  agc tcc acc ctg aat  gaa gtc aac acc tcc     3634
tca acc atc tcc tgt  gac agc ccc ctg gag  ccc cag gac gaa cca     3679
gag cca gag ccc cag  ctt gag ctc cag gtg  gag ccg gag cca gag     3724
ctg gaa cag ttg ccg  gat tcg ggg tgc cct  gcg cct cgg gcg gaa     3769
gca gag gat agc ttc  ctg tag ggggctggcc cctaccctgc cctgcctgaa     3820
gctccccccc tgccagcacc cagcatctcc tggcctggcc tgaccgggct tcctgtcagc     3880
caggctgccc ttatcagctg tccccttctg gaagctttct gctcctgacg tgttgtgccc     3940
caaaccctgg ggctggctta ggaggcaaga aaactgcagg ggccgtgacc agccctctgc     4000
ctccagggag gccaactgac tctgagccag ggttccccca gggaactcag ttttcccata     4060
tgtaagatgg gaaagttagg cttgatgacc cagaatctag gattctctcc ctggctgaca     4120
ggtggggaga ccgaatccct ccctgggaag attcttggag ttactgaggt ggtaaattaa     4180
cttttttctg ttcagccagc tacccctcaa ggaatcatag ctctctcctc gcacttttat     4240
ccacccagga gctagggaag agaccctagc ctccctggct gctggctgag ctagggccta     4300
gccttgagca gtgttgcctc atccagaaga aagccagtct cctccctatg atgccagtcc     4360
ctgcgttccc tggcccgagc tggtctgggg ccattaggca gcctaattaa tgctggaggc     4420
tgagccaagt acaggacacc cccagcctgc agcccttgcc cagggcactt ggagcacacg     4480
cagccatagc aagtgcctgt gtccctgtcc ttcaggccca tcagtcctgg ggctttttct 4540
ttatcaccct cagtcttaat ccatccacca gagtctagaa ggccagacgg gccccgcatc 4600
tgtgatgaga atgtaaatgt gccagtgtgg agtggccacg tgtgtgtgcc agtatatggc 4660
cctggctctg cattggacct gctatgaggc tttggaggaa tccctcaccc tctctgggcc 4720
tcagtttccc cttcaaaaaa tgaataagtc ggacttatta actctgagtg ccttgccagc 4780
actaacattc tagagtattc caggtggttg cacatttgtc cagatgaagc aaggccatat 4840
accctaaact tccatcctgg gggtcagctg ggctcctggg agattccaga tcacacatca 4900
cactctgggg actcaggaac catgcccctt ccccaggccc ccagcaagtc tcaagaacac 4960
agctgcacag gccttgactt agagtgacag ccggtgtcct ggaaagcccc cagcagctgc 5020
cccagggaca tgggaagacc acgggacctc tttcactacc cacgatgacc tccgggggta 5080
tcctgggcaa aagggacaaa gagggcaaat gagatcacct cctgcagccc accactccag 5140
cacctgtgcc gaggtctgcg tcgaagacag aatggacagt gaggacagtt atgtcttgta 5200
aaagacaaga agcttcagat gggtacccca agaaggatgt gagaggtggg cgctttggag 5260
gtttgcccct cacccaccag ctgccccatc cctgaggcag cgctccatgg gggtatggtt 5320
ttgtcactgc ccagacctag cagtgacatc tcattgtccc cagcccagtg ggcattggag 5380
gtgccagggg agtcagggtt gtagccaaga cgcccccgca cggggagggt tgggaagggg 5440
gtgcaggaag ctcaacccct ctgggcacca accctgcatt gcaggttggc accttacttc 5500
cctgggatcc ccagagttgg tccaaggagg gagagtgggt tctcaatacg gtaccaaaga 5560
tataatcacc taggtttaca aatattttta ggactcacgt taactcacat ttatacagca 5620
gaaatgctat tttgtatgct gttaagtttt tctatctgtg tacttttttt taagggaaag 5680
attttaatat taaacctggt gcttctcact cacaaaaa 5718

<210> 10
<211> 1106
<212> PRT
<213> Homo sapiens

<400> 10

<210>  11
<211>  22
<212>  DNA
<213>  Artificial Sequence

<220>
<223>  Forward primer

<400>  11
caagtggacc atctcagacc ac                                                22

<210>  12
<211>  22
<212>  DNA
<213>  Artificial Sequence

<220>
<223>  Reverse primer

<400>  12
atttaagcat cttgacggcc ac                                                22

<210>  13
<211>  30
<212>  DNA
<213>  Artificial Sequence

<220>
<223>  ATF7IP-1 Fwd

<400>  13
ttcagaatgg acagtttaga agaacctcag                                        30

<210>  14
<211>  20
<212>  DNA
<213>  Artificial Sequence

<220>
<223>  ATF7IP-1 Rev

<400>  14
tctgctggag agcacgtttc                                                   20

<210>  15
<211>  23
<212>  DNA
<213>  Artificial Sequence

<220>
<223>  ATF7IP-2 Fwd

<400>  15
aagcttgcac cttctgagga tga                                               23


<210>  16
<211>  20
<212>  DNA
<213>  Artificial Sequence

<220>
<223>  ATF7IP-2 Rev

<400>  16
atcggatctc gtaacgtggc                                                   20


<210>  17
<211>  28
<212>  DNA
<213>  Artificial Sequence

<220>
<223>  ATF7IP-PDGFRB-3 Fwd

<400>  17
tggtcctctc tgatgaagag gatatttc                                          28


<210>  18
<211>  20
<212>  DNA
<213>  Artificial Sequence

<220>
<223>  ATF7IP-PDGFRB-3 Rev

<400>  18
tcatcgtggc ctgagaatgg                                                   20


<210>  19
<211>  25
<212>  DNA
<213>  Artificial Sequence

<220>
<223>  EBF1-PDGFRB Fwd

<400>  19
atgtttggga ttcaggaaag catcc                                             25


<210>  20
<211>  20
<212>  DNA

<213>  Artificial Sequence

<220>
<223>  EBF1-PDGFRB Rev

<400>  20
atgcggtaac cccgtttgat                                                    20


<210>  21
<211>  22
<212>  DNA
<213>  Artificial Sequence

<220>
<223>  ATF7IP-PDGFRB detection Fwd-1

<400>  21
cgtgaatgta acacatcgtc ca                                                 22


<210>  22
<211>  21
<212>  DNA
<213>  Artificial Sequence

<220>
<223>  ATF7IP-PDGFRB detection Rev-1

<400>  22
agctcccacg tggagtcata g                                                  21


<210>  23
<211>  20
<212>  DNA
<213>  Artificial Sequence

<220>
<223>  EBF1-PDGFRBdetection Fwd-1

<400>  23
gggcgtgaat tcgttcagtg                                                    20


<210>  24
<211>  20
<212>  DNA
<213>  Artificial Sequence

<220>
<223>  EBF1-PDGFRB detection Rev-1

<400>  24
atcggatctc gtaacgtggc                                                    20


<210>  25
<211>  20
<212>  DNA
<213>  Artificial Sequence

<220>

<223>  WT-PDGFRB detection Fwd-1

<400>  25
tccagcacct tcgttctgac                                                    20


<210>  26
<211>  20
<212>  DNA
<213>  Artificial Sequence

<220>
<223>  WT-PDGFRB detection Rev-1

<400>  26
ccagaaaagc cacgttggtg                                                    20

## Description

### TECHNICAL FIELD

The present invention relates mainly to a method for detecting a gene fusion serving as a responsible mutation for cancer, and a method for identifying patients with cancer or subjects with a risk of cancer, in whom a substance suppressing the expression and/or activity of a polypeptide encoded by a fusion polynucleotide produced by said gene fusion show a therapeutic effect.

### BACKGROUND ART

Cancer is the first-ranked disease among causes of death in Japan. Cancer cells are known to become malignant due to genetic alterations. Identifying genetic alterations characteristic of cancer type is expected to contribute not only to the clarification of carcinogenic mechanisms but also to, for example, the diagnosis and prognosis prediction of cancers and the development and selection of a cancer therapy.

Chimeric genes (hereinafter also referred to as "fusion genes") are formed by a mechanism in which two genes encoding proteins which inherently function as completely distinct molecules fuse together due to abnormalities such as chromosomal translocation, and as a consequence, those chimeric genes produce fusion proteins having abnormal functions. Chimeric genes are known to trigger the development of leukemia and other cancers, and are used as indicators for diagnosis and as targets for drug discovery. Particularly in recent years, there have been increasing examples of successful development of molecular targeted therapies against cancers having chimeric genes associated with constituents (e.g., kinases) of carcinogenesis-related signaling pathways. For example, it is reported that tyrosine kinase inhibitors (e.g., crizotinib) targeting ALK protein are effective in the treatment of breast cancer patients bearing the EML4-ALK fusion gene, and that tyrosine kinase inhibitors (e.g., imatinib) targeting the protein encoded by the BCR-ABL fusion gene are effective in the treatment of leukemia patients bearing this fusion gene.

Acute lymphoblastic leukemia (ALL) is a cancer in which lymphocytes become malignant at an immature stage and grow abnormally. ALL develops at any age from infancy to adulthood, and is known as the most common cancer in infancy. As for ALL, many chimeric genes have been reported as genetic alterations. For example, Non Patent Literature 1 discloses that, in order to identify genetic alternations in IKZF1-mutated, BCR-ABL1-negative ALL with poor outcome (Ph-like ALL), RNA sequencing and whole genome sequencing were performed on B-progenitor ALL (B-ALL) cases identified as Ph-like ALL, thereby identifying the in-frame fusions NUP214-ABL1, EBF1-PDGFRB, BCR-JAK2, STRN3-JAK2, PAX5-JAK2, ETV6-ABL1, RANBP2-ABL1, and RCSD1-ABL1. This literature also discloses that the EBF1-PDGFRB, BCR-JAK2, and NUP214-ABL1 fusions are potential targets for tyrosine kinase inhibitor therapy.

### CITATION LIST

### NON PATENT LITERATURE

[Non Patent Literature 1] Roberts K. G., et al., Cancer Cell, 2012, 22 (2), 153-66.

### SUMMARY OF INVENTION

### TECHNICAL PROBLEM

Identification of mutations in various cancers including ALL and other leukemias has not yet been made thoroughly, and there is still a demand for further identification of mutations that can serve as indicators for predicting the effectiveness of drug treatments.

Accordingly, the objects of the present invention include but are not limited to the following: to identify a mutation that can serve as an indicator for predicting the effectiveness of drug treatments in various cancers including ALL and other leukemias; to provide a means for detecting said mutation; and to provide a means for identifying, based on said mutations, patients with cancer or subjects with a risk of cancer, in whom a drug targeting a gene having said mutation or a protein encoded by said gene shows a therapeutic effect.

### SOLUTION TO PROBLEM

As the result of conducting intensive studies with a view to achieving the above-mentioned objects, the present inventors identified the ATF7IP-PDGFRB gene as a novel fusion gene expressed in acute lymphoblastic leukemia (ALL). As for the PDGFRB gene, the fusion gene of this gene with the EBF1 gene has been reported in Non Patent Literature 1 noted above, but the presence of the fusion gene of this gene with the ATF7IP gene was wholly unexpected even considering any prior art literatures including the aforementioned one.

The ATF7IP-PDGFRB gene is considered to trigger the development of ALL as a result of producing a fusion protein between a SETDB1-binding protein (ATF7IP) and a tyrosine kinase (PDGFRB) and delivering a normally absent growth stimulus to cells. Therefore, targeted drugs such as tyrosine kinase inhibitors are highly likely to exhibit therapeutic effects against cancers positive for this gene fusion. In fact, it was confirmed that the tyrosine kinase inhibitor, dasatinib, is effective for ATF7IP-PDGFRB gene fusion-positive ALL patients.

The present inventors have made further studies based on these findings and completed the present invention.

More specifically, the present invention is defined as follows.
[1] A method for detecting a gene fusion, the method comprising the step of detecting an ATF7IP-PDGFRB fusion polypeptide comprising the SETDB1-binding domain of ATF7IP and the transmembrane region and kinase domain of PDGFRB, and having kinase activity, or a fusion polynucleotide encoding said polypeptide, in an isolated sample from a subject.
[2] The method according to [1], wherein the fusion polypeptide is any one of (i) to (iii) mentioned below:
   (i) a polypeptide consisting of the amino acid sequence of SEQ ID NO: 2 or 4,
   (ii) a polypeptide consisting of an amino acid sequence derived from the amino acid sequence of SEQ ID NO: 2 or 4 by deletion, substitution or addition of one or more amino acids, and the polypeptide having kinase activity, or
   (iii) a polypeptide consisting of an amino acid sequence having a sequence identity of at least 80% to the amino acid sequence of SEQ ID NO: 2 or 4, and the polypeptide having kinase activity.
[3] The method according to [1] or [2], wherein the fusion polynucleotide is any one of (i) to (iv) mentioned below:
   (i) a polynucleotide consisting of the nucleotide sequence of SEQ ID NO: 1 or 3,
   (ii) a polynucleotide that hybridizes under stringent conditions with a polynucleotide consisting of a nucleotide sequence complementary to the polynucleotide consisting of the nucleotide sequence of SEQ ID NO: 1 or 3, and which encodes a polypeptide having kinase activity,
   (iii) a polynucleotide that consists of a nucleotide sequence derived from the nucleotide sequence of SEQ ID NO: 1 or 3 by deletion, substitution or addition of one or more nucleotides, and which encodes a polypeptide having kinase activity, or
   (iv) a polynucleotide that has a sequence identity of at least 80% to the polynucleotide consisting of the nucleotide sequence of SEQ ID NO: 1 or 3, and which encodes a polypeptide having kinase activity.
[4] The method according to any one of [1] to [3], wherein the gene fusion is a responsible mutation (driver mutation) for cancer.
[5] The method according to [4], wherein the cancer is acute lymphoblastic leukemia.
[6] A method for identifying a patient with cancer or a subject with a risk of cancer, in whom a substance suppressing the expression and/or activity of a polypeptide encoded by a fusion polynucleotide produced by a gene fusion serving as a responsible mutation (driver mutation) for cancer shows a therapeutic effect, the method comprising the steps of:
   (1) detecting an ATF7IP-PDGFRB fusion polypeptide comprising the SETDB1-binding domain of ATF7IP and the transmembrane region and kinase domain of PDGFRB, and having kinase activity, or a fusion polynucleotide encoding said polypeptide, in an isolated sample from a subject, and
   (2) determining that the substance suppressing the expression and/or activity of the polypeptide shows a therapeutic effect in the subject, in the case where the fusion polypeptide or the fusion polynucleotide encoding said polypeptide is detected.
[7] A kit for detecting a gene fusion, the kit comprising any or a combination of (A) to (C) mentioned below:
   (A) a polynucleotide that serves as a probe designed to specifically recognize an ATF7IP-PDGFRB fusion polynucleotide;
   (B) polynucleotides that serve as a pair of primers designed to enable specific amplification of an ATF7IP-PDGFRB fusion polynucleotide; or
   (C) an antibody that specifically recognizes an ATF7IP-PDGFRB fusion polypeptide.
[8] The kit according to [7], wherein the gene fusion is a responsible mutation (driver mutation) for cancer.
[9] An isolated ATF7IP-PDGFRB fusion polypeptide or a fragment thereof, which comprises the SETDB1-binding domain of ATF7IP and the transmembrane region and kinase domain of PDGFRB, and has kinase activity.
[10] A polynucleotide encoding the fusion polypeptide or the fragment thereof according to [9].
[11] A therapeutic agent for ATF7IP-PDGFRB gene fusion-positive cancer.
[12] The therapeutic agent according to [11], comprising, as an active ingredient, a substance suppressing the expression and/or activity of an ATF7IP-PDGFRB fusion polypeptide comprising the SETDB1-binding domain of ATF7IP and the transmembrane region and kinase domain of PDGFRB, and having kinase activity.
[13] The therapeutic agent according to [11] or [12], wherein the cancer is acute lymphoblastic leukemia.
[14] The therapeutic agent according to any one of [11] to [13], wherein the substance suppressing the expression and/or activity of an ATF7IP-PDGFRB fusion polypeptide comprising the SETDB1-binding domain of ATF7IP and the transmembrane region and kinase domain of PDGFRB, and having kinase activity is a substance inhibiting the kinase activity of PDGFRB.
[15] The therapeutic agent according to [14], wherein the substance inhibiting the kinase activity of PDGFRB is imatinib mesylate, dasatinib, nilotinib, ponatinib, rebastinib or bafetinib.
[16] The therapeutic agent according to [14], wherein the substance inhibiting the kinase activity of PDGFRB is dasatinib.
[17] A drug for use in the treatment of cancer, the drug comprising a substance suppressing the expression and/or activity of an ATF7IP-PDGFRB fusion polypeptide comprising the SETDB1-binding domain of ATF7IP and the transmembrane region and kinase domain of PDGFRB, and having kinase activity.
[18] The drug according to [17], wherein the cancer is ATF7IP-PDGFRB gene fusion-positive cancer.
[19] The drug according to [17] or [18], wherein the cancer is acute lymphoblastic leukemia.
[20] The drug according to any one of [17] to [19], wherein the substance suppressing the expression and/or activity of an ATF7IP-PDGFRB fusion polypeptide comprising the SETDB1-binding domain of ATF7IP and the transmembrane region and kinase domain of PDGFRB, and having kinase activity is a substance inhibiting the kinase activity of PDGFRB, preferably imatinib mesylate, dasatinib, nilotinib, ponatinib, rebastinib or bafetinib, more preferably dasatinib.
[21] A method for treatment of cancer, comprising the step of administering to a subject a therapeutically effective amount of a substance suppressing the expression and/or activity of an ATF7IP-PDGFRB fusion polypeptide comprising the SETDB1-binding domain of ATF7IP and the transmembrane region and kinase domain of PDGFRB, and having kinase activity.
[22] The method according to [21], wherein the subject is a patient with ATF7IP-PDGFRB gene fusion-positive cancer.
[23] The method according to [21], wherein the subject is a patient with acute lymphoblastic leukemia.
[24] The method according to [21], wherein the substance suppressing the expression and/or activity of an ATF7IP-PDGFRB fusion polypeptide comprising the SETDB1-binding domain of ATF7IP and the transmembrane region and kinase domain of PDGFRB, and having kinase activity is a substance inhibiting the kinase activity of PDGFRB.
[25] The method according to [24], wherein the substance inhibiting the kinase activity of PDGFRB is imatinib mesylate, dasatinib, nilotinib, ponatinib, rebastinib or bafetinib.
[26] The method according to [24], wherein the substance inhibiting the kinase activity of PDGFRB is dasatinib.
[27] A method for screening a cancer therapeutic agent, the method comprising the steps of:
   (1) bringing a test substance into contact with a cell that expresses an ATF7IP-PDGFRB fusion polypeptide comprising the SETDB1-binding domain of ATF7IP and the transmembrane region and kinase domain of PDGFRB, and having kinase activity;
   (2) determining whether the expression and/or activity of the fusion polypeptide is suppressed or not; and
   (3) selecting the substance determined to suppress the expression and/or activity of the fusion polypeptide, as a cancer therapeutic agent.

### ADVANTAGEOUS EFFECTS OF INVENTION

The present invention makes it possible to detect an unknown responsible mutation for the particular cancer, which has been first identified according to the present invention; to identify, based on the presence of said responsible mutation, patients with said cancer or subjects with a risk of said cancer, in whom a cancer treatment takes effect; and to treat said patients.

### BRIEF DESCRIPTION OF DRAWINGS

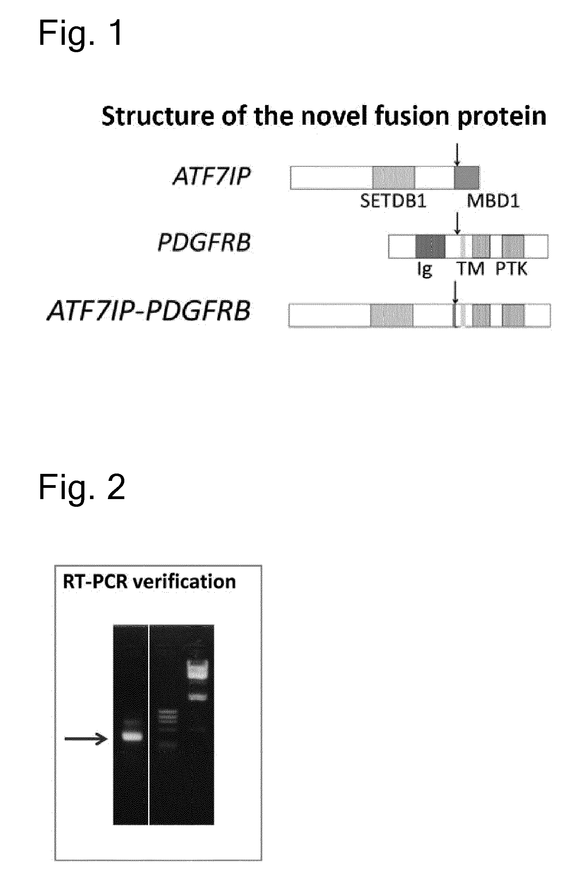

[Fig. 1] Fig. 1 is a schematic diagram showing an example of the domain structure of the ATF7IP-PDGFRB fusion protein. The downward arrows indicate the breakpoints in the ATF7IP protein and PDGFRB protein, and the point of fusion in the ATF7IP-PDGFRB fusion protein. Abbreviations: SETDB 1, SETDB1-binding domain; MBD 1, MBD1-binding domain; Ig, Ig-like domain; TM, Transmembrane region; and PTK, Kinase domain.
[Fig. 2] Fig. 2 is an electrophoretograph showing the results of detecting the ATF7IP-PDGFRB gene fusion by RT-PCR with cDNA synthesized from leukemic cell-derived RNA being used as a template. The arrow indicates the amplification of a gene fragment which implies a gene fusion.

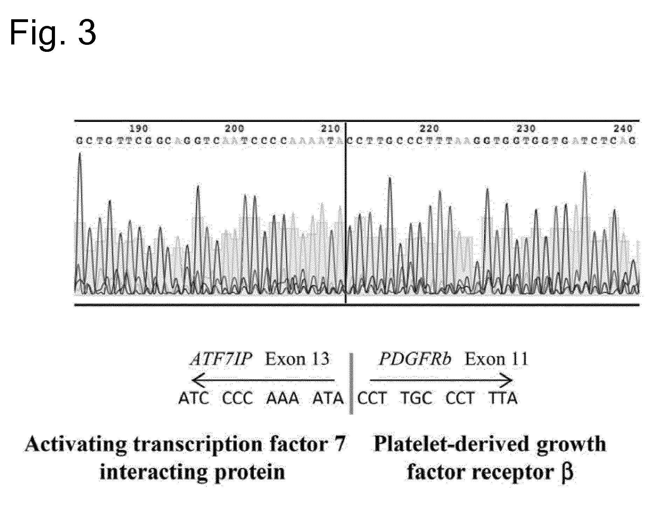

[Fig. 3] Fig. 3 shows the results of nucleotide sequencing an amplified gene fragment.

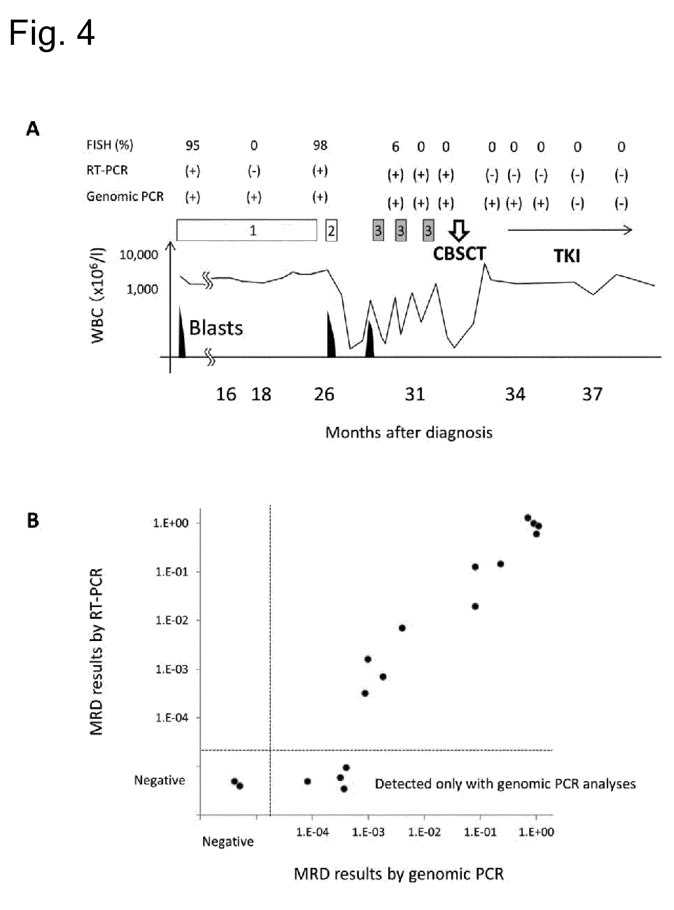

[Fig. 4] Fig. 4 shows the clinical course of a patient with ATF7IP/PDGFRB-positive ALL. (A) The therapeutic response was evaluated by fluorescence *in situ* hybridization (FISH), RT-PCR (Kobayashi K., et al., Br J Haematol, 2014, 165, 836-841), and genomic PCR. Chemotherapy regimens were as follows: block 1: standard-risk ALL therapy (Tokyo Children's Cancer Study Group's study L99-15), maintenance therapy was started at 16 months after the initial diagnosis; block 2: ALL re-induction therapy; and block 3: AML regimen (Tsukimoto I., et al., J Clin Oncol, 2009, 27, 4007-4013). Abbreviations: CBSCT, cord blood stem cell transplantation; TKI, tyrosine kinase inhibitor; and FISH, fluorescence *in situ* hybridization. (B) Results of detecting MRD in 17 samples using RT-PCR and genomic PCR. The levels of detection by genomic PCR and RT-PCR are indicated on the x-axis and y-axis, respectively. In 2 samples, no MRD was detected by either of the two methods; in 4 samples, MRD was detected only by genomic PCR; and in 11 samples, MRD was detected within the quantitative range by both of the two methods. For the 4 samples with discordant results, the genomic PCR products were verified by clonal sequencing to rule out possibly incidental false-positive results. The dotted lines represent the thresholds for the quantifiable range of MRD detection.

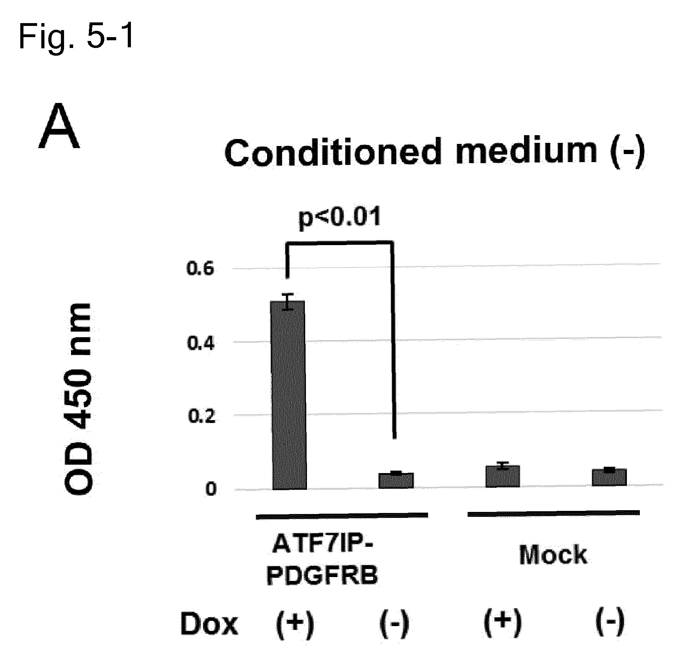

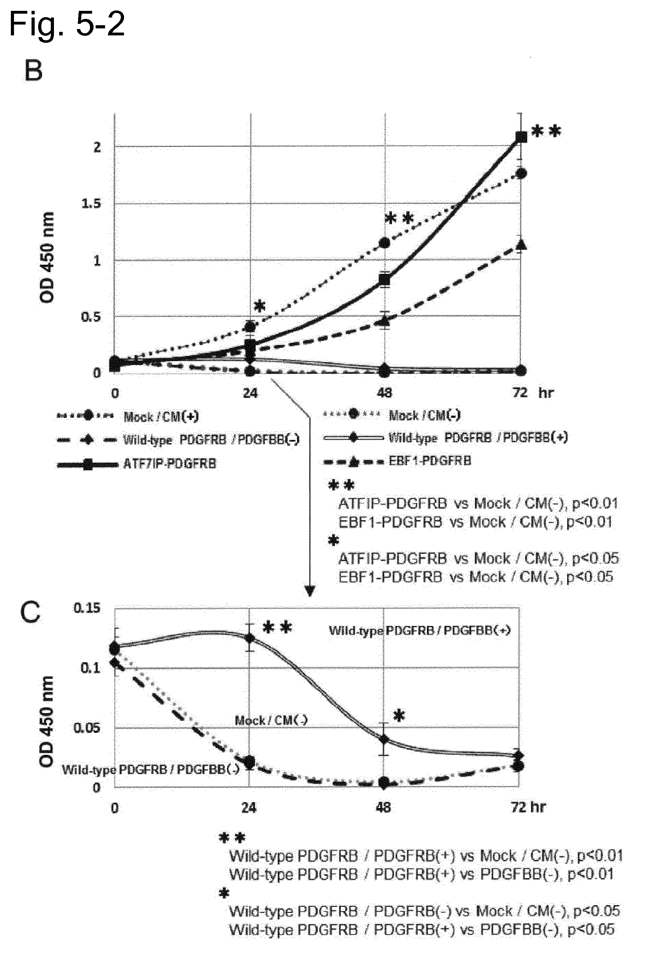

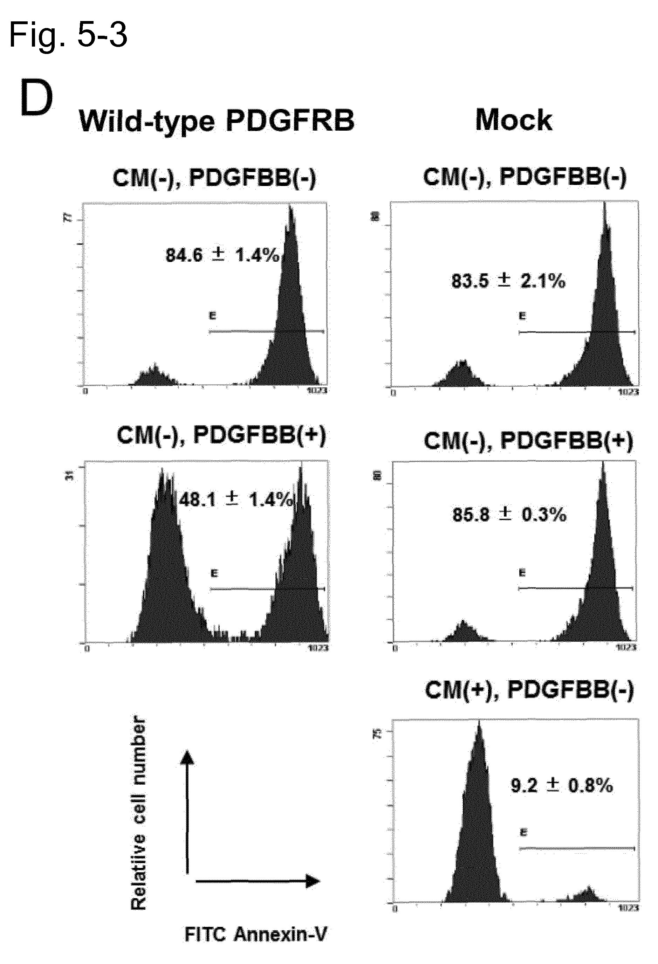

[Fig. 5-1] Fig. 5 illustrates that IL-3-independent cell proliferation is induced by ATF7IP- and EBF1-PDGFRB but not by wild-type (WT-) PDGFRB. (A) Ba/F3 cells were transfected with an ATF7IP-PDGFRB-expressing retroviral vector or an empty vector (Mock) by employing a tetracycline-inducible system. After 10-hour exposure to retrovirus followed by 24-hour treatment with or without doxycycline (Dox), the cells were washed to withdraw conditioned medium. After 60-hour incubation, the numbers of living cells were estimated by water-soluble tetrazolium salt (WST) assay. The mean ± SEM of triplicates for each treatment is indicated by a bar graph.
[Fig. 5-2] (B) Cloned Ba/F3 cells transfected with ATF7IP-PDGFRB, EBF1-PDGFRB, and wild-type PDGFRB (WT-PDGFRB) were cultured in the absence of WEHI-3-conditioned medium (CM) for the indicated periods, and examined by WST assay. WT-PDGFRB transfectants were tested under the following two conditions, *i.e.,* in the presence or absence of 100 ng/mL of PDGFBB. As a control, Ba/F3 cells transfected with an empty vector in the presence or absence of WEHI-3-conditioned medium were also examined. The OD values are expressed as the mean ± SEM of triplicates. The data shown are representative of three independent experiments. (C) The results for WT-PDGFRB with or without PDGFBB and for Mock without conditioned medium, as shown in (B), were extracted and plotted on a graph with magnified Y-axis.
[Fig. 5-3] (D) The same sample preparations of WT-PDGFRB transfectants as in (B) were examined by Annexin V-binding assay. As controls, Mock cells treated with or without PDGFBB and/or conditioned medium were also similarly examined. Experiments were performed in triplicate. For each type of samples, a typical cytogram and the mean ± SEM of positivity (%) are presented. X-axis: fluorescence intensity; Y-axis: relative cell number.

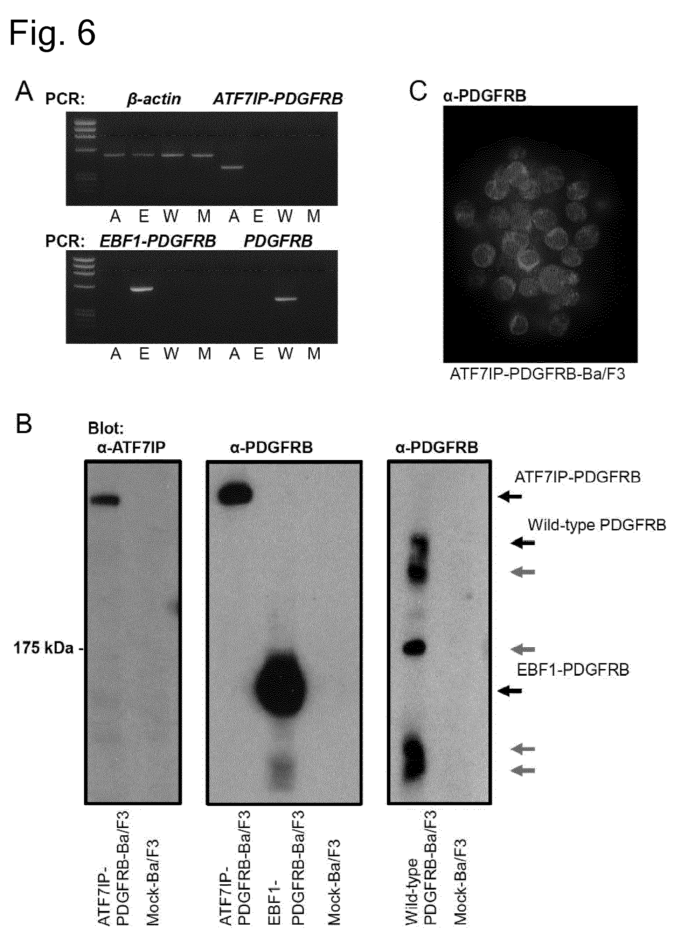

[Fig. 6] Fig. 6 shows the expression of PDGFRB-related chimeric molecules and wild-type (WT-) PDGFRB in transfectants. (A) Total RNAs were extracted from 1 × 10⁷ Ba/F3 cells transfected with ATF7IP-PDGFRB (A), EBF1-PDGFRB (E), WT-PDGFRB (W), and an empty vector (Mock, M), and the expressions of the different genes and the internal control β-actin were examined by PCR. (B) The expressions of different types of PDGFRB were examined by immunoblot analysis. In the case of ATF7IP-PDGFRB, the fusion protein was detected by any of anti-ATF7IP antibody and anti-PDGFRB antibody. The gray arrows indicate several unexpected bands with smaller molecular weights than predicted, as detected in the case of WT-PDGFRB. (C) The expression of ATF7IP-PDGFRB in Ba/F3 cells was detected by immunocytochemistry.

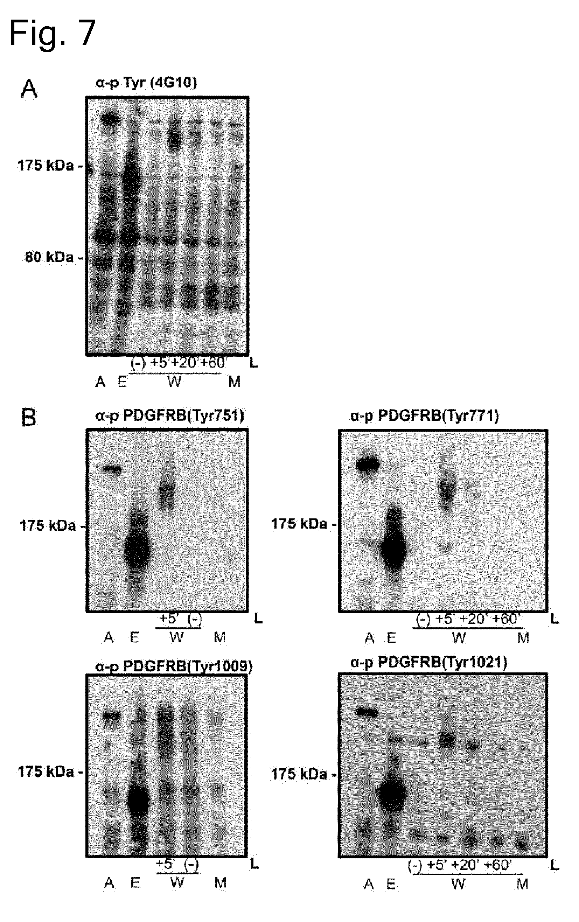

[Fig. 7] Fig. 7 shows increased tyrosine phosphorylation mediated by the expression of PDGFRBs. (A) Cell lysates were prepared from Ba/F3 transfectants, and protein tyrosine phosphorylation was detected by immunoblotting using the anti-phosphotyrosine antibody 4G10. (B) The same sample preparations as in (A) were examined in the same way using phospho-specific anti-PDGFRB antibodies for the tyrosine residues indicated in the figure. Abbreviations: A, ATF7IP-PDGFRB; E, EBF1-PDGFRB; W, WT-PDGFRB; L, with ligand stimulation (100 ng/mL of PDGFBB); and M, Mock/empty vector.

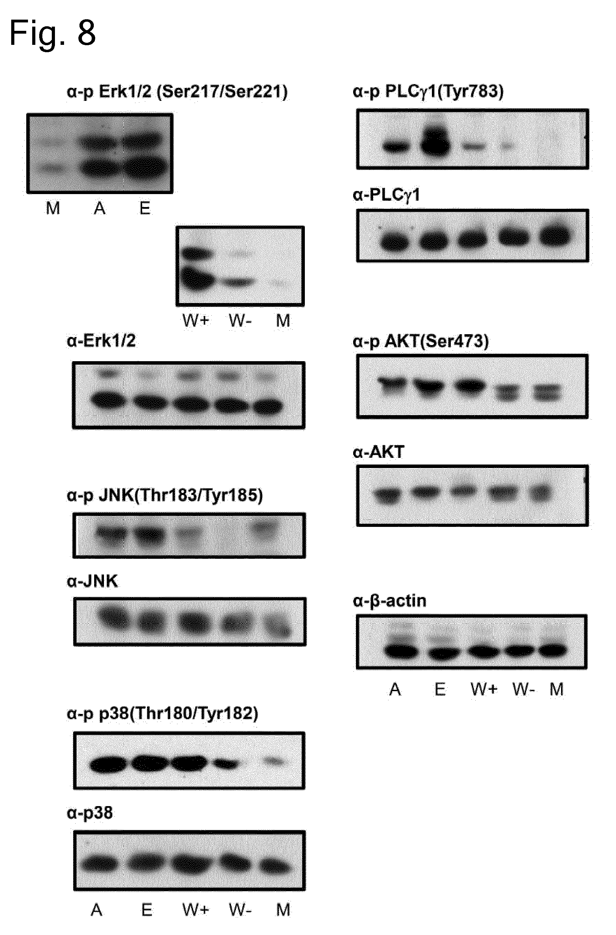

[Fig. 8] Fig. 8 shows enhanced phosphorylation of signaling molecules located downstream of PDGFRB. The same sample preparations as in Fig. 7 were examined in the same way using the antibodies indicated in the figure. Abbreviations: A, ATF7IP-PDGFRB; E, EBF1-PDGFRB; W, WT-PDGFRB; +, with ligand stimulation (with 100 ng/mL of PDGFBB for 5 minutes); -, without ligand stimulation; and M, Mock/empty vector.

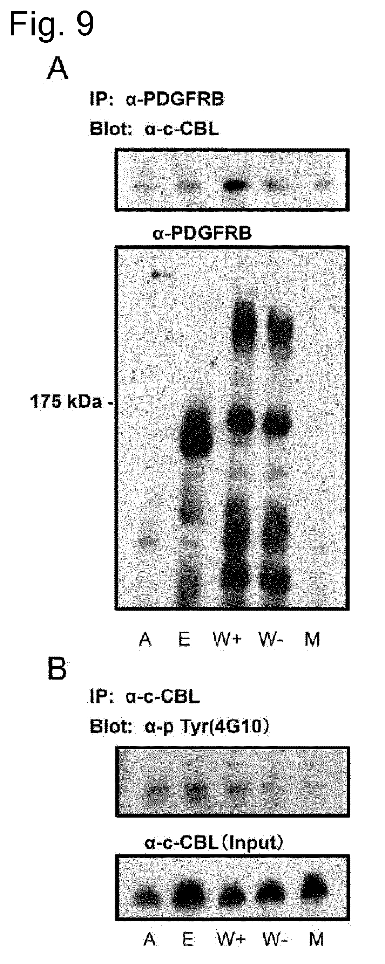

[Fig. 9] Fig. 9 shows phosphorylation and binding to PDGFRBs of c-Cbl in Ba/F3 transfectants. (A) Immunoprecipitation was performed on the indicated cell lysates using an anti-PDGFRB antibody. After separation on SDS-PAGE, immunoblot was performed with either an anti-c-Cbl or anti-PDGFRB antibody. (B) Using the same sample preparations, immunoprecipitation and immunoblot analysis were performed with anti-c-Cbl and phosphotyrosine antibodies, respectively. Abbreviations: A, ATF7IP-PDGFRB; E, EBF1-PDGFRB; W+, WT-PDGFRB with PDGFBB (100 ng/mL for 5 minutes); W-, WT-PDGFRB without PDGFBB; and M, Mock/empty vector.

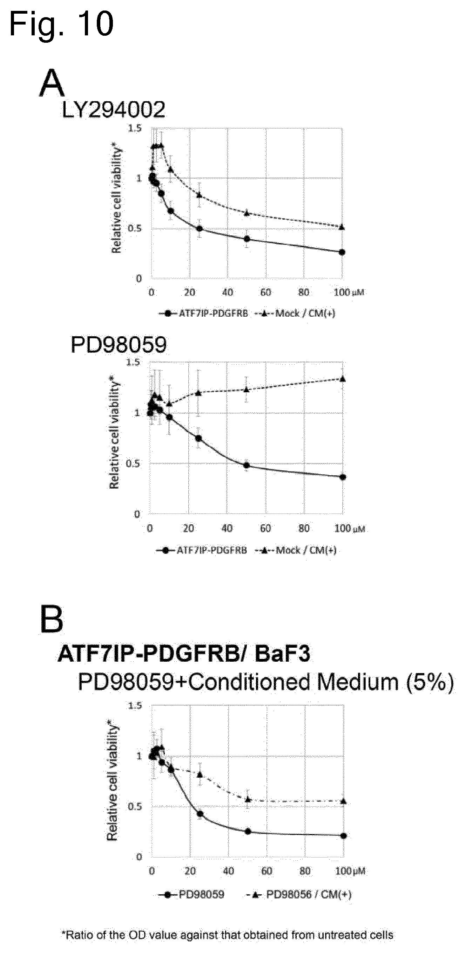

[Fig. 10] Fig. 10 shows the effects of PI3 kinase and MEK inhibitors on proliferation of Ba/F3 transfectants bearing PDGFRBs. (A) ATF7IP-PDGFRB-expressing Ba/F3 cells in the absence of WEHI-3-conditioned medium (CM) and Mock Ba/F3 cells in the presence of CM were each treated with or without either the PI3 kinase inhibitor LY294002 or the MEK inhibitor PD98059 at the indicated concentrations for 24 hours. Subsequently, the number of living cells was estimated for each type of the cells by WST assay in the same way as in Fig. 5B. The ratio of the OD value obtained from the inhibitor-treated cells relative to that obtained from the inhibitor-untreated cells was calculated and expressed as the means ± SEM of triplicates. (B) ATF7IP-PDGFRB-expressing Ba/F3 cells were treated with PD98059 in the presence or absence of CM, and examined in the same way as in (A).

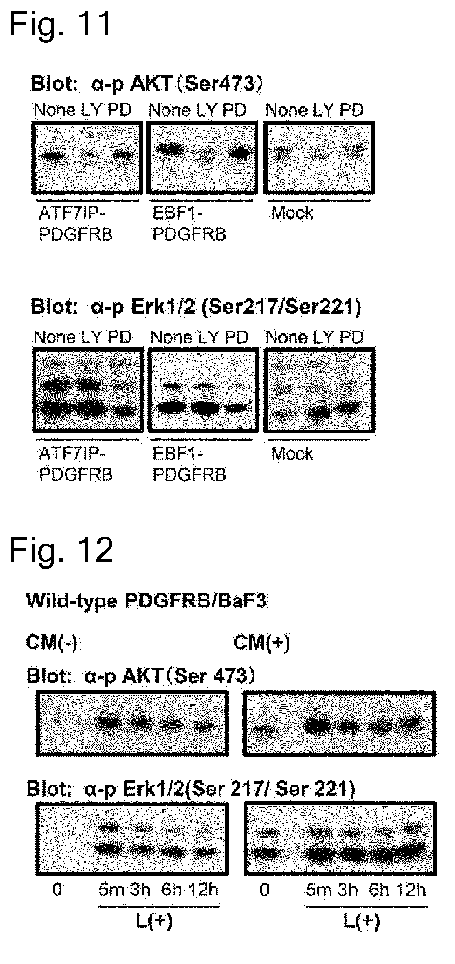

[Fig. 11] Fig. 11 shows the effects of PI3 kinase and MEK inhibitors on the phosphorylation of AKT and MAP kinases. Cell lysates were prepared from the same sample preparations as in Fig. 10. Immunoblot analysis was performed in the same way as in Fig. 8. Abbreviations: None, without inhibitor; LY, with LY294002; and PD, with PD98059.
[Fig. 12] Fig. 12 shows the effects of PDGFBB and IL-3 on the phosphorylation of AKT and MAP kinases. WT-PDGFRB-expressing cells were maintained in the presence (CM(+)) or absence (CM(-)) of WEHI-3-conditioned medium for 12 hours. After stimulation with PDGFBB, cell lysates were prepared for the indicated periods. Immunoblot analysis was performed in the same way as in Fig. 8.

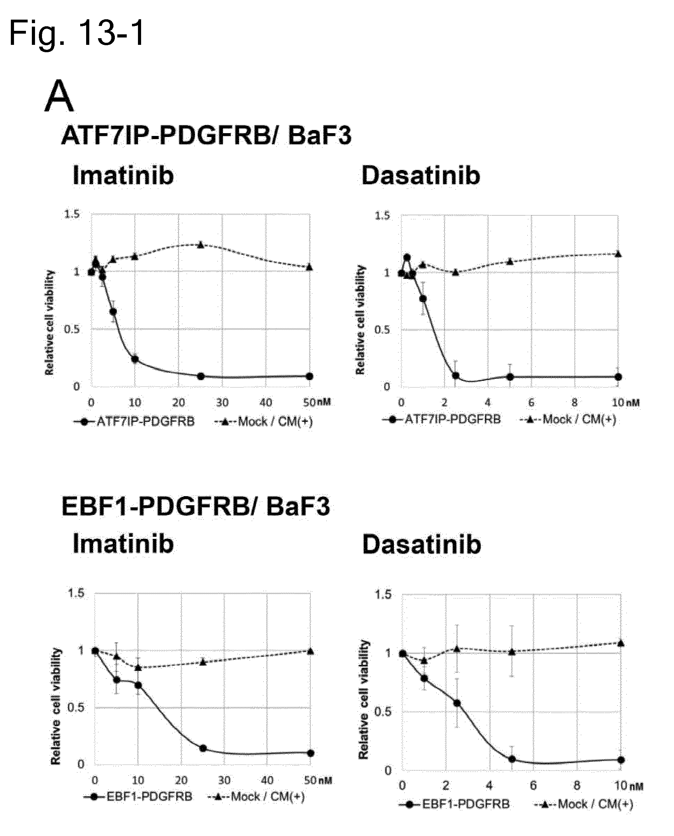

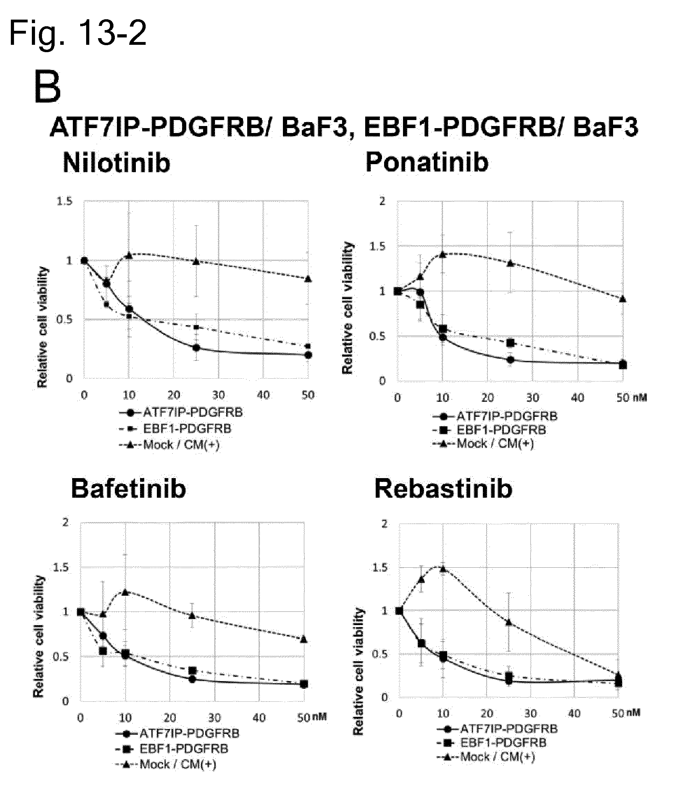

[Fig. 13-1] Fig. 13 shows the effects of tyrosine kinase inhibitors on proliferation of Ba/F3 transfectants bearing PDGFRBs. (A) ATF7IP- or EBF1-PDGFRB-expressing Ba/F3 cells and Mock Ba/F3 cells maintained in the presence of conditioned medium (CM(+)) were treated with or without tyrosine kinase inhibitors at the indicated concentrations for 24 hours. Subsequently, the number of living cells was estimated for each type of the cells by WST assay in the same way as in Fig. 5B. The ratio of the OD value obtained from the inhibitor-treated cells relative to that obtained from the inhibitor-untreated cells was calculated and expressed as the means ± SEM of triplicates.
[Fig. 13-2] (B) The effects of nilotinib, bafetinib, rebastinib, and ponatinib on proliferation of Ba/F3 transfectants bearing PDGFRBs.

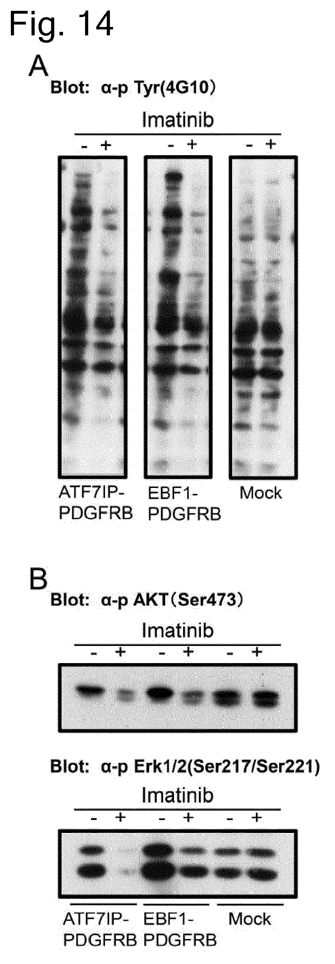

[Fig. 14] Fig. 14 shows the effects of tyrosine kinase inhibitors on phosphorylation of AKT and MAP kinases. Cell lysates were prepared from the same sample preparations treated with 100 nm of imatinib for 12 hours as in Fig. 13. Immunoblot analysis was performed as in Fig. 8.

### DESCRIPTION OF EMBODIMENTS

As disclosed below in Examples, the present inventors first found the ATF7IP-PDGFRB gene fusion serving as a responsible mutation (driver mutations) for cancer, in cancer tissues. On the basis of this finding, the present invention mainly provides a method for detecting said gene fusion; a method for identifying, based on the presence of said responsible mutation, a patient with cancer or a subject with a risk of cancer, in whom a cancer treatment takes effect; a method for treatment of cancer; a cancer therapeutic agent; and a method for screening a cancer therapeutic agent.

For the purpose of the present invention, the term "responsible mutation for cancer" is a term used interchangeably with the term "driver mutation", and refers to a mutation that is present in cancer tissues and which is capable of inducing carcinogenesis of cells. Typically speaking, if a mutation is found in a cancer tissue in which none of known oncogene mutations exists (in other words, if a mutation exists mutually exclusively with the known oncogene mutations), then the mutation can be said as a responsible mutation for cancer.

### <Specific responsible mutation for cancer>

Hereafter, the inventive gene fusion is specifically explained. For the purpose of the present specification, the "point of fusion" in a fusion polynucleotide refers to a boundary that connects gene segments extending toward the 5'- and 3'-ends, in other words, a boundary between two nucleotide residues. The "point of fusion" in a fusion polypeptide refers to a boundary that connects polypeptides extending toward the N- and C-termini, in other words, a boundary between two amino acid residues, or, if a gene fusion occurs in one codon, one amino acid residue *per se* encoded by the codon.

The ATF7IP-PDGFRB gene fusion is a mutation that expresses a fusion protein between ATF7IP protein and PDGFRB protein (hereinafter also referred to as the "ATF7IP-PDGFRB fusion polypeptide") and which is caused by a translocation (t(5;12)) having breakpoints in regions 12p13.1 and 5q33.1 of a human chromosome.

ATF7IP (activating transcription factor 7 interacting protein 1) is a protein also known as MCAF1, which is encoded by the gene located on chromosome 12p13.1 in humans, and is typified by a protein consisting of the amino acid sequence of SEQ ID NO: 6 or 8 (NCBI accession No. NP_060649.3 (26-Oct-2013) or NP_851997.1 (08-Nov-2013)). Although the function of ATF7IP protein is yet to be fully clarified, the protein is believed to bind a transcription factor to regulate its transcriptional activity. ATF7IP protein is characterized by having the SETDB1-binding domain and the MBD1-binding domain (Fig. 1). The SETDB1-binding domain corresponds to, for example, the amino acid sequence at positions 562 to 817 of the amino acid sequence of SEQ ID NO: 6 in humans. The MBD1-binding domain corresponds to, for example, the amino acid sequence at positions 1154 to 1270 of the amino acid sequence of SEQ ID NO: 6 in humans.

PDGFRB protein is a protein encoded by the gene located on chromosome 5q33.1 in humans, and is typified by a protein consisting of the amino acid sequence of SEQ ID NO: 10 (NCBI accession No. NP_002600.1 (27-Oct-2013)). PDGFRB protein is characterized by having an Ig-like domain, a transmembrane region, and a kinase domain (Fig. 1). The Ig-like domain corresponds to, for example, the amino acid sequence at positions 228 to 415 of the amino acid sequence of SEQ ID NO: 10 in humans. The transmembrane region corresponds to, for example, the amino acid sequence at position 533 to 553 of the amino acid sequence of SEQ ID NO: 10 in humans. The kinase domain corresponds to, for example, the amino acid sequence at position 562 to 953 of the amino acid sequence of SEQ ID NO: 10 in humans.

The ATF7IP-PDGFRB fusion polypeptide is a polypeptide comprising the SETDB1-binding domain of ATF7IP protein and the transmembrane region and kinase domain of PDGFRB protein, and having kinase activity.

The ATF7IP-PDGFRB fusion polypeptide can contain all of the SETDB1-binding domain of ATF7IP protein, or can contain part of the SETDB1-binding domain as long as said part of the domain is capable of binding SETDB1. Whether said part of the SETDB1-binding domain is capable of binding SETDB1 can be confirmed by analysis by GST pull-down assay as described in J Biol Chem, 2005, 280 (14), 13928-35.

The ATF7IP-PDGFRB fusion polypeptide can contain all, part or none of the MBD1-binding domain of ATF7IP protein.

The ATF7IP-PDGFRB fusion polypeptide can contain all of the kinase domain of PDGFRB protein, or can contain part of the kinase domain as long as the ATF7IP-PDGFRB fusion polypeptide has kinase activity.

The expression saying that the ATF7IP-PDGFRB fusion polypeptide "has kinase activity" means that said fusion polypeptide is active as an enzyme phosphorylating tyrosine due to the kinase domain derived from PDGFRB protein. The kinase activity of the ATF7IP-PDGFRB fusion polypeptide is determined by a conventional method, and is commonly determined by incubating the fusion polypeptide with a substrate (e.g., synthetic peptide substrate) and ATP under appropriate conditions and then detecting phosphorylated tyrosine in the substrate. The kinase activity can also be measured using a commercially available measurement kit.

The ATF7IP-PDGFRB fusion polypeptide can contain all or part of the transmembrane region of PDGFRB protein, but is preferable to contain all of the transmembrane region.

In the present invention, the polynucleotide encoding the ATF7IP-PDGFRB fusion polypeptide (hereinafter also referred to as the "ATF7IP-PDGFRB fusion polynucleotide") is a polynucleotide that encodes the polypeptide comprising the SETDB1-binding domain of ATF7IP protein and the transmembrane region and kinase domain of PDGFRB protein, and having kinase activity. The ATF7IP-PDGFRB fusion polynucleotide can be in the form of any of mRNA, cDNA and genomic DNA.

The ATF7IP-PDGFRB fusion polynucleotide according to the present invention can be, for example, an ATF7IP-PDGFRB fusion polynucleotide encoding the polypeptide mentioned below:
(i) a polypeptide consisting of the amino acid sequence of SEQ ID NO: 2 or 4,
(ii) a polypeptide consisting of an amino acid sequence derived from the amino acid sequence of SEQ ID NO: 2 or 4 by deletion, substitution or addition of one or more amino acids, and the polypeptide having kinase activity, or
(iii) a polypeptide consisting of an amino acid sequence having a sequence identity of at least 80% to the amino acid sequence of SEQ ID NO: 2 or 4, and the polypeptide having kinase activity.

As disclosed below in Examples, the amino acid sequence of SEQ ID NO: 2 or 4 is an amino acid sequence encoded by the ATF7IP-PDGFRB fusion polynucleotide comprising partial sequences, which was found in samples from human cancer tissues.

As used above in (ii), the phrase "one or more amino acids" refers to generally 1 to 50 amino acids, preferably 1 to 30 amino acids, more preferably 1 to 10 amino acids, still more preferably one to several amino acids (for example, 1 to 5 amino acids, 1 to 4 amino acids, 1 to 3 amino acids, 1 or 2 amino acids, or one amino acid).

As used above in (iii), the phrase "sequence identity of at least 80%" refers to a sequence identity of preferably at least 85%, more preferably at least 90% or at least 95%, still more preferably at least 97%, at least 98% or at least 99%. Amino acid sequence identity can be determined using the BLASTX or BLASTP program (Altschul S. F., et al., J. Mol. Biol., 1990, 215: 403) which is based on the BLAST algorithm developed by Karlin and Altschul (Proc. Natl. Acad. Sci. USA, 1990, 87: 2264-2268; and Proc. Natl. Acad. Sci. USA, 1993, 90: 5873). In the process of making amino acid sequence analysis using BLASTX, the parameter setting is typically made as follows: score = 50 and wordlength = 3. In the process of making amino acid sequence analysis using the BLAST and Gapped BLAST programs, the default parameters of these programs are used. The specific procedures for conducting these analyses are well known to those skilled in the art (*e.g.,* http://www.ncbi.nlm.nih.gov/).

Also, the ATF7IP-PDGFRB fusion polynucleotide according to the present invention can be, for example, any one of the polynucleotides mentioned below:
(i) a polynucleotide consisting of the nucleotide sequence of SEQ ID NO: 1 or 3,
(ii) a polynucleotide that hybridizes under stringent conditions with a polynucleotide consisting of a nucleotide sequence complementary to the polynucleotide consisting of the nucleotide sequence of SEQ ID NO: 1 or 3, and which encodes a polypeptide having kinase activity,
(iii) a polynucleotide that consists of a nucleotide sequence derived from the nucleotide sequence of SEQ ID NO: 1 or 3 by deletion, substitution or addition of one or more nucleotides, and which encodes a polypeptide having kinase activity, or
(iv) a polynucleotide that has a sequence identity of at least 80% to the polynucleotide consisting of the nucleotide sequence of SEQ ID NO: 1 or 3, and which encodes a polypeptide having kinase activity.

As disclosed below in Examples, the nucleotide sequence of SEQ ID NO: 1 or 3 is a nucleotide sequence of an ATF7IP-PDGFRB fusion polynucleotide comprising partial sequences, which is found in samples from human cancer tissues.

As used above in (ii), the phrase "under stringent conditions" refers to moderately or highly stringent conditions, unless particularly specified.

The moderately stringent conditions can be easily designed by those skilled in the art on the basis of, for example, the length of the polynucleotide of interest. Basic conditions are described in Sambrook, et al., Molecular Cloning: A Laboratory Manual, 3rd ed., ch. 6-7, Cold Spring Harbor Laboratory Press, 2001. Typically, the moderately stringent conditions comprise: prewashing of a nitrocellulose filter in 5×SSC, 0.5% SDS, 1.0 mM EDTA (pH 8.0); hybridization in ca. 50% formamide, 2-6×SSC at about 40-50°C (or any other similar hybridization solution like a Stark's solution in ca. 50% formamide at about 42°C); and washing of the filter in 0.5-6×SSC, 0.1% SDS at about 40°C-60°C. The moderately stringent conditions preferably comprise hybridization in 6×SSC at about 50°C, and may comprise the prewashing and/or washing under the above-mentioned conditions.

The highly stringent conditions can also be easily designed by those skilled in the art on the basis of, for example, the length of the polynucleotide of interest. The highly stringent conditions involve a higher temperature and/or a lower salt concentration than the moderately stringent conditions. Typically, the highly stringent conditions comprise hybridization in 0.2-6×SSC, preferably 6×SSC, more preferably 2×SSC, still more preferably 0.2×SSC, at about 65°C. In any case, the highly stringent conditions preferably comprise washing in 0.2×SSC, 0.1% SDS at about 65-68°C.

In any case, as a buffer for use in hybridization, prewashing and washing, SSPE (1×SSPE: 0.15 M NaCl, 10 mM NaH₂PO₄, and 1.25 mM EDTA, pH 7.4) can be used in place of SSC (1×SSC: 0.15 M NaCl and 15 mM sodium citrate). In any case, washing can be done for about 15 minutes after the completion of hybridization.

As used above in (iii), the phrase "one or more nucleotides" refers to generally 1 to 50 nucleotides, preferably 1 to 30 nucleotides, more preferably 1 to 10 nucleotides, still more preferably one to several nucleotides (for example, 1 to 5 nucleotides, 1 to 4 nucleotides, 1 to 3 nucleotides, 1 or 2 nucleotides, or one nucleotide).

As used above in (iv), the phrase "sequence identity of at least 80%" refers to a sequence identity of preferably at least 85%, more preferably at least 90% or at least 95%, still more preferably at least 97%, at least 98% or at least 99%. Nucleotide sequence identity can be determined using the BLASTN program (Altschul S. F., et al., J. Mol. Biol., 1990, 215: 403) which is based on the above-mentioned BLAST algorithm. In the process of making nucleotide sequence analysis using BLASTN, the parameter setting is typically made as follows: score = 100 and wordlength = 12.

### <Method for detecting a gene fusion>

The present invention provides a method for detecting the ATF7IP-PDGFRB gene fusion (hereinafter also referred to as the "inventive detection method"). This gene fusion preferably causes a responsible mutation (driver mutation) for cancer. The inventive detection method comprises the step of detecting an ATF7IP-PDGFRB fusion polynucleotide or a polypeptide encoded thereby, in an isolated sample from a subject.

In the inventive detection method, the subject is not particularly limited as long as it is a mammal. Examples of the mammal include: rodents such as mouse, rat, hamster, chipmunk and guinea pig; rabbit, pig, cow, goat, horse, sheep, mink, dog, cat; and primates such as human, monkey, cynomolgus monkey, rhesus monkey, marmoset, orangutan, and chimpanzee, with human being preferred.

The subject may be not only a subject affected by cancer, but also a subject suspected of being affected by cancer or a subject with a future risk of cancer. The "cancer" to which the inventive detection method is to be applied is not particularly limited as long as it is a cancer in which the ATF7IP-PDGFRB gene fusion can be detected, with leukemia being preferred, acute lymphoblastic leukemia (ALL) being more preferred, and B-progenitor acute lymphoblastic leukemia (B-ALL) being particularly preferred.

The "isolated sample" from the subject encompasses not only biological samples (for example, cells, tissues, organs (e.g., small intestine, spleen, kidney, liver, stomach, lung, adrenal gland, heart, brain, pancreas, and aorta), body fluids *(e.g.,* blood, lymphs, bone marrow fluid), digestive juices, sputum, bronchoalveolar/bronchial lavage fluids, urine, and feces), but also nucleic acid extracts from these biological samples (e.g., genomic DNA extracts, mRNA extracts, and cDNA and cRNA preparations from mRNA extracts) and protein extracts. The genomic DNA, mRNA, cDNA or protein can be prepared by those skilled in the art through considering various factors including the type and state of the sample and selecting a known technique suitable therefor. The sample may also be the one that is fixed with formalin or alcohol, frozen, or embedded in paraffin.

Further, the "isolated sample" is preferably derived from a tissue, organ or body fluid affected or suspected of being affected by the above-mentioned cancer, and more preferably derived from a blood or bone marrow fluid affected or suspected of being affected by the above-mentioned cancer. Examples of the "isolated sample" include, but are not limited to, cells derived from the blood or bone marrow fluid of a patient with leukemia (preferably ALL, more preferably B-ALL) or a subject suspected of being affected by leukemia, and Total RNAs extracted from said cells.

In the inventive detection method, the detection of a fusion polynucleotide or a polypeptide encoded thereby can be made using a *per se* known technique.

If the object to be detected is a transcript from a genomic DNA (mRNA, or cDNA prepared from mRNA), a fusion polynucleotide in the form of mRNA or cDNA can be detected using, for example, RT-PCR, sequencing, TaqMan probe method, Northern blotting, dot blotting, or cDNA microarray analysis.

If the object to be detected is a genomic DNA, a fusion polynucleotide in the form of genomic DNA can be detected using, for example, *in situ* hybridization (ISH), genomic PCR, sequencing, TaqMan probe method, Southern blotting, or genome microarray analysis.

Any of the above-mentioned detection techniques can be used alone or in combination. For example, since the ATF7IP-PDGFRB gene fusion is believed to contribute to carcinogenesis by expressing an ATF7IP-PDGFRB fusion polypeptide, it is also preferred that if a fusion polynucleotide in the form of genomic DNA is detected (*e.g.,* by *in situ* hybridization or the like), production of a transcript or a protein should be further confirmed (e.g., by RT-PCR, immunostaining or the like).

If a fusion polynucleotide is detected by a hybridization technique (*e.g.*, TaqMan probe method, Northern blotting, Southern blotting, dot blotting, microarray analysis, *in situ* hybridization (ISH)), there can be used a polynucleotide that serves as a probe designed to specifically recognize the fusion polynucleotide. As used herein, the phrase "specifically recognize the fusion polynucleotide" means that under stringent conditions, the probe distinguishes and recognizes the fusion polynucleotide from other polynucleotides, including wild-type genes from which to derive both segments of the fusion polynucleotide each extending from the point of fusion toward the 5'- or 3'-end.

Since biological samples (e.g., biopsy samples) obtained in the course of treatment or diagnosis are often fixed in formalin, it is preferred to use *in situ* hybridization in the inventive detection method, from the viewpoints that the genomic DNA to be detected is stable even when fixed in formalin and that the detection sensitivity is high.

According to *in situ* hybridization, the genomic DNA (fusion polynucleotide) encoding a fusion polypeptide can be detected by hybridizing, to such a biological sample, the following polynucleotide (a) or (b) which has a chain length of at least 15 nucleotides and serves as a probe(s) designed to specifically recognize said fusion polynucleotide:
(a) a polynucleotide serving as at least one probe selected from the group consisting of a probe that hybridizes to the nucleotide sequence of the fusion partner gene toward the 5'-end (ATF7IP gene) and a probe that hybridizes to the nucleotide sequence of the fusion partner gene toward the 3'-end (PDGFRB gene); or
(b) a polynucleotide serving as a probe that hybridizes to a nucleotide sequence containing the point of fusion between the fusion partner gene toward the 5'-end and the fusion partner gene toward the 3'-end.

The ATF7IP gene according to the present invention, as far as it is derived from humans, and is typified by a gene consisting of the DNA sequence from position 14518566 to position 14655864 in the genome sequence identified in Genbank accession No.NC_000012.11.

The PDGFRB gene according to the present invention, as far as it is derived from humans, and is typified by a gene consisting of the DNA sequence (encoded into complementary strand) from position 149493402 to position 149535447 in the genome sequence identified in Genbank accession No. NC_000005.9.

However, the DNA sequences of genes can change in nature (*i.e.,* in a non-artificial way) due to their mutations and the like. Thus, such native mutants can also be encompassed by the present invention (the same applies hereinafter).

The polynucleotide mentioned in (a) according to the present invention can be of any type as far as it is capable of detecting the presence of the genomic DNA encoding a fusion polypeptide in the above-mentioned biological sample by hybridizing to a nucleotide sequence(s) targeted by said polynucleotide, *i.e.,* the nucleotide sequence of the fusion partner gene toward the 5'-end (ATF7IP gene) and/or the nucleotide sequence of the fusion partner gene toward the 3'-end (PDGFRB gene); preferably, the polynucleotide (a) is any of the polynucleotides mentioned below in (a1) to (a3):
(a1) a combination of a polynucleotide that hybridizes to the nucleotide sequence of that region of the fusion partner gene toward the 5'-end which extends upstream from its breakpoint toward the 5'-end (this polynucleotide is hereinafter also referred to as the "5' fusion partner gene probe 1"), and a polynucleotide that hybridizes to the nucleotide sequence of that region of the fusion partner gene toward the 3'-end which extends downstream from its breakpoint toward the 3'-end (this polynucleotide is hereinafter also referred to as the "3' fusion partner gene probe 1");
(a2) a combination of a polynucleotide that hybridizes to the nucleotide sequence of that region of the fusion partner gene toward the 5'-end which extends upstream from its breakpoint toward the 5'-end (this polynucleotide is hereinafter also referred to as the "5' fusion partner gene probe 1"), and a polynucleotide that hybridizes to the nucleotide sequence of that region of the fusion partner gene toward the 5'-end which extends downstream from its breakpoint toward the 3'-end (this polynucleotide is hereinafter also referred to as the "5' fusion partner gene probe 2"); and
(a3) a combination of a polynucleotide that hybridizes to the nucleotide sequence of that region of the fusion partner gene toward the 3'-end which extends upstream from its breakpoint toward the 5'-end (this polynucleotide is hereinafter also referred to as the "3' fusion partner gene probe 2"), and a polynucleotide that hybridizes to the nucleotide sequence of that region of the fusion partner gene toward the 3'-end which extends downstream from its breakpoint toward the 3'-end (this polynucleotide is hereinafter also referred to as the "3' fusion partner gene probe 1").

As for (a1) to (a3) mentioned above, the nucleotide sequence of that region of the fusion partner gene toward the 5'-end which extends upstream from its breakpoint toward the 5'-end typically contains a cording region for all or part of the SETDB1-binding domain of ATF7IP protein.

As for (a1) to (a3) mentioned above, the nucleotide sequence of that region of the fusion partner gene toward the 3'-end which extends downstream from its breakpoint toward the 3'-end typically contains cording regions for all or part of the transmembrane region of PDGFRB protein and for all or part of the kinase domain of the same protein.

The polynucleotides mentioned above in (a1) can be exemplified by the polynucleotide combination mentioned below in (a1-1):
(a1-1) a combination of a polynucleotide that hybridizes to a coding region for all or part of the SETDB1-binding domain of ATF7IP protein, and a polynucleotide that hybridizes to a cording region for all or part of the kinase domain of PDGFRB protein.

In the present invention, the region to which the polynucleotide for use for *in situ* hybridization as mentioned above in (a) is to hybridize (such a region is hereinafter referred to as the "target nucleotide sequence") is preferred to be located not more than 1000000 nucleotides away from the point of fusion between the fusion partner gene toward the 5'-end (ATF7IP gene) and the fusion partner gene toward the 3'-end (PDGFRB gene), from the viewpoints of specificity for the target nucleotide sequence and detection sensitivity.

In the present invention, the polynucleotide for use for *in situ* hybridization as mentioned above in (b) can be of any type as far as it is capable of detecting the presence of the genomic DNA encoding a fusion polypeptide in the above-mentioned biological sample by hybridizing to a nucleotide sequence targeted by said polynucleotide, *i.e.,* a nucleotide sequence containing the point of fusion between the fusion partner gene toward the 5'-end and the fusion partner gene toward the 3'-end; and typical examples of the polynucleotide (b) are those which each hybridize to a nucleotide sequence containing the point of fusion in the nucleotide sequence of SEQ ID NO: 1 or 3.

Further, in the present invention, the polynucleotide for use for *in situ* hybridization as mentioned above in (a) or (b) is preferred to be a group consisting of multiple types of polynucleotides which can cover the entire target nucleotide sequence, from the viewpoints of specificity for the target nucleotide sequence and detection sensitivity. In such a case, each of the polynucleotides constituting the group has a length of at least 15 nucleotides, preferably from 100 to 1000 nucleotides.

The polynucleotide for use for *in situ* hybridization as mentioned above in (a) or (b) is preferably labeled with a fluorescent dye or the like for the purpose of detection. Examples of such a fluorescent dye include, but are not limited to, DEAC, FITC, R6G, TexRed, and Cy5. Aside from the fluorescent dye, the polynucleotide may also be labeled with a radioactive isotope (*e.g.,* ¹²⁵I, ¹³¹I, ³H, ¹⁴C, ³³P, ³²P), an enzyme (*e.g.,* β-galactosidase, β-glucosidase, alkaline phosphatase, peroxidase, malate dehydrogenase), or a luminescent substance (e.g., luminol, luminol derivative, luciferin, lucigenin, 3,3'-diaminobenzidine (DAB)).

When *in situ* hybridization is performed using a combination of 5' fusion partner gene probe 1 and 3' fusion partner gene probe 1, a combination of 5' fusion partner gene probe 1 and 5' fusion partner gene probe 2, or a combination of 3' fusion partner gene probe 2 and 3' fusion partner gene probe 1, the probes of each combination are preferably labeled with different dyes from each other. If, as the result of *in situ* hybridization using such a combination of probes labeled with different dyes, an overlap is observed between the signal (e.g., fluorescence) emitted from the label on 5' fusion partner gene probe 1 and the signal emitted from the label on 3' fusion partner gene probe 1, then it can be determined that a genomic DNA encoding a fusion polypeptide of interest has been detected successfully. Also, if a split is observed between the signal emitted from the label on 5' fusion partner gene probe 1 and the signal emitted from the label on 5' fusion partner gene probe 2, or between the signal emitted from the label on 3' fusion partner gene probe 2 and the signal emitted from the label on 3' fusion partner gene probe 1, then it can be determined that a genomic DNA encoding a fusion polypeptide of interest has been detected successfully.

Polynucleotide labeling can be effected by a known method. For example, polynucleotides can be labeled by nick translation or random priming, in which the polynucleotides are caused to incorporate substrate nucleotides labeled with a fluorescent dye or the like.

The conditions for hybridizing the polynucleotide mentioned above in (a) or (b) to the above-mentioned biological sample by *in situ* hybridization can vary with various factors including the length of said polynucleotide; and exemplary highly stringent hybridization conditions are 0.2×SSC at 65°C, and exemplary low stringent hybridization conditions are 2.0×SSC at 50°C. Those skilled in the art could realize comparable stringent hybridization conditions to those mentioned above, by appropriately selecting salt concentration (*e.g.*, SSC dilution rate), temperature, and various other conditions including concentrations of surfactant (*e.g.*, NP-40) and formamide, and pH.

Besides the *in situ* hybridization, other examples of the method for detecting a genomic DNA encoding a fusion polypeptide of interest using the polynucleotide mentioned above in (a) or (b) include Southern blotting, Northern blotting and dot blotting. According to these methods, the fusion gene of interest is detected by hybridizing said polynucleotide (a) or (b) to a membrane in which a nucleic acid extract from the above-mentioned biological sample is transcribed. In the case of using said polynucleotide (a), if a polynucleotide that hybridizes to the nucleotide sequence of the fusion partner gene toward the 5'-end and a polynucleotide that hybridizes to the nucleotide sequence of a fusion partner gene toward the 3'-end both recognize the same band developed in the membrane, then it can be determined that a genomic DNA encoding the fusion polypeptide of interest has been detected successfully.

Additional examples of the method for detecting a genomic DNA encoding a fusion polypeptide of interest using said polynucleotide (b) include genome microarray analysis and DNA microarray analysis. According to these methods, the genomic DNA is detected by preparing an array in which said polynucleotide (b) is immobilized on a substrate and bringing the above-mentioned biological sample into contact with the polynucleotide immobilized on the array. The substrate is not particularly limited as long as it allows conversion of an oligo- or polynucleotide into a solid phase, and examples include glass plate, nylon membrane, microbeads, silicon chip, and capillary.

In the inventive detection method, it is also preferred to detect a fusion polynucleotide of interest using PCR.

In the process of PCR, there can be used polynucleotides serving as a pair of primers designed to specifically amplify a fusion polynucleotide using DNA (*e.g*., genomic DNA, cDNA) or RNA prepared from the above-mentioned biological sample as a template. As used herein, the phrase "specifically amplify a fusion polynucleotide" means that the primers do not amplify wild-type genes from which to derive both segments of a fusion polynucleotide of interest each extending from the point of fusion toward the 5'- or 3'-end, but can amplify said fusion polynucleotide alone. It is acceptable to amplify all of the fusion polynucleotide or to amplify that part of the fusion polynucleotide which contains the point of fusion.

The "polynucleotides serving as a pair of primers" to be used for PCR or the like consist of a sense primer (forward primer) and an anti-sense primer (reverse primer) that specifically amplify a target fusion polynucleotide. The sense primer is designed from the nucleotide sequence of that region of said fusion polynucleotide which extends from the point of fusion toward the 5'-end. The anti-sense primer is designed from the nucleotide sequence of that region of said fusion polynucleotide which extends from the point of fusion toward the 3'-end. From the viewpoints of the accuracy and sensitivity of PCR detection, these primers are commonly designed such that a PCR product of not more than 5 kb in size can be amplified. The primers can be designed as appropriate by a known method, for example, using the Primer Express® software (Applied Biosystems). The length of these polynucleotides is generally not less than 15 nucleotides (preferably not less than 16, 17, 18, 19 or 20 nucleotides, more preferably not less than 21 nucleotides) and not more than 100 nucleotides (preferably not more than 90, 80, 70, 60, 50 or 40 nucleotides, more preferably not more than 30 nucleotides).

Preferred examples of the "polynucleotides serving as a pair of primers" include: a primer set that consists of a polynucleotide consisting of the nucleotide sequence of SEQ ID NO: 11 and a polynucleotide consisting of the nucleotide sequence of SEQ ID NO: 12; and a primer set that consists of a polynucleotide consisting of the nucleotide sequence of SEQ ID NO: 21 and a polynucleotide consisting of the nucleotide sequence of SEQ ID NO: 22.

In the process of detecting a fusion polynucleotide by PCR, sequencing is performed on PCR products to sequence a nucleotide sequence containing the point of fusion, whereby it can be confirmed that a gene segment toward the 5'-end and a gene segment toward the 3'-end are joined in-frame and/or that a specified domain is contained in the fusion polynucleotide. Sequencing can be done by a known method -- it can be easily done by using a sequencer (e.g., ABI-PRISM 310 Genetic Analyzer (Applied Biosystems Inc.)) in accordance with its operating instructions.

Also, in the process of detecting a fusion polynucleotide by PCR, it can be confirmed by the TaqMan probe method that a gene segment toward the 5'-end and a gene segment toward the 3'-end are joined in-frame and/or that a specified domain is contained in the fusion polynucleotide. The probe to be used in the TaqMan probe method can be exemplified by the polynucleotide mentioned above in (a) or (b). The probe is labeled with a reporter dye (*e.g.,* FAM, FITC, VIC) and a quencher (*e.g.,* TAMRA, Eclipse, DABCYL, MGB).

The above-mentioned primers and probes can be in the form of DNA, RNA, or DNA/RNA chimera, and is preferably in the form of DNA. Alternatively, the primers and probes may be such that part or all of the nucleotides are substituted by an artificial nucleic acid such as PNA (polyamide nucleic acid: a peptide nucleic acid), LNA™ (Locked Nucleic Acid; a bridged nucleic acid), ENA® (2'-O,4'-C-Ethylene-bridged Nucleic Acid), GNA (glycerol nucleic acid) or TNA (threose nucleic acid). Further, the primers and probes can be double- or single-stranded, and are preferably single-stranded.

As far as the primers and probes are capable of specifically hybridizing to a target sequence, they may contain one or more nucleotide mismatches, generally have at least 80% identity, preferably at least 90%, at least 91%, at least 92%, at least 93%, or at least 94% identity, more preferably at least 95%, at least 96%, at least 97%, at least 98%, or at least 99% identity, and most preferably 100% identity, to a sequence complementary to the target sequence.

The primers and probes can be synthesized, for example, according to a conventional method using an automatic DNA/RNA synthesizer on the basis of the information on the nucleotide sequences disclosed in the present specification.

In the inventive detection method, it is also acceptable to detect a fusion polynucleotide of interest by whole-transcriptome sequencing (RNA sequencing) or genome sequencing. These techniques can be carried out, for example, using a next-generation sequencer (*e.g*., Genome Analyzer IIx (Illumina), HiSeq sequencer (HiSeq 2000, Illumina), Genome Sequencer FLX System (Roche)), or the like according to the manufacturer's instructions. RNA sequencing can be done by, for example, preparing a cDNA library from a total RNA using a commercially available kit (*e.g.*, mRNA-Seq sample preparation kit (Illumina)) according to the manufacturer's instructions and sequencing the prepared library using a next-generation sequencer.

In the inventive detection method, if the object to be detected is a translation product of a fusion polynucleotide (*i.e.,* fusion polypeptide), the translation product can be detected using, for example, immunostaining, Western blotting, RIA, ELISA, flow cytometry, immunoprecipitation, or antibody array analysis. These techniques use an antibody that specifically recognizes a fusion polypeptide. As used herein, the phrase "specifically recognizes a fusion polypeptide" means that the antibody does not recognize other proteins than said fusion polypeptide, including wild-type proteins from which to derive both segments of said fusion polynucleotide each extending from the point of fusion toward the Nor C-terminus, but recognizes said fusion polypeptide alone. The antibody that "specifically recognizes a fusion polypeptide", which is to be used in the inventive detection method, can be one antibody or a combination of two or more antibodies.

The "antibody that specifically recognizes a fusion polypeptide" can be exemplified by an antibody specific to a polypeptide containing the point of fusion of said fusion polypeptide (hereinafter referred to as the "fusion point-specific antibody"). As referred to herein, the "fusion point-specific antibody" means an antibody that specifically binds to the polypeptide containing said point of fusion but does not bind to wild-type proteins from which to derive the segments of the fusion polypeptide each extending toward the N- or C-terminus.

Also, the "antibody that specifically recognizes a fusion polypeptide" can be exemplified by a combination of an antibody binding to a polypeptide consisting of that region of the fusion polypeptide which extends from the point of fusion toward the N-terminus and an antibody binding to a polypeptide consisting of that region of the fusion polypeptide which extends from the point of fusion toward the C-terminus. The fusion polypeptide can be detected by performing sandwich ELISA, immunostaining, immunoprecipitation, Western blotting or the like using these two antibodies.

In the present invention, examples of the antibodies include, but are not limited to, natural antibodies such as polyclonal antibodies and monoclonal antibodies (mAb), and chimeric, humanized and single-stranded antibodies which can be prepared using genetic recombination techniques, and binding fragments thereof. The "binding fragments" refers to partial regions of the above-mentioned antibodies which have specific binding activity, and specific examples include Fab, Fab', F(ab')₂, Fv, and single-chain antibodies. The class of antibody is not particularly limited, and any antibody having any isotype, such as IgG, IgM, IgA, IgD or IgE, is acceptable, with IgG being preferred in consideration of ease of purification or other factors.

The "antibody that specifically recognizes a fusion polypeptide" can be prepared by those skilled in the art through selection of a known technique as appropriate. Examples of such a known technique include: a method in which a polypeptide containing the point of fusion of the fusion polypeptide, a polypeptide consisting of that region of the fusion polypeptide which extends from the point of fusion toward the N-terminus, or a polypeptide consisting of that region of the fusion polypeptide which extends from the point of fusion toward the C-terminus is inoculated into an immune animal, the immune system of the animal is activated, and then the serum (polyclonal antibody) of the animal is collected; as well as monoclonal antibody preparation methods such as hybridoma method, recombinant DNA method, and phage display method. Commercially available antibodies may also be used. If an antibody having a labeling agent attached thereto is used, the target protein can be detected directly by detecting this label. The labeling agent is not particularly limited as long as it is capable of binding to an antibody and is detectable, and examples include peroxidase, β-D-galactosidase, microperoxidase, horseradish peroxidase (HRP), fluorescein isothiocyanate (FITC), rhodamine isothiocyanate (RITC), alkaline phosphatase, biotin, and radioactive materials. Besides the direct detection of the target protein using the antibody having a labeling agent attached thereto, the target protein can also be detected indirectly using a secondary antibody having a labeling agent attached thereto, Protein G or A, or the like.

### <Kit for detecting a gene fusion>

As described above, a fusion polynucleotide produced by the ATF7IP-PDGFRB gene fusion, or a polypeptide encoded thereby, can be detected using such a primer, probe, or antibody or a combination thereof as mentioned above, whereby the gene fusion can be detected. Thus, the present invention provides a kit for detecting a gene fusion (preferably a gene fusion serving as a responsible mutation (driver mutation) for cancer), the kit comprising any or a combination of the polynucleotides and antibody mentioned below (hereinafter also referred to as the "inventive kit"):
(A) a polynucleotide that serves as a probe designed to specifically recognize an ATF7IP-PDGFRB fusion polynucleotide;
(B) polynucleotides that serve as a pair of primers designed to enable specific amplification of an ATF7IP-PDGFRB fusion polynucleotide; or
(C) an antibody that specifically recognizes an ATF7IP-PDGFRB fusion polypeptide.

In addition to the above-mentioned polynucleotide(s) or antibody, the inventive kit can also contain an appropriate combination of other components, including: a substrate required for detecting a label attached to the polynucleotide(s) or the antibody; a positive control (*e.g.*, ATF7IP-PDGFRB fusion polynucleotide, ATF7IP-PDGFRB fusion polypeptide, or cells bearing the same); a negative control; a PCR reagent; a counterstaining reagent for use for *in situ* hybridization or the like (*e.g.,* DAPI); a molecule required for antibody detection (*e.g*., secondary antibody, Protein G, Protein A); and a buffer solution for use in sample dilution or washing. The inventive kit can also contain instructions for use thereof. The above-mentioned inventive detection method can be easily carried out by using the inventive kit.

The inventive detection method and kit, which enable detection of the gene fusion newly discovered as a responsible mutation for cancer, are very useful in identifying subjects positive for said gene fusion and applying personalized medicine to each of the subjects, as described below.

### <Method for identifying a patient with cancer or a subject with a risk of cancer>

The ATF7IP-PDGFRB gene fusion, serving as a responsible mutation for cancer, is believed to lead to constitutive activation of PDGFRB kinase activity, thereby contributing to malignant transformation of cancers. Thus, cancer patients in whom said gene fusion is detected are highly probable to be responsive to the treatment with a substance suppressing the expression and/or activity of a polypeptide encoded by a fusion polynucleotide produced by said gene fusion.

Thus, the present invention provides a method for identifying a patient with cancer or a subject with a risk of cancer, in whom a substance suppressing the expression and/or activity of a polypeptide encoded by a fusion polynucleotide produced by a gene fusion serving as a responsible mutation (driver mutation) for cancer shows a therapeutic effect (hereinafter also referred to as the "inventive identification method").

The inventive identification method comprises the steps of:
(1) detecting an ATF7IP-PDGFRB fusion polypeptide comprising the SETDB1-binding domain of ATF7IP and the transmembrane region and kinase domain of PDGFRB, and having kinase activity, or a fusion polynucleotide encoding said polypeptide, in an isolated sample from a subject, and
(2) determining that a substance suppressing the expression and/or activity of the polypeptide shows a therapeutic effect in the subject, in the case where the fusion polypeptide or the fusion polynucleotide encoding said polypeptide is detected.

In the inventive identification method, the phrase "a patient with cancer or a subject with a risk of cancer" refers to a mammal, preferably human, which is, or is suspected of being, affected by cancer. The "cancer" to which the inventive identification method is to be applied is not particularly limited as long as it is a cancer in which the ATF7IP-PDGFRB gene fusion can be detected, with leukemia being preferred, acute lymphoblastic leukemia (ALL) being more preferred, and B-progenitor acute lymphoblastic leukemia (B-ALL) being particularly preferred.

In the inventive identification method, the "therapeutic effect" is not particularly limited as long as it is a beneficial effect of cancer treatment for a patient, and examples include a tumor shrinkage effect, a progression-free survival prolongation effect, and a life lengthening effect.

In the inventive identification method, the "substance suppressing the expression and/or activity of a polypeptide encoded by a fusion polynucleotide produced by a gene fusion serving as a responsible mutation (driver mutation) for cancer", which is to be evaluated for its effectiveness in the treatment of cancer associated with the ATF7IP-PDGFRB gene fusion (this substance is hereinafter also referred to as the "substance to be evaluated in the inventive identification method"), is not particularly limited as long as it is a substance that directly or indirectly inhibits the expression and/or function of an ATF7IP-PDGFRB fusion polypeptide.

Examples of the substance inhibiting the expression of an ATF7IP-PDGFRB fusion polypeptide include: siRNAs (small interfering RNAs), shRNAs (short hairpin RNA), miRNAs (micro RNAs), and antisense nucleic acids which suppress the expression of an ATF7IP-PDGFRB fusion polypeptide; expression vectors capable of expressing these polynucleotides; and low-molecular-weight compounds.

Examples of the substance inhibiting the function of an ATF7IP-PDGFRB fusion polypeptide include substances inhibiting PDGFRB kinase activity (*e.g.*, low-molecular-weight compounds), and antibodies binding to an ATF7IP-PDGFRB fusion polypeptide.

These substances may be substances that specifically suppress the expression and/or activity of an ATF7IP-PDGFRB fusion polypeptide, or may be substances that suppress even the expression and/or activity of wild-type PDGFRB protein. Specific examples of such substances include substances inhibiting PDGFRB kinase activity (*i.e.,* tyrosine kinase inhibitors), such as imatinib mesylate (GLEEVEC®), dasatinib, nilotinib, ponatinib, rebastinib and bafetinib, with dasatinib being preferred.

These substances can be prepared by a *per se* known technique on the basis of the sequence information of an ATF7IP-PDGFRB fusion polynucleotide and/or an ATF7IP-PDGFRB fusion polypeptide which are disclosed in the present specification, or other data. Commercially available substances may also be used.

These substances are effective as cancer therapeutic agents for a subject, in the case where an ATF7IP-PDGFRB fusion polynucleotide or a polypeptide encoded thereby is detected in an isolated sample from said subject.

Step (1) in the inventive identification method can be carried out in the same way as the step included in the inventive detection method mentioned above.

At step (2) in the inventive identification method, the substance to be evaluated in the inventive identification method is determined to show a therapeutic effect in a subject (*i.e.,* a patient with cancer or a subject with a risk of cancer), in the case where a fusion polynucleotide of interest or a polypeptide encoded thereby is detected in an isolated sample from the subject at step (1); however, the substance to be evaluated in the inventive identification method is determined to be unlikely to show a therapeutic effect in the subject, in the case where neither the fusion polynucleotide of interest nor the polypeptide encoded thereby is detected.

According to the inventive identification method, it is possible to detect a subject positive for the gene fusion newly discovered as a responsible mutation for cancer from among patients with cancer or subjects with a risk of cancer, and to identify a patient with cancer or a subject with a risk of cancer, in whom a substance suppressing the expression and/or activity of a polypeptide encoded by a fusion polynucleotide produced by said gene fusion shows a therapeutic effect; thus, the present invention is useful in that it makes it possible to provide a suitable treatment for such a subject.

### <Method for treatment of cancer and cancer therapeutic agent>

As described above, the inventive identification method identifies a patient with cancer, in whom a substance suppressing the expression and/or activity of a polypeptide encoded by a fusion polynucleotide produced by the ATF7IP-PDGFRB gene fusion shows a therapeutic effect. Thus, efficient cancer treatment can be performed by administering said substance selectively to a cancer patient who carries said fusion gene. Therefore, the present invention provides a method for treating cancer, comprising the step of administering said substance to a subject in whom said substance is determined to show a therapeutic effect by the inventive identification method mentioned above (hereinafter also referred to as the "inventive treatment method").

Also, since the substance to be administered in the inventive treatment method functions as a cancer therapeutic agent, the present invention further provides a cancer therapeutic agent comprising, as an active ingredient, a substance suppressing the expression and/or activity of a polypeptide encoded by a fusion polynucleotide produced by the ATF7IP-PDGFRB gene fusion (hereinafter also referred to as the "inventive cancer therapeutic agent").

In the context of the inventive treatment method and inventive cancer therapeutic agent, the cancer is not particularly limited as long as it is ATF7IP-PDGFRB gene fusion-positive cancer, with leukemia being preferred, acute lymphoblastic leukemia (ALL) being more preferred, and B-progenitor acute lymphoblastic leukemia (B-ALL) being particularly preferred. As referred to herein, the "ATF7IP-PDGFRB gene fusion-positive cancer" refers to a cancer in which an ATF7IP-PDGFRB fusion polynucleotide or a polypeptide encoded thereby is detected by the inventive detection method.

The inventive cancer therapeutic agent can be exemplified by the substances that are mentioned, in relation to the inventive identification method, as examples of the substance suppressing the expression and/or activity of a polypeptide encoded by a fusion polynucleotide produced by the ATF7IP-PDGFRB gene fusion.

The inventive cancer therapeutic agent can be formulated into a pharmaceutical composition with the use of a pharmaceutically acceptable carrier, excipient and/or other additives which are commonly used in pharmaceutical manufacturing.

The method for administering the inventive cancer therapeutic agent is selected as appropriate depending on various factors including the type of this suppressing agent and the type of cancer, and exemplary modes of administration that can be adopted include oral, intravenous, intraperitoneal, transdermal, intramuscular, intratracheal (aerosol), rectal and intravaginal administrations

The dose of the inventive cancer therapeutic agent can be determined as appropriate in consideration of various factors, including the activity and type of an active ingredient, the mode of administration (*e.g.*, oral or parenteral administration), the severity of a disease, and the animal species, drug receptivity, body weight, and age of the subject to be treated with the inventive agent.

The treatment method and cancer therapeutic agent of the present invention are useful in that they allow treatment of patients having the particular responsible mutation for cancer which has been conventionally unknown and was first discovered according to this invention.

### <Method for screening a cancer therapeutic agent>

The present invention provides a method for screening a cancer therapeutic agent which shows a therapeutic effect in a cancer patient having the ATF7IP-PDGFRB gene fusion (hereinafter also referred to as the "inventive screening method"). According to the inventive screening method, a substance suppressing the expression and/or activity of an ATF7IP-PDGFRB fusion polypeptide can be obtained as a cancer therapeutic agent.

The test substance to be subjected to the inventive screening method can be any compound or composition, and can be exemplified by nucleic acids (*e.g.*, nucleoside, oligonucleoside, polynucleoside), saccharides (*e.g.*, monosaccharide, disaccharide, oligosaccharide, polysaccharide), fats (*e.g.*, saturated or unsaturated, straight-chain, branched-chain and/or cyclic fatty acids), amino acids, proteins (*e.g.*, oligopeptide, polypeptide), low-molecular-weight compounds, compound libraries, random peptide libraries, natural ingredients (*e.g.*, ingredients derived from microbes, animals and plants, marine organisms, and others), foods, and the like.

The inventive screening method can be of any type as long as it enables evaluation of whether a test substance suppresses the expression and/or activity of an ATF7IP-PDGFRB fusion polypeptide. Typically, the inventive screening method comprises the following steps:
(1) bringing a test substance into contact with a cell that expresses an ATF7IP-PDGFRB fusion polypeptide comprising the SETDB1-binding domain of ATF7IP and the transmembrane region and kinase domain of PDGFRB, and having kinase activity;
(2) determining whether the expression and/or activity of the fusion polypeptide is suppressed or not; and
(3) selecting the substance determined to suppress the expression and/or activity of the fusion polypeptide, as a cancer therapeutic agent.

At step (1), the cell expressing an ATF7IP-PDGFRB fusion polypeptide is brought into contact with the test substance. A test substance-free solvent (*e.g.*, DMSO) can be used as a control. The contact can be effected in a medium. The medium is selected as appropriate depending on various factors including the type of the cell to be used, and examples include a minimum essential medium (MEM) supplemented with about 5-20% fetal bovine serum, a Dulbecco's modified eagle's medium (DMEM), RPMI1640 medium, and 199 medium. The culture conditions are also selected as appropriate depending on various factors including the type of the cell to be used, and for example, the pH of the medium is in the range of approximately from 6 to 8, the culture temperature is in the range of approximately from 30 to 40°C, and the culture time is in the range of approximately from 12 to 72 hours.

Examples of the cell expressing an ATF7IP-PDGFRB fusion polypeptide include, but are not limited to, cancer tissue-derived cells intrinsically expressing said fusion polypeptide, cell lines induced from said cells, and cell lines made by genetic engineering. Whether a cell expresses an ATF7IP-PDGFRB fusion polypeptide can also be confirmed using the inventive detection method described above. The cell is generally a mammalian cell, preferably a human cell.

At step (2), it is determined whether the test substance suppresses the expression and/or activity of said fusion polypeptide or not. The expression of a fusion polypeptide can be measured by determining the mRNA or protein level in a cell using a known analysis technique such as Northern blotting, quantitative PCR, immunoblotting, or ELISA. Also, the activity of a fusion polypeptide can be measured by a known analysis technique (*e.g.*, kinase activity assay). The resulting measured value is compared with the value measured in a control cell not contacted with the test substance. The comparison between the measured values is made preferably based on the presence or absence of a significant difference. If the value measured in the cell contacted with the test substance is significantly lower than that measured in the control cell, it can be determined that the test substance suppresses the expression and/or activity of said fusion polypeptide.

Alternatively, since the cell expressing such a fusion polypeptide shows enhanced growth, the growth of said cell can be used as an indicator for the determination at this step. In this case, the growth of the cell contacted with the test substance is measured as a first step. The cell growth measurement can be made by a *per se* known technique such as cell count, ³H-thymidine incorporation, or BRDU. Next, the growth of the cell contacted with the test substance is compared with that of a control cell not contacted with the test substance. The growth level comparison is made preferably based on the presence or absence of a significant difference. The value for the growth of the control cell not contacted with the test substance can be a value measured prior to, or at the same time as, the measurement of the growth of the cell contacted with the test substance, and the value measured at the same time is preferred from the viewpoints of the accuracy and reproducibility of the test. If the results of the comparison show that the growth of the cell contacted with the test substance is suppressed, it can be determined that the test substance suppresses the expression and/or activity of said fusion polypeptide.

At step (3), the test substance determined to suppress the expression and/or activity of said fusion polypeptide at step (2) is selected as a cancer therapeutic agent.

Thus, the inventive screening method makes it possible to obtain a cancer therapeutic agent applicable to the treatment of patients having a responsible mutation for cancer which has been conventionally unknown.

### <Isolated fusion polypeptide or fragment thereof, and polynucleotide encoding the same>

The present invention provides an isolated ATF7IP-PDGFRB fusion polypeptide (hereinafter also referred to as the "inventive fusion polypeptide") or a fragment thereof, which comprises the SETDB1-binding domain of ATF7IP and the transmembrane region and kinase domain of PDGFRB, and has kinase activity.

For the purpose of the present specification, the "isolated" substance refers to a substance substantially separated or purified from other substances (preferably, biological factors) found in an environment in which the substance naturally occurs (*e.g.,* in a cell of an organism) (for example, if the substance of interest is a nucleic acid, the "other substances" corresponds to other factors than nucleic acids as well as nucleic acids containing other nucleic acid sequences than that of the nucleic acid of interest; and if the substance of interest is a protein, the "other substances" corresponds to other factors than proteins as well as proteins containing other amino acid sequences than that of the protein of interest). For the purpose of the specification, the term "isolated" means that a substance has a purity of preferably at least 75% by weight, more preferably at least 85% by weight, still more preferably at least 95% by weight, and most preferably at least 96% by weight, at least 97% by weight, at least 98% by weight, at least 99% by weight, or 100%. Examples of the "isolated" polynucleotide and polypeptide include not only polynucleotides and polypeptides purified by standard purification techniques but also chemically synthesized polynucleotides and polypeptides.

The "fragment" refers to a fragment of the inventive fusion polypeptide, which consists of a consecutive partial sequence comprising sequences upstream and downstream from the point of fusion. The sequence upstream from the point of fusion, as contained in said partial sequence, can comprise at least one amino acid residue (for example, at least 2, 3, 4, 5, 6, 7, 8, 9, 10, 20, 30, 40, 50 or 100 amino acid residues) from the point of fusion to the N-terminus of the inventive fusion polypeptide. The sequence downstream from the point of fusion, as contained in said partial sequence, can comprise at least one amino acid residue (for example, at least 2, 3, 4, 5, 6, 7, 8, 9, 10, 20, 30, 40, 50 or 100 amino acid residues) from the point of fusion to the C-terminus of the inventive fusion polypeptide. The length of the fragment is not particularly limited, and is generally at least 8 amino acid residues (for example, at least 9, 10, 11, 12, 13, 14, 15, 20, 25, 50 or 100 amino acid residues).

Also, the inventive fusion polypeptide can be, for example, any of the isolated fusion polypeptides mentioned below:
(i) a polypeptide consisting of the amino acid sequence of SEQ ID NO: 2 or 4,
(ii) a polypeptide consisting of an amino acid sequence derived from the amino acid sequence of SEQ ID NO: 2 or 4 by deletion, substitution or addition of one or more amino acids, and the polypeptide having kinase activity, or
(iii) a polypeptide consisting of an amino acid sequence having a sequence identity of at least 80% to the amino acid sequence of SEQ ID NO: 2 or 4, and the polypeptide having kinase activity.

The meanings of the terms used in relation to the inventive fusion polypeptide are as defined above in <Specific responsible mutation for cancer>.

Further, the present invention provides an isolated polynucleotide encoding the inventive fusion polypeptide or the fragment thereof as described above (hereinafter also referred to as the "inventive polynucleotide"). The inventive polynucleotide can be in the form of any of mRNA, cDNA and genomic DNA. Also, the polynucleotides may be double- or single-stranded.

A typical example of the cDNA encoding the ATF7IP-PDGFRB fusion polypeptide is a polynucleotide consisting of the nucleotide sequence of SEQ ID NO: 1 or 3.

The inventive polynucleotide can be made by a *per se* known technique. For example, the inventive polynucleotide can be extracted using a known hybridization technique from a cDNA library or genomic library prepared from cancer tissues or the like harboring an ATF7IP-PDGFRB fusion polynucleotide. The inventive polynucleotides can also be prepared by amplification utilizing a known gene amplification technique (PCR), with mRNA, cDNA or genomic DNA prepared from the cancer tissues or the like being used as a template. Alternatively, the polynucleotides can be prepared utilizing a known gene amplification or genetic recombination technique such as PCR, restriction enzyme treatment, or site-directed mutagenesis (Kramer, W. & Fritz, H. J., Methods Enzymol., 1987, 154, 350), using, as starting materials, the cDNAs of wild-type genes from which to derive those segments of the ATF7IP-PDGFRB fusion polynucleotide which extend toward the 5'- or 3'-end.

The inventive fusion polypeptide or the fragment thereof can also be made by a *per* se known technique. For example, after such a polynucleotide prepared as mentioned above is inserted into an appropriate expression vector, the vector is introduced into a cell-free protein synthesis system (*e.g.*, reticulocyte extract, wheat germ extract) and the system is incubated, or alternatively the vector is introduced into appropriate cells (*e.g., E coli.,* yeast, insect cells, animal cells) and the resulting transformant is cultured; in either of those ways, the inventive fusion polypeptide can be prepared.

The inventive fusion polypeptide or the fragment thereof can be used as a marker in the inventive detection method or the like, or can be used in other applications including preparation of an antibody against the inventive fusion polypeptide.

### EXAMPLES

Hereunder, the present invention will be more specifically described by way of working examples, but this invention is not limited to the examples given below.

### Example 1

### <Samples>

Total RNAs were extracted using the miRNeasy mini kit (Qiagen) from leukemic cells obtained from 300 children affected by pediatric leukemia (acute lymphoblastic leukemia). The present study was conducted with the approval by the institutional review boards of the institutions involved in the study.

### <RNA sequencing>

cDNA libraries for RNA sequencing were prepared using the TruSeq RNA sample preparation kit v2 Set B (Illumina) according to the manufacturer's standard protocol. The sequences of the total RNAs were analyzed by paired-end sequencing using a next-generation sequencer (HiSeq1000 Illumina).

### <Detection of fusion transcripts>

Detection of fusion transcripts was performed using the DeFuse program (http://sourceforge.net/apps/mediawiki/defuse/index.php?title=DeFuse), a dedicated algorithm for fusion gene detection in cancer cells.

### <RT-PCR and Sanger sequencing>

cDNAs were synthesized using the ReverTra Ace® qPCR RT Master Mix (Toyobo) from the total RNAs extracted from leukemic cells from the subjects, and the resulting cDNAs were subjected to PCR amplification with rTaq DNA Polymerase (Toyobo). Agarose gel electrophoresis was performed to verify the amplification of gene fragments of desired size. The amplified gene fragments were TA-cloned into the pGEM-T Easy vector (Promega). Nucleotide sequencing was done using the BigDye® Terminator v3.1 Cycle Sequencing Kit (Applied Biosystems) and the ABI 3130xl Genetic Analyzer (Applied Biosystems), with the T7 and SP6 sequences located respectively toward the 5'- and 3'-ends of the multicloning site of this vector being used as primers.

This example describes the identification of a novel fusion transcript in leukemic cells.

In order to identify a novel fusion transcript potentially serving as a target for treatment, transcriptome analysis was carried out on the leukemic cells obtained from 300 children with pediatric leukemia (acute lymphoblastic leukemia).

As a result of the analysis of the paired-end reads obtained by the RNA sequencing, the novel fusion gene ATF7IP-PDGFRB conceivably produced by a chromosomal translocation (t(5;12)), as shown in Fig. 1, was identified in one subject.

Further, RT-PCR was performed using the following primers on the sample with the detected ATF7IP-PDGFRB gene, and the obtained PCR product was subjected to sequence analysis to verify the expression of this novel fusion gene (FIGs. 2 and 3).

| | |
|---|---|
| Forward primer: | CAAGTGGACCATCTCAGACCAC (SEQ ID NO: 11) |
| Reverse primer: | ATTTAAGCATCTTGACGGCCAC (SEQ ID NO: 12) |

The ATF7IP-PDGFRB gene is a fusion gene between the ATF7IP gene located at chromosome 12p13.1 and the PDGFRB gene located at chromosome 5q33.1.

These results demonstrated that the gene fusion discovered in Example 1 can be detected by verifying the presence or absence of the amplification of a specific band by RT-PCR and determining the nucleotide sequence of a PCR product.

Additionally, the gene expression pattern displayed in the ATF7IP-PDGFRB gene-positive subject was a Ph-like phenotype, *i.e.,* a pattern in which genes are expressed in a similar profile to BCR-ABL fusion gene-positive leukemia (Ph1-ALL), a poor-prognosis subgroup of pediatric leukemia but no BCR-ABL fusion gene is detected; thus, it was considered that the ATF7IP-PDGFRB gene-positive subject may belong to a peculiar poor-prognosis subgroup of acute lymphoblastic leukemia. Therefore, it was suggested that detection of the ATF7IP-PDGFRB gene may make it possible to identify a previously unknown poor-prognosis subgroup of ALL.

### Example 2

This example describes a monotherapy with the tyrosine kinase inhibitor (TKI) dasatinib in a patient with recurrent Ph-like acute lymphoblastic leukemia bearing ATF7IP/PDGFRB translocation.

### Case report

On February 2011, an 8-year-old male child developed standard-risk ALL. The child successfully completed induction and consolidation therapies, but experienced a relapse at 26 months after the diagnosis, even while receiving maintenance chemotherapy. The observation of ATF7IP/PDGFRB by both fluorescence *in situ* hybridization (FISH) and RT-PCR analyses provided an evidence to confirm the recurrence of the original clone (Fig. 4A). The RT-PCR performed 8 months before the relapse showed a negative result for minimum residual disease (MRD) (Kobayashi K., et al., Br J Haematol, 2014, 165, 836-841); thus, it was recognized that molecular monitoring with RT-PCR is not sensitive enough to predict an early relapse. Therefore, it was decided to establish an alternative MRD monitoring approach, and to perform genome mapping to search for a fusion sequence in the ATF7IP/PDGFRB translocation. A genomic breakpoint was identified between ATF7IP intron 13 and PDGFRB exon 10, and a primer pair targeting a leukemia-specific DNA fingerprint was established. It is of particular note that the leukemia-specific DNA sequence was readily detected even from the remission sample obtained at 18 months after the diagnosis (Fig. 4A). This suggests that genomic PCR-based monitoring of MRD, even when it is detected only at a very low level, is a powerful predictor of early relapse.

The relapsing blast cells showed complete refractoriness to the ALL-type protocol with dexamethasone, vincristine, cytarabine, methotrexate, and L-asparaginase. Thus, the therapy was switched to a reinduction AML-type regimen with etoposide, cytarabine, and mitoxantrone (Tsukimoto I., et al., J Clin Oncol, 2009, 27, 4007-4013). Following 3 courses of the therapy, cytogenic remission was achieved, but MRD was still detected within the quantitative range. The child consecutively received cord blood stem cell transplantation (CBSCT) followed by a myeloablative conditioning regimen with etoposide (50 mg/kg/day on day -4) and melphalan (70 mg/m²/day on days -3 and -2), along with total body irradiation (12 Gy). The subsequent clinical course was uneventful with a major molecular response based on RT-PCR analysis, but the fusion-specific leukemic DNA sequence was persistently detected with genomic PCR even at 34 months after the initial diagnosis. The cut-off value of MRD for predicting relapse was largely unknown; however, as was evidenced in the former clinical analyses, the presence of leukemic DNA even in the molecular remission sample suggested that the child was at high risk of impending relapse. Thus, it was decided to initiate a therapy with the second-generation TKI dasatinib (66 mg/m²/day) for the purpose of eradication of the MRD. The therapeutic response was prompt, with the MRD disappearing within 3 months according to the leukemia-specific genomic PCR analysis (Fig. 4A). The child had remained in complete molecular remission at least for 12 months.

In order to confirm the clinical outcome of TKI monotherapy for Ph-like ALL, it is necessary to perform longitudinal disease monitoring with durable sensitivity and specificity. In fact, RT-PCR analysis of the pathognomic fusion transcript, *i.e.,* BCR/ABL, is commonly used for the therapeutic monitoring of TKI (Hughes T., et al., Blood, 2006, 108, 28-37), but it has also been shown that RT-PCR negativity does not directly correlate with the clinical outcome in CML (Chu S., et al., Blood, 2011, 118, 5565-5572). For the development of personalized therapy on hematological malignancy, a detailed analysis of MRD is not only important for the diagnosis but also for the justification of TKI therapy. In this regard, it is particularly important to note that recent increasing evidences suggest that genomic PCR targeting a junctional DNA sequence is more sensitive than conventional RT-PCR in adult CML patients (Mattarucchi E., et al., J Mol Diagn, 2009, 11, 482-487; Bartley P. A., et al., Int J Lab Hemato, 2010, 32, e222-228), but its clinical significance in BCP-ALL is largely unknown. For that reason, a paired comparison of MRD detection sensitivity between RT-PCR and genomic PCR was performed using 17 samples obtained from the patient at different points of time (Fig. 4B). In the 11 samples, in which MRD levels were detectable within the quantitative range of the PCR assay, there was observed a clear correlation in results between the two methods. Conversely, discordant results were found in 4 samples; namely, leukemic DNA was detected even from the molecular-remission samples with negative RT-PCR results. In contrast, no sample was found in which MRD was positive by RT-PCR but negative by genomic PCR. Thus, it is considered that the discordance in results between RT-PCR and genomic PCR analyses is likely to be mostly due to a difference in detection sensitivity rather than sample variations. Furthermore, it is possible to speculate that the fusion transcript level examined with RT-PCR is less closely related to the number of residual leukemic cells due to various reasons such as transcriptional silencing, especially with a quiescent cellular status, and possible involvement of mRNA degradation (Shibata Y., et al., Genome Medicine, 2010, 2, 70). In contrast, genomic PCR is directly related to the measurement of the number of leukemic cells, as each leukemic cell contains one copy of the fusion DNA sequence. Since DNA is more stable than RNA and does not require a reverse transcription step, a DNA-based MRD analysis may also facilitate a retrospective research, as was shown in the present case. Furthermore, Pagani, *et al.* reported that fusion-specific DNA can be detected in 30% or more of RT-PCR-negative CML patients on long-term TKI treatment (Pagani I. S., et al., Oncoscience, 2014, 1, 510-521). The report stressed that the genomic PCR targeting the BCR/ABL genomic breakpoint could be utilized in combination with conventional techniques for the long-term monitoring of CML patients with TKI therapy. Thus, the above-mentioned observations including those obtained in this example collectively suggest that genomic PCR targeting a fusion sequence can also be utilized as one of the reliable supplementary techniques for MRD monitoring in Ph-like ALL patients receiving TKI therapy.

### Discussion

The advent of TKI has been of marked therapeutic benefit, not only for CML patients, but also for patients with other rare types of pediatric hematological malignancy, such as Ph-like ALL (Roberts K. G., et al., N Engl J Med, 2014, 371, 1005-1015; Weston B. W., et al., J Clin Oncol, 2013, 31, e413-416; Lengline E., et al., Haematologica, 2013, 98, e146-148). Furthermore, it is of particular note that patients with myeloid malignancies bearing PDGFRB fusion genes show excellent therapeutic outcomes in response to the TKI imatinib mesylate, with a 10-year overall survival rate of 90% without secondary resistance (Cheah C. Y., et al., Blood, 2014, 123, 3574-3577). Thus, it is believed that companion diagnostics focusing on molecular targets, such as the PDGFRB fusion gene, may facilitate future personalized cancer therapy for children.

### Example 3

This example describes that the ATF7IP-PDGFRB fusion kinase induces cell transformation.

### Materials and methods

### Reagents

The TKIs used were imatinib, dasatinib, nilotinib (LC Laboratories, Woburn, MA, USA), ponatinib, rebastinib, and bafetinib (Selleck, Houston, TX, USA). The mitogen-activated protein kinase kinase (MEK) inhibitor, PD98059, and the phosphatidylinositol 3-kinase (PI3 kinase) inhibitor, LY294002, were purchased from Merck Millipore (Darmstadt, Germany).

### Plasmid construction

The cDNA of wild-type (WT-) PDGFRB was purchased from Promega Corporation (Madison, WI, USA) as Flexi® ORF clone pF1KB7023 (kazusa clone cp01083). The coding region was isolated by digestion with the Sfg I and Pme I enzymes followed by blunt ending with a T4 DNA polymerase. After the attachment of protruding dATP to the 3'-end by an rTaq polymerase (Toyobo Co., Ltd., Osaka, Japan), WT-PDGFRB cDNA was subcloned into the pGEM-T Easy or pGEM-T vector (Promega) in the reverse direction (from SP6 to T7 site).

The use of the clinical materials for the research was approved by the institutional review boards, and informed consent was obtained from the parents or guardians. A 5' portion of ATF7IP cDNA contained in the ATF7IP-PDGFRB chimeric gene was amplified from a clinical specimen of the patient carrying the fusion gene, by PCR with KOD plus ver.2 (Toyobo) using the following primers, with said portion being divided into three parts, ATF7IP-1, -2, and -3:
ATF7IP-1 forward (Fwd): 5'-TTCAGAATGGACAGTTTAGAAGAACCTCAG-3' (SEQ ID NO: 13),
ATF7IP-1 reverse (Rev): 5'-TCTGCTGGAGAGCACGTTTC-3' (SEQ ID NO: 14),
ATF7IP-2 Fwd: 5'-AAGCTTGCACCTTCTGAGGATGA-3' (SEQ ID NO: 15),
ATF7IP-2 Rev: 5'-ATCGGATCTCGTAACGTGGC-3' (SEQ ID NO: 16),
ATF7IP-PDGFRB-3 Fwd: 5'-TGGTCCTCTCTGATGAAGAGGATATTTC-3' (SEQ ID NO: 17), and
ATF7IP-PDGFRB-3 Rev: 5'-TCATCGTGGCCTGAGAATGG-3' (SEQ ID NO: 18).

The PCR products having protruding dATP attached to the 3'-end as described above were each subcloned into the pGEM-T Easy vector (Promega) in the forward direction (from T7 to SP6 site), as described previously (Tomita, et al., Leukemia Research, 2014, 38, 361-370). To obtain the full-length ORF of ATF7IP-PDGFRB cDNA, the cDNA fragments ATF7IP-3, -2 and -1 digested respectively with the following combinations, Not I/Pmi I, Not I/Afi I, and Not I/Cla I, were subcloned into pGEM-T-WT-PDGFRB by sequential ligation.

Likewise, a 5' portion of EBF1 cDNA was amplified using the following primers:
EBF1-PDGFRB Fwd: 5'-ATGTTTGGGATTCAGGAAAGCATCC-3' (SEQ ID NO: 19), and
EBF1-PDGFRB Rev: 5'-ATGCGGTAACCCCGTTTGAT-3' (SEQ ID NO: 20).
The PCR products were subcloned into the pGEM-T Easy vector. The full length ORF of EBF1-PDGFRB cDNA was obtained by ligation of the Sph I (blunted) and Bam HI fragments of EBF1 cDNA with the NdeI (blunted) and Bam HI fragments of pGEM-T-WT-PDGFRB.

To obtain a retroviral expression vector, the full-length inserts of the cDNAs of the chimeric gene were digested with the Sph I (blunted) and Not I enzymes and subcloned into the Nru I and Not I sites of the pRetroX Tight vector (Clontech Laboratories, Inc., Madison, WI, USA). In the case of WT-PDGFRB, the full-length insert of the cDNA was isolated by Eco RI digestion and subcloned into the Eco RI site in the pRetroX Tight vector.

The expressions of the ATF7IP-PDGFRB, EBF1-PDGFRB, and WT-PDGFRB genes were amplified from cDNAs prepared from Ba/F3 cells transfected with said genes, by means of PCR using an rTaq polymerase (Toyobo), as described previously (Iijima, et al., Eur J Immunol, 2012, 42, 3405-3415). The amplification was performed using the following primers:
ATF7IP-PDGFRB detection Fwd-1: 5'-CGTGAATGTAACACATCGTCCA-3' (SEQ ID NO: 21),
ATF7IP-PDGFRB detection Rev-1: 5'-AGCTCCCACGTGGAGTCATAG-3' (SEQ ID NO: 22),
EBF1-PDGFRB detection Fwd-1: 5'-GGGCGTGAATTCGTTCAGTG-3' (SEQ ID NO: 23),
EBF1-PDGFRB detection Rev-1: 5'-ATCGGATCTCGTAACGTGGC-3' (SEQ ID NO: 24),
WT-PDGFRB detection Fwd-1: 5'-TCCAGCACCTTCGTTCTGAC-3' (SEQ ID NO: 25), and
WT-PDGFRB detection Rev-1: 5'-CCAGAAAAGCCACGTTGGTG-3' (SEQ ID NO: 26).

As an internal control, the primer set for the detection of mouse β-actin (Agilent Technologies, Santa Clara, CA, USA) was also used.

### Cells, cell culture, and transfection

Ba/F3 cells and WEHI-3 cells (RIKEN Bioresource Center, Tsukuba, Ibaraki, Japan) were maintained, as described previously (Tomita, et al., Leukemia Research, 2014, 38, 361-370). In the case of Ba/F3 cells, 10% (vol/vol) conditioned medium prepared from the WEHI-3 cell line containing IL-3 was supplemented for maintenance, as described previously (Tomita, et al., Leukemia Research, 2014, 38, 361-370).

Chimera gene-inducible cell lines were generated by employing retroviral transfection using the Retro-X™ Tet-On® Advanced Inducible Expression System and Retro-X™ Universal Packaging System (Clontech), basically following a previously described procedure (Iijima, et al., Eur J Immunol, 2012, 42, 3405-3415). In these systems, the Tet-on Advanced vector carrying the neomycin-resistant gene and the pRetroX Tight expression vector carrying the puromycin-resistant gene were used. In a total of 3 mL of the medium, 6×10⁵ Ba/F3 cells transduced with the Tet-on Advanced vector were further infected with the pRetroX Tight retrovirus containing either ATF7IP-PDGFRB or EBF1-PDGFRB, and exposed to this retrovirus for 10 hours. The cells were then washed and divided into two aliquots, which were treated with neomycin and puromycin for drug selection in the presence or absence of 1 µg/mL of doxycycline (DOX), whereby the expression of the intended proteins was induced. After 24-hour incubation, the cells were washed again to withdraw the WEHI-3-conditioned medium. Half of each aliquot of treated cells was plated for cell proliferation assay in triplicate, and the remaining half was subjected to sequential drug selection. The stable drug-resistant cells were cloned by repeating limiting dilution 3 times and used in the following experiments. A WT-PDGFRB or Mock transfectant bearing an empty vector was established in the same way, except that WEHI-3 conditioned medium was left present consistently as the transfectant cannot proliferate in an IL-3-independent manner.

### Cell proliferation assay

The chimeric gene transfectants and Mock Ba/F3 cells were each washed twice with RPMI-1640 medium, dispensed in triplicate into 96-well plates (2.5 × 10⁴ cells/0.1 mL per well) in the presence or absence of the reagents, and cultured at 37°C for the periods indicated in the figures. Cell proliferation was analyzed using water-soluble tetrazolium salt (WST) assay (Cell Counting Kit-8, Dojindo, Kumamoto, Japan), as described previously (Yamada, et al., Int J Hematol, 2013, 97, 73-82). The data were analyzed by F-test. P values less than 0.05 were considered significant. All experiments were performed at least three times to calculate means and SEM values.

### Flow cytometric analysis and immunocytochemistry

The frequency of apoptotic cells was quantified using the MEBCYTO® Apoptosis Kit (Medical and Biological Laboratories, Nagoya, Japan), and then analyzed according to the manufacturer's protocol, as described previously (Tomita, et al., Leukemia Research, 2014,38,361-370).

For immunocytochemistry, the cells were treated with the PerFix-nc Kit (Beckman Coulter, Inc., Indianapolis IN, USA) according to the manufacturer's protocol and stained with a combination of rabbit monoclonal anti-PDGFRB antibody (Ab) (Cell Signaling Technology, Inc., Danvers, MA, USA) and Alexa Fluor® 546 goat anti-rabbit IgG (Life Technologies, Waltham, MA, USA). The cells were mounted on a slide glass, counterstained with 4',6-diamidino-2-phenylindole (DAPI) using Fluoroshield Mounting Medium with DAPI (Immunobioscience Corp., Mukilteo, WA, USA), and visualized by DeltaVision Elite (GE Healthcare Life Sciences, Buckinghamshire, UK).

### Immunoblotting and immunoprecipitation

Immunoblot analysis was performed as described previously (Kiyokawa, et al., J Biol Chem, 1997, 272, 18656-18665; Tomita, et al., Leukemia Research, 2014, 38, 361-370) using the following antibodies: anti-phosphotyrosine (4G10; Merck Millipore), anti-PDGF receptor β (28E1), anti-phospho-PDGF receptor β Tyr751 (C63G6), anti-phospho-PDGF receptor β Tyr 771 (76D6), anti-phospho-PDGF receptor β Tyr1009 (42F9), anti-phospho-PDGF receptor β Tyr1021 (6F10), anti-phospho-p44/42 MAPK (ERK1/2, Tyr202/Tyr204), anti-phospho-p38 MAPK (Thr180/Tyr182), anti-phospho-JNK (Thr183/Tyr185, 81E11), anti-phospho-AKT (Ser473), anti-phospho-PLCγ1 (Tyr783), anti-Casitas B-lineage lymphoma binding (Cbl), anti-p38 MAPK antibody and anti-JNK antibody (Cell Signaling Technology); anti-ATF7IP (MCAF1) (Abcam, Cambridge, UK); anti-ERK, anti-PLCγ, (BD Biosciences, San Jose, CA, USA); anti-β-actin (Sigma-Aldrich, St. Louis, MO); and horseradish peroxidase (HRP)-conjugated secondary antibody (Dako, Glostrup, Denmark). A 50-µg sample of each cell lysate was electrophoretically separated on an SDS-polyacrylamide gel and transferred to a nitrocellulose membrane (Amersham Hybond-ECL; GE Healthcare), as described previously (Kiyokawa, et al., J Biol Chem, 1997, 272, 18656-18665). The membranes were incubated with an appropriate combination of primary and secondary antibodies, washed, and detected with an enhanced chemiluminescent reagent system (ECL) and Western Blotting Detection Reagents (GE) (Kiyokawa, et al., J Biol Chem, 1997, 272, 18656-18665).

PDGFRB and chimeric proteins were immunoprecipitated from 1 mg of cell lysate prepared from each Ba/F3 transfectant, with the use of a combination of appropriate antibodies and protein G-agarose (F. Hoffmann-La Roche Ltd., Basel, Schweiz), as described previously (Kiyokawa, et al., J Biol Chem, 1997, 272, 18656-18665). Immunoprecipitates were separated on an SDS-polyacrylamide gel and then subjected to immunoblot analysis as described above using appropriate combinations indicated in the figures.

### Results

The introduction of PDGFRB-related chimeric molecules induces IL-3-independent proliferation of Ba/F3 cells, but the introduction of WT-PDGFRB molecules does not induce such proliferation.

Firstly, a tetracycline-inducible gene expression system was used to examine whether the expression of chimeric molecules gives rise to IL-3-independent proliferation in Ba/F3 cells. After transfection with an ATF7IP-PDGFRB-expression vector, each aliquot of the Ba/F3 cells was treated in the presence or absence of DOX, and then WEHI-3-conditioned medium containing IL-3 was withdrawn. As shown in Fig. 5A, the one aliquot of Ba/F3 transfectants treated in the presence of DOX survived and grew in the absence of the conditioned medium while the other aliquot of Ba/F3 transfectants treated in the absence of DOX was unable to survive without the conditioned medium; consequently, selection of ATF7IP-PDGFRB transfectants was readily achieved. In contrast, the Mock-transfected Ba/F3 cells were unable to survive without the conditioned medium independently of DOX treatment. Similarly, the Ba/F3 cells transfected with EBF1-PDGFRB also exhibited IL-3-independent proliferation after DOX treatment, whereas the WT-PDGFRB transfectants did not (data not shown). After the cloning of the transfectants, it was further confirmed that the ATF7IP-PDGFRB and EBF1-PDGFRB transfectants showed cell proliferation even in the absence of the conditioned medium containing IL-3, whereas the Mock-transfected Ba/F3 cells were able to proliferate only in the presence of the conditioned medium (Fig. 5B). The growth rates of the PDGFRB-related chimera transfectants were equivalent to that of parental Ba/F3 cells growing in the presence of the conditioned medium (data not shown). The WT-PDGFRB transfectants were unable to survive without the conditioned medium even if the ligand PDGFBB was present (Fig. 5B). Interestingly, the addition of PDGFBB partially prolonged the survival of the WT-PDGFRB transfectants in the absence of the conditioned medium, as assessed by WST and Annexin V assays (Figs. 5C & D).

Next, it was confirmed that the above-described effects were actually mediated by the introduced PDGFRB-related chimeric molecules. As shown in Figs. 6A & B, appropriate mRNA and protein expressions in each of the transfectants were observed by RT-PCR and immunoblot analyses. As shown in Fig. 6C, immunocytochemistry revealed the cytoplasmic localization of ATF7IP-PDGFRB chimeric proteins.

### Intracellular cellular signaling mediated by PDGFRB-related chimeric molecules in Ba/F3 cells

Next assessed was the molecular basis of the IL-3-independent cell proliferation due to the expression of PDGFRB-related chimeric molecules. Since PDGFRB is a receptor tyrosine kinase, the state of tyrosine phosphorylation of cellular proteins and PDGFRB-related chimeric molecules themselves was examined. As shown in Fig. 7A, intracellular proteins were tyrosine-phosphorylated in the ATF7IP-PDGFRB- and EBF1-PDGFRB-expressing Ba/F3 cells, but not in the Mock cells. As for the case of WT-PDGFRB, stimulation with PDGFBB induces the tyrosine phosphorylation of WT-PDGFRB proteins. Also examined was the tyrosine phosphorylation of the particular residues located in PDGFRB. As shown in Fig. 7B, the Tyr751, Tyr771, Tyr1009, and Tyr1021 residues on both ATF7IP-PDGFRB and EBF1-PDGFRB were phosphorylated. In addition, the same tyrosine residues on WT-PDGFRB were phosphorylated after PDGFBB stimulation (Fig. 7B).

Next examined was the phosphorylation of molecules located downstream of PDGFRB. Upon phosphorylation, the Tyr751 residue on PDGFRB allowed the binding and activation of PI3 kinase and Nckβ (Kazlauskas, et al., Mol Cell Biol, 1992, 12, 2534-2544; Panayotou, et al., EMBO J, 1992, 11, 4261-4272; Nishimura, et al., Mol Cell Biol, 1993, 13, 6889-6896). Consistent with the phosphorylation of Tyr751 on PDGFRB, Ser473 on AKT, a downstream molecule of PI3 kinase, as well as Thr183/Tyr185 and Thr180/Tyr182 located respectively on c-Jun N-terminal kinase (JNK) and p38 MAPK, which are downstream molecules of Nckβ, were phosphorylated in the ATF7IP-PDGFRB- and EBF1-PDGFRB-transduced Ba/F3 cells unlike in the Mock cells, as assessed by immunoblot analysis (Fig. 8). In the WT-PDGFRB-transduced Ba/F3 cells, the same serine residue on AKT was also phosphorylated after stimulation with PDGFBB (Fig. 8). Similarly, the phosphorylation of the Tyr771 residue on PDGFRB allowed the binding and activation of RasGap (Kashishian, et al., EMBO J, 1992, 11, 1373-1382; Kazlauskas, et al., Mol Cell Biol, 1992, 12, 2534-2544). Consistent with the phosphorylation of the Tyr771 residue on PDGFRB, the phosphorylation levels of the Ser217/Ser221 residues on p44/42 MAP kinases (Erk1/2), downstream molecules of the Ras signaling pathway, revealed increased phosphorylation levels in both the two types of Ba/F3 cells containing PDGFRB-related chimeric kinases, like in WT-PDGFRB stimulated with PDGFBB (Fig. 8). Consistent with the phosphorylation of Tyr1009 and 1021 on PDGFRB, which allow the activation of PLC-γ (Reddi, *et al.,* 2007), the Tyr783 residue on PLC-γ was phosphorylated in the Ba/F3 transfectants containing PDGFRB-related chimeras. Interestingly, the phosphorylation of the same residue on PLC-γ in the WT-PDGFRB-expressing cells was not significant even after stimulation with PDGFBB (Fig. 8).

### Phosphorylation of c-Cbl and binding of c-Cbl to PDGFRB in Ba/F3 cells

As shown in Fig. 6B, immunoblot analysis of WT-PDGFRB revealed some unexpected bands of WT-PDGFRB protein with smaller molecular weights besides the predicted size, independently of PDGFBB stimulation, whereas the same test of ATF7IP- and EBF1-PDGFRB showed unique bands to the respective chimeric kinases. Since it has been documented that various growth factor receptors, including PDGFRB, undergo ligand-binding-mediated polyubiquitination and are thought to play a negative regulatory role in mitogenic signaling (Mori, et al., J Biol Chem, 1993, 268, 577-583; Waterman, et al., J Biol Chem, 1999, 274, 22151-22154), the above-described observation indicates the polyubiquitination of WT-PDGFRB in Ba/F3 cells. In addition, it has been reported that c-Cbl is responsible for the ubiquitination of PDGFRB (Miyake, et al., J Biol Chem, 1999, 274, 16619-16628), while ETV6-PDGFRB, a PDGFRB-related fusion protein detected in myeloproliferative neoplasms (MPNs), can escape from c-Cbl-induced ubiquitination and degradation (Toffalini F., et al., Haematologica, 2009, 94, 1085-1093). Therefore, the relationship between chimeric PDGFRB proteins and c-Cbl was examined. As shown in Fig. 9, immunoprecipitation and immunoblot analyses revealed that tyrosine residues on c-Cbl were phosphorylated in both ATF7IP- and EBF1-PDGFRBs as well as in PDGFBB-stimulated WT-PDGFRB. On the other hand, significant binding of c-Cbl protein to WT-PDGFRB after PDGFBB stimulation was observed, while ATF7IP- and EBF1-PDGFRB presented only limited binding of c-Cbl protein (Fig. 9). The data indicate that WT-PDGFRB activates c-Cbl and then is polyubiquitinated by it upon ligand stimulation, whereas ATF7IP- and EBF1-PDGFRB are constitutively phosphorylated by but do not bind with c-Cbl.

### Roles of MAP kinases and AKT in ATF7IP-PDGFRB-medicated, IL-3-independent proliferation of Ba/F3 cells

Since ATF7IP-PDGFRB has been shown to phosphorylate MAP kinases and AKT, an examination was made on the effects of MEK and PI3 kinase inhibitors on the IL-3-independent proliferation of the ATF7IP-PDGFRB-expressing Ba/F3 cells. As presented in Fig. 10A, the AKT inhibitor LY294002 induced cell death in both of the ATF7IP-PDGFRB-expressing Ba/F3 cells and the Mock-expressing Ba/F3 cells. Interestingly, the MEK inhibitor PD98059 induced cell death only in the ATF7IP-PDGFRB-expressing Ba/F3 cells, and not in the Mock-expressing Ba/F3 cells. Also, it was confirmed that the simultaneous addition of the conditioned medium containing IL3 partially avoided MEK inhibitor-induced cell death in the ATF7IP-PDGFRB-expressing Ba/F3 cells (Fig. 10B). Further, it was confirmed that treatment with the MEK inhibitor PD98059 markedly reduced the phosphorylation of MAP kinase, as assessed by immunoblotting (Fig. 11). Similarly, treatment with the PI3 kinase inhibitor LY294002 clearly reduced the phosphorylation of AKT (Fig. 11).

Also examined were the kinetics of MAP kinase and AKT phosphorylation mediated by WT-PDGFRB. As shown in Fig. 12, PDGFBB stimulation of the WT-PDGFRB-expressing Ba/F3 cells starved for IL-3 induced the transient but significant phosphorylation of both MAP kinases and AKT, but this phosphorylation diminished in a time-dependent manner even though PDGFBB was left present consistently. In the presence of the conditioned medium containing IL-3, the conditioned medium itself induced the phosphorylation of MAP kinases and AKT, and thus the phosphorylation of both of these proteins was somewhat prolonged after PDGFBB stimulation (Fig. 12).

### Effects of TKIs on survival of Ba/F3 cells expressing PDGFRB-related chimeric molecules

Next examined were the effects ofTKIs on the IL-3-independent proliferation of the ATF7IP-PDGFRB-expressing Ba/F3 cells. When the ATF7IP-PDGFRB-expressing Ba/F3 cells were exposed to imatinib or dasatinib, cell proliferation was significantly inhibited in a concentration-dependent manner, and the viable cell number decreased to below 10% after 48-hour incubation with imatinib at a concentration of 25 nM and dasatinib at a concentration of 2.5 nM (Fig. 13). The calculated IC₅₀ value of imatinib against the ATF7IP-PDGFRB-expressing Ba/F3 cells at 24 hours of incubation was 45.6 nM, being much lower than the value of 600 nM estimated for BCR-ABL-expressing Ba/F3 cells in the previous literature (Tomita, et al., Leukemia Research, 2014, 38, 361-370). Also examined were the effects of other next-generation TKIs, including nilotinib, bafetinib, rebastinib, and ponatinib. As shown in Fig. 13, these TKIs effectively reduced the viable cell number of the ATF7IP-PDGFRB-expressing Ba/F3 cells. Similarly, the EBF1-PDGFRB-expressing Ba/F3 cells also exhibited marked sensitivity to the TKIs (Fig. 13). Further, it was confirmed that the phosphorylation of MAP kinases/AKT and PDGFRB actually diminished in the ATF7IP- and EBF1-PDGFRB-expressing Ba/F3 cells treated with TKI, as assessed by immunoblot analysis (Fig. 14).

### Discussion

In this example, it was demonstrated that the expression of ATF7IP-PDGFRB induces IL-3-independent proliferation of Ba/F3 cells, which indicates the transforming potential of ATF7IP-PDGFRB, as in the cases of EBF1-PDGFRB and other PDGFRB-related fusion-kinases detected in MPNs (Carroll M., et al., Proc Natl Acad Sci USA, 1996, 93, 14845-14850; Wilbanks A. M., et al., Experimental Hematology, 2000, 28, 584-593; Toffalini F., et al., J Biol Chem, 2010, 285, 12268-12278; Roberts K. G., et al., Cancer Cell, 2012, 22, 153-166). The data obtained in this example show that ATF7IP-PDGFRB is constitutively phosphorylated on tyrosine residues and activates downstream molecules, including AKT, PLC-γ, MAP kinases, p38 MAP kinase, and JNK. Furthermore, the activation of AKT and MAP kinases was shown to be essential for the IL-3-independent survival of Ba/F3 cells. In contrast, the data of this example also clarified that WT-PDGFRB transduced in Ba/F3 cells was not sufficient for the IL-3-independent survival of Ba/F3 cells, while stimulation with PDGFBB partially relieved Ba/F3 cells from the apoptotic cell death induced by the withdrawal of IL-3.

As for the mechanism of the constitutive activation of ATF7IP-PDGFRB, the deregulation of c-Cbl-mediated ubiquitination was believed to be involved in it (Toffalini F., et al., Haematologica, 2009, 94, 1085-1093). It has been well-documented that PDGFRB and other receptor tyrosine kinases activated by binding with ligands phosphorylate and activate c-Cbl promptly, leading to c-Cbl-mediated polyubiquitination of the receptor tyrosine kinases, and that this process is essential for the self-down-regulation of activated growth-factor receptor (Miyake S., et al., J Biol Chem, 1999, 274, 16619-16628; Yokouchi M., et al., J Biol Chem, 1999, 274, 31707-31712). Consistent with previous reports, the marked fragmentation of WT-PDGFRB, but not ATF7IP- PDGFRB or EBF1-PDGFRB, was observed in Ba/F3 transfectants. Also, in Ba/F3 transfectants, there was observed an increased binding of c-Cbl to WT-PDGFRB after stimulation with PDGFBB, but no significant binding of c-Cbl to ATF7IP-PDGFRB or EBF1-PDGFRB was observed. The data suggest that the activation of WT-PDGFRB with ligands is self-limited by induction of c-Cbl-mediated ubiquitination and thus is not enough to fully support IL-3-independent cell survival. In contrast, constitutively activated ATF7IP- and EBF1-PDGFRB phosphorylate c-Cbl protein but do not bind with c-Cbl and, thus, can escape from ubiquitination (Toffalini F., et al., Haematologica, 2009, 94, 1085-1093). Persistent activation of downstream molecules mediated by ATF7IP- and EBF1-PDGFRB may be critical for the IL-3-independent survival of Ba/F3 cells.

The mechanism of the deregulation of c-Cbl-mediated ubiquitination that occur in ATF7IP-PDGFRB may be explained in some ways. For example, Toffalini and co-workers reported that since the TEL-PDGFRB and FIP1L1-PDGFRA fusion proteins, unlike wild-type receptors, are not inserted in the plasma membrane but reside in the cytoplasm, the lack of ubiquitination of these fusion proteins is associated with a difference in their localization (Toffalini F., et al., Haematologica, 2009, 94, 1085-1093). On the other hand, in this example, significant phosphorylation of PLC-γ was observed in both of the ATF7IP- and EBF1-PDGFRB-transduced Ba/F3 cells, but no such phosphorylation was observed in the WT-PDGFRB-transduced cells even if they were stimulated with PDGFBB. Since it has been reported that c-Cbl and PLC-γ compete for the Tyr1021 residue as a docking site and are activated by PDGFRB in a mutually exclusive manner (Reddi A. L., et al., J Biol Chem, 2007, 282, 29336-29347), it is likely that ATF7IP-PDGFRB may preferentially activate PLC-y and, thus, escape from binding with c-Cbl due to the mutually exclusive effect, avoiding c-Cbl-mediated ubiquitination.

As shown in this example, the PI3 kinase inhibitor leads to cell death in both of the ATF7IP-PDGFRB-transduced cells and the Mock cells maintained by IL-3-containing WEHI-3-conditioned medium, which indicates that signals mediated by AKT are essential for cell survival in both cases. In contrast, the MEK inhibitor leads to cell death in the ATF7IP-PDGFRB-transduced cells but not in the Mock cells, which indicates the difference between the cell survival signals induced by ATF7IP-PDGFRB and those induced by the growth factor, and which suggests that MAP kinase-mediated signals are more critical in ATF7IP-PDGFRB-mediated cell transformation. One possible explanation is that the IL-3 receptor can simultaneously activate a surrogate pathway for cell survival other than the MAP kinase cascade, while PDGFRB can not. Alternatively, since PDGFRB induces not only MAP kinase but also both p38 MAP kinase and JNK which mediate apoptotic signals, MAP kinase-mediated anti-apoptotic signals may be essential for overcoming the apoptotic signals mediated by p38 MAP kinase and JNK in growth factor-independent cell proliferation, while the IL-3 receptor may activate another pathway that regulates the above-mentioned apoptotic signals mediated by p38 MAP kinase and JNK. The reason why the growth factor receptors simultaneously activate both survival signals mediated by MAP kinases and apoptotic signals mediated by p38 MAP kinase and JNK is yet to be clarified (Yu J., et al., J Biol Chem, 2000, 275, 19076-19082), but the reason may be due to an alternate self-regulating mechanism of growth factor receptor activation.

Importantly, the data obtained in this example also demonstrate that the ATF7IP-PDGFRB-expressing Ba/F3 cells show high sensitivity to various TKIs, including imatinib, dasatinib, and other new-generation TKIs. The estimated IC₅₀ value of imatinib is much lower than that of BCR-ABL1. This observation is consistent with the clinical observations of other researchers who reported the effectiveness of imatinib in patients with PDGFRB-rearranged leukemia (Weston B. W., et al., J Clin Oncol, 2013, 31, e413-416; Lengline E., et al., Haematologica, 2013, 98, e146-48; Cheah C. Y., et al., Blood, 2014, 123, 3574-3577). TKIs can effectively inhibit the kinase activity of PDGFRB and reduce the activation of AKT and MAP kinases, which is required for cell survival. The high sensitivity of rearranged PDGFRB kinases to TKIs is important in the therapeutic consideration of the use of TKIs in patients with PDGFRB-rearranged ALL.

As stated above, ATF7IP-PDGFRB and EBF1-PDGFRB have so far been found only in ALL patients, but other PDGFRB fusion genes have been found only in myeloid neoplasms, which suggests selectivity between chimeric partners of PDGFRB and the lineage of the developing neoplasms, though the precise mechanism of this selectivity is yet to be clarified. Interestingly, Tomasson and co-workers reported that, in a murine BMT model, the transduction of TEL-PDGFRB into the whole bone marrow caused a rapidly fatal myeloproliferative disease, whereas phenylalanine mutants with tyrosine residues 579/581 in the fusion gene developed T-cell lymphomas (Tomasson M. H., et al., J Clin Invest, 2000, 105, 423-432). The difference in chimeric partner may give rise to a difference in conformation of the PDGFRB kinase domain which affects the lineage commitment of the developing neoplasms. An elucidation of the mechanism of the selectivity of ATF7IP-PDGFRB for ALL development is awaited.

In conclusion, ATF7IP-PDGFRB has a transforming potential and exhibits significant sensitivity to TKIs as compared to BCR-ABL1. The observations obtained in this example are consistent with the clinical findings, and indicate the therapeutic benefit of TKIs for a subset of BCP-ALL harboring PDGFRB-related chimeric kinases. In addition, further clarification of the molecular basis of chimeric PDGFRB kinase-mediated cell transformation should provide a new therapeutic strategy against hematologic malignancies caused by PDGFRB translocation.

### INDUSTRIAL APPLICABILITY

The present invention makes it possible to detect a gene fusion newly discovered as a responsible mutation for cancer; to identify patients with cancer or subjects with a risk of cancer, in whom a substance suppressing the expression and/or activity of a polypeptide encoded by a fusion polynucleotide produced by said gene fusion shows a therapeutic effect; and to provide suitable treatment (personalized medicine) for such cancer patients.

### SEQUENCE LISTING FREE TEXT

SEQ ID NOs: 1 and 3: ATF7IP-PDGFRB fusion polynucleotides
SEQ ID NOs: 2 and 4: ATF7IP-PDGFRB fusion polypeptides
SEQ ID NOs: 5 and 7: ATF7IP cDNAs
SEQ ID NOs: 6 and 8: ATF7IP polypeptides
SEQ ID NO: 9: PDGFRB cDNA
SEQ ID NO: 10: PDGFRB polypeptide
SEQ ID NO: 11: Forward primer
SEQ ID NO: 12: Reverse primer
SEQ ID NO: 13: ATF7IP-1 Fwd
SEQ ID NO: 14: ATF7IP-1 Rev
SEQ ID NO: 15: ATF7IP-2 Fwd
SEQ ID NO: 16: ATF7IP-2 Rev
SEQ ID NO: 17: ATF7IP-PDGFRB-3 Fwd
SEQ ID NO: 18: ATF7IP-PDGFRB-3 Rev
SEQ ID NO: 19: EBF1-PDGFRB Fwd
SEQ ID NO: 20: EBF1-PDGFRB Rev
SEQ ID NO: 21: ATF7IP-PDGFRB detection Fwd-1
SEQ ID NO: 22: ATF7IP-PDGFRB detection Rev-1
SEQ ID NO: 23: EBF1-PDGFRB detection Fwd-1
SEQ ID NO: 24: EBF1-PDGFRB detection Rev-1
SEQ ID NO: 25: WT-PDGFRB detection Fwd-1
SEQ ID NO: 26: WT-PDGFRB detection Rev-1

## Claims

1. A method for detecting a gene fusion, the method comprising the step of detecting an ATF7IP-PDGFRB fusion polypeptide comprising the SETDB1-binding domain of ATF7IP and the transmembrane region and kinase domain of PDGFRB, and having kinase activity, or a fusion polynucleotide encoding said polypeptide, in an isolated sample from a subject.

2. The method according to claim 1, wherein the fusion polypeptide is any one of (i) to (iii) mentioned below:
(i) a polypeptide consisting of the amino acid sequence of SEQ ID NO: 2 or 4,
(ii) a polypeptide consisting of an amino acid sequence derived from the amino acid sequence of SEQ ID NO: 2 or 4 by deletion, substitution or addition of one or more amino acids, and the polypeptide having kinase activity, or
(iii) a polypeptide consisting of an amino acid sequence having a sequence identity of at least 80% to the amino acid sequence of SEQ ID NO: 2 or 4, and the polypeptide having kinase activity.

3. The method according to claim 1 or 2, wherein the fusion polynucleotide is any one of (i) to (iv) mentioned below:
(i) a polynucleotide consisting of the nucleotide sequence of SEQ ID NO: 1 or 3,
(ii) a polynucleotide that hybridizes under stringent conditions with a polynucleotide consisting of a nucleotide sequence complementary to the polynucleotide consisting of the nucleotide sequence of SEQ ID NO: 1 or 3, and which encodes a polypeptide having kinase activity,
(iii) a polynucleotide that consists of a nucleotide sequence derived from the nucleotide sequence of SEQ ID NO: 1 or 3 by deletion, substitution or addition of one or more nucleotides, and which encodes a polypeptide having kinase activity, or
(iv) a polynucleotide that has a sequence identity of at least 80% to the polynucleotide consisting of the nucleotide sequence of SEQ ID NO: 1 or 3, and which encodes a polypeptide having kinase activity.

4. The method according to any one of claims 1 to 3, wherein the gene fusion is a responsible mutation (driver mutation) for cancer.

5. The method according to claim 4, wherein the cancer is acute lymphoblastic leukemia.

6. A method for identifying a patient with cancer or a subject with a risk of cancer, in whom a substance suppressing the expression and/or activity of a polypeptide encoded by a fusion polynucleotide produced by a gene fusion serving as a responsible mutation (driver mutation) for cancer shows a therapeutic effect, the method comprising the steps of:
(1) detecting an ATF7IP-PDGFRB fusion polypeptide comprising the SETDB1-binding domain of ATF7IP and the transmembrane region and kinase domain of PDGFRB, and having kinase activity, or a fusion polynucleotide encoding said polypeptide, in an isolated sample from a subject, and
(2) determining that the substance suppressing the expression and/or activity of the polypeptide shows a therapeutic effect in the subject, in the case where the fusion polypeptide or the fusion polynucleotide encoding said polypeptide is detected.

7. A kit for detecting a gene fusion, the kit comprising any or a combination of (A) to (C) mentioned below:
(A) a polynucleotide that serves as a probe designed to specifically recognize an ATF7IP-PDGFRB fusion polynucleotide;
(B) polynucleotides that serve as a pair of primers designed to enable specific amplification of an ATF7IP-PDGFRB fusion polynucleotide; or
(C) an antibody that specifically recognizes an ATF7IP-PDGFRB fusion polypeptide.

8. The kit according to claim 7, wherein the gene fusion is a responsible mutation (driver mutation) for cancer.

9. An isolated ATF7IP-PDGFRB fusion polypeptide or a fragment thereof, which comprises the SETDB1-binding domain of ATF7IP and the transmembrane region and kinase domain of PDGFRB, and has kinase activity.

10. A polynucleotide encoding the fusion polypeptide or the fragment thereof according to claim 9.

11. A therapeutic agent for ATF7IP-PDGFRB gene fusion-positive cancer.

12. The therapeutic agent according to claim 11, comprising, as an active ingredient, a substance suppressing the expression and/or activity of an ATF7IP-PDGFRB fusion polypeptide comprising the SETDB1-binding domain of ATF7IP and the transmembrane region and kinase domain of PDGFRB, and having kinase activity.

13. The therapeutic agent according to claim 11 or 12, wherein the cancer is acute lymphoblastic leukemia.

14. The therapeutic agent according to any one of claims 11 to 13, wherein the substance suppressing the expression and/or activity of an ATF7IP-PDGFRB fusion polypeptide comprising the SETDB1-binding domain of ATF7IP and the transmembrane region and kinase domain of PDGFRB, and having kinase activity is a substance inhibiting the kinase activity of PDGFRB.

15. The therapeutic agent according to claim 14, wherein the substance inhibiting the kinase activity of PDGFRB is imatinib mesylate, dasatinib, nilotinib, ponatinib, rebastinib or bafetinib.

16. The therapeutic agent according to claim 14, wherein the substance inhibiting the kinase activity of PDGFRB is dasatinib.

17. A method for screening a cancer therapeutic agent, the method comprising the steps of:
(1) bringing a test substance into contact with a cell that expresses an ATF7IP-PDGFRB fusion polypeptide comprising the SETDB1-binding domain of ATF7IP and the transmembrane region and kinase domain of PDGFRB, and having kinase activity;
(2) determining whether the expression and/or activity of the fusion polypeptide is suppressed or not; and
(3) selecting the substance determined to suppress the expression and/or activity of the fusion polypeptide, as a cancer therapeutic agent.
